(19)

Europäisches
Patentamt
European
Patent Office
Office européen
des brevets

(11)  **EP 4 439 067 A1**

(12)  **EUROPEAN PATENT APPLICATION**

(43) Date of publication:
**02.10.2024  Bulletin 2024/40**

(51) International Patent Classification (IPC):
***G01N 33/84*** *(2006.01)*

(21) Application number: **23305426.1**

(22) Date of filing: **28.03.2023**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
- **Centre National de la Recherche Scientifique
  75016 Paris (FR)**
- **Université Toulouse III - Paul Sabatier
  31400 Toulouse (FR)**

(72) Inventors:
- **DAVEZAC, Noélie
  31500 Toulouse (FR)**

- **BORDENEUVE-GUIBE, Joel
  31400 Toulouse (FR)**
- **COUSTHAM, Corentin
  31400 Toulouse (FR)**
- **MERABET, Nadège
  13016 Marseille (FR)**

(74) Representative: **Novagraaf Technologies
2 rue Sarah Bernhardt
CS90017
92665 Asnières-sur-Seine Cedex (FR)**

Remarks:
The complete document including Reference
Table(s) and the Sequence Listing(s) can be
downloaded from the EPO website

(54)  **METHOD FOR IDENTIFYING REACTIVE OXYGEN SPECIES**

(57)    The invention relates to a method for in vitro quantifying the reactive oxygen species, or ROS, appearance rate in a biological sample, the method comprising
- quantifying the amount of enzyme of mitochondrial complex I and III, and
- calculating from the above amount the ROS, appearance rate.

EP 4 439 067 A1

**Description**

The invention relates to a method for identifying reactive oxygen species, in particular involved in oxidative stress.

Reactive oxygen species are involved in various cell signaling pathways that are necessary for cell growth and survival. Correlations have been shown between misregulation of ROS and various diseases, including cancer, diabetes, heart disease, and neurodegenerative diseases. Hydrogen peroxide has been a focus of research geared toward understanding ROS in health and disease because it is a relatively long-lived ROS; thus, it is able to travel through a cell or even across cell membranes before it reacts with a target biomolecule.

Various efforts have been made to detect hydrogen peroxide. For example, fluorescent scaffolds with boronate detection groups have been synthesized for the examination of hydrogen peroxide in cellular systems. Chemiluminescent probes based on peroxalate nanoparticles or on luminol, have been developed.

There remains a need in the field for compounds and methods of detecting ROS, including hydrogen peroxide.

Thus, the invention relates to a method for in vitro quantifying the reactive oxygen species, or ROS, appearance rate in a biological sample, the method comprising:

a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

- the reduced form of Nicotinamide adenine dinucleotide, or NADH,
- the oxidized form of Nicotinamide adenine dinucleotide, or NAD+,
- quinone, or Q
- quinol, or $QH_2$,
- molecular oxygen, or $O_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,
- $[QH_2]$, corresponding to the amount of quinol measured in said biological sample,
- $[O_2]$, corresponding to the amount of molecular oxygen measured in said biological sample,
- $[CytC_{Red}]$ or $[CytC_{red}]$, corresponding to the amount of reduced form of cytochrome C measured in said biological sample,
- $[CytC_{ox}]$, corresponding to the amount of oxidized form of cytochrome C measured in said biological sample,

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH], [NAD^+]) = \frac{\frac{[NADH]}{K_M^{NADH}}}{1 + \frac{[NADH]}{K_M^{NADH}} + \frac{[NAD^+]}{K_M^{NAD^+}}} \text{ in which } K_M^{NADH} = \frac{K_{M,0}^{NADH}}{1 + e^{\frac{f_Q - k_1}{k_2}}} \ ;$$

$$f_Q([Q], [QH_2]) = \frac{\frac{[Q]}{K_M^Q}}{1 + \frac{[Q]}{K_M^Q} * I_{s,Q,QH_2} + \frac{[QH_2]}{K_M^{QH_2}} * I_{s,Q,QH_2}} \text{ in which } I_{s,Q,QH_2} = 1 + \frac{[Q]}{K_{is}^Q} + \frac{[QH_2]}{K_{is}^{QH_2}} \ ;$$

and

$$f_{O_2}([O_2]) = \frac{\frac{[O_2]}{K_{O_2}}}{\left(1 + \frac{[NAD^+]}{K_i^{NAD^+}}\right)(1 + \alpha f_Q)}$$

wherein

$V^+$ = 1727.2 mmol/min/mg ;
$K_{M,0}^{NADH}$ = 10,6
$K_1$ = 174.1, $K_2$= 681.2,
$K_M^{NAD+}$ = 1028.6 $\mu$M,
$K_M^Q$= 11.6 $\mu$M, $K_{is}^Q$= 719128.5 $\mu$M,
$K_M^{QH2}$ = 16.3 $\mu$M, $K_{is}^{QH2}$ = 36.2 $\mu$M,
$K_i^{NAD+}$ = 26.4
$K_{O2}$ = 4871.0 and
$\alpha$= 5.

c) calculating a second Ros appearance rate $V_{2\,Ros}$ of ROS in said sample

$$V_{2\,Ros} = V2^+ * f_{QH_{2o}} * f_{cytC_{ox}} * (1 + \frac{[Q]}{K_M^{Q_o}}) * f_{O_2}$$

wherein

$$f_{QH_{2o}}([QH_2],[Q]) = \frac{\frac{[QH_2]}{K_M^{QH_{2o}}}}{1 + \frac{[QH_2]}{K_M^{QH_{2o}}} + \frac{[Q]}{K_M^{Q_o}}} \text{ in which } K_M^{QH_{2o}} = \frac{K_{M,0}^{QH_{2o}}}{1 + e^{-\frac{f_{cytC_{ox}} - k_1}{k_2}}}$$

$$f_{cytC_{ox}}([cytC_{ox}],[cytC_{red}]) = \frac{\frac{[cytC_{ox}]}{K_M^{cytC_{ox}}}}{1 + \frac{[cytC_{ox}]}{K_M^{cytC_{ox}}} + \frac{[cytC_{red}]}{K_M^{cytC_{red}}}} \text{ and } f_{Q_i}([Q],[QH_2]) = \frac{\frac{[Q]}{K_M^{Q_i}}}{1 + \frac{[Q]}{K_M^{Q_i}} + \frac{[QH_2]}{K_M^{QH_{2i}}}},$$

and

$$f_{O_2}([O_2]) = \frac{\frac{[O_2]}{K'_{O_2}}}{1 + \alpha' f_{Q_i} + \beta f_{cytC_{ox}}}$$

wherein

$V2^+$ = 3579.29 mmol/min/mg ;
$K_1$ = 23.05, $K_2$= 472.12,
$K_{M,0}^{QH2o}$ =184.77 $\mu$M, $K_M^{QH2i}$ = 397,14,
$K_M^{Qo}$= 43.08 $\mu$M, $K_M^{Qi}$ = 225.21 $\mu$M,
$K_M^{cytCox}$ = 8.11 $\mu$M, $K_M^{cytCred}$= 419.16$\mu$M,
$K'_{O2}$ = 914.51,
$\alpha'$= 22.02 and $\beta$=173.54,

d) obtaining the Ros appearance rate such that

$$VRos = V_{1Ros} + V_{2\,Ros.}$$

The invention is based on the unexpected observation made by the inventors that an optimized model for measuring the activity of both mitochondrial Complex I and Complex III allows to simply assess the amount of Ros that can be produced in a cell, and then in an individual.

The invention allows then to measure ROS production, not by measuring Ros themselve, but by measuring the activity of the enzymes and enzyme complexes from which the ROS are produced.

Reactive oxygen species (hereafter referred as ROS or Ros ) are highly reactive chemicals formed from diatomic oxygen ($O_2$). Examples of ROS include the following species: hydroxyl radical (HO'), hydroxide ion (HO$^-$), triplet oxygen ($O_2^{2\bullet}$) superoxide anion ($O_2^{\bullet-}$), peroxide ion ($O_2^{-2}$), hydrogen peroxide ($H_2O_2$), and nitric oxide (NO'). ROS are known to be produced by mitochondria, in particular buy Complexes I and III of the respiratory chain.

Complex I of mitochondria is the first enzyme of the respiratory chain. This complex, i.e. the enzymes contained in the complex, oxidizes NADH, which is generated through the Krebs cycle in the mitochondrial matrix, and uses the two electrons to reduce ubiquinone to ubiquinol. Ubiquinol is reoxidized by the cytochrome bc1 complex and transfers electrons to reduce molecular oxygen to water at complex IV. The redox energy released during this process is used to transfer protons from the mitochondrial matrix to the periplasmic space that generates proton-motive force across the inner mitochondrial membrane at complex I, III, and IV. Complex V uses this proton-motive force to produce ATP from ADP and inorganic phosphate. This entire process constitutes OXPHOS. Because complex I is the major entry point for electrons to the respiratory chain and is suggested as the rate-limiting step in overall respiration, it plays a central role in energy metabolism.

Complex I is the largest and most complicated component of the respiratory chain. Using the bovine heart as the model system, previous work has characterized all the complex I subunits and cloned the encoding genes. However, the functions of the individual subunits are largely unclear. Complex I is the only component of the mammalian respiratory chain whose three-dimensional structure is available only at a low resolution. Mitochondria are also major factors in modulating calcium signaling [4,5], which is a universal secondary messenger. As we will discuss further, complex I has been implicated in the regulation of reactive oxygen species (ROS), which are important molecules in various signaling pathways, including apoptosis.

Mitochondrial complex III generates superoxide during the ubiquinone (Q)-cycle. The Q-cycle involves the transfer of two electrons to ubiquinone from complex I and complex II resulting in the reduction of ubiquinone to ubiquinol (QH2). The subsequent oxidation of ubiquinol at complex III requires the donation of those two electrons to the single electron carrier cytochrome c. The first electron transfer at complex III is to the Reiske iron-sulfur center protein (RISP). This electron is then transferred to cytochrome c1 and subsequently to cytochrome c. This one electron transfer from ubiquinol results in the unstable radical ubisemiquinone ($Q^{\bullet-}$) which can donate its unpaired electron to oxygen to generate superoxide within the Q-cycle. Under most circumstances however, the unpaired electron of ubisemiquinone is transferred to the two heme groups of cytochrome b (Heme bL and Heme bH). The two hemes have different electron affinities because they are located in different polypeptide environments. Heme bL is located closer to the intermembrane space and has a lower affinity for electrons than Heme bH which is located closer to their matrix side. Ubisemiquinone transfers its electron to bL to form ubiquinone. Heme bL in turn donates an electron to Heme bH, which subsequently reduces another molecule of ubiquinone, forming ubisemiquinone. The Q-cycle at this stage is only half complete as only one electron from ubiquinol has been transferred to cytochrome c. After a second round of the Q-cycle, two molecules of ubiquinol have been oxidized on the intermembrane side of the inner membrane, two molecules of cytochrome c have been reduced, and one molecule of ubiquinone has been reduced on the matrix side of the inner membrane.

Based on the data of the literature, the inventors were able to propose an optimized model for both mitochondrial complexes I and III. By simply measuring the concentration of specific substrate/products of these two complexes, and applying the optimized model, the inventors can easily and rapidly, but also efficiently quantify the ROS amount in a cell, a biological sample, and by extension to a patient.

In the method defined above the concentration of

- the reduced form of Nicotinamide adenine dinucleotide, or NADH,
- the oxidized form of Nicotinamide adenine dinucleotide, or NAD+,
- quinone, or Q
- quinol, or $QH_2$,
- molecular oxygen, or $O_2$,
- the reduced form of cytochrome C, or cytCred or $cytC_{Red}$, and
- the oxidized form of cytochrome C, or cytCox,

is measured.

Intracellular NAD+ and NADH levels can be measured with an NAD+/NADH Assay kit (Abcam) according to the manufacturer's instructions. Other similar tests are also usable.

Quinone reduced and oxidized levels can be performed as described in (Galinier et al., 2006).

The reduced form of cytochrome C, or cytCred, and the oxidized form of cytochrome C, or cytCox can be measured as described in Abramczyk, H.;Brozek-Pluska, B.; Kopec, M.; Surmacki, J.; Blaszczyk, M.; Radek, M. Redox Imbalance and Biochemical Changes in Cancer by Probing Redox-Sensitive Mitochondrial Cytochromes in Label-Free Visible Resonance Raman Imaging. Cancers 2021, 13, 960. and Abramczyk, H.;et al Scientific reports 2022.

Other methods known in the art can be used.

Then, the optimized equations as defined above are applied for each of the complexes I and III, in order to obtain the respective ROS appearance rate for each complex. In order to determine the global ROS appearance rate by adding the result for each complex.

In other words, the invention relates to a method for quantifying, preferably *in vitro,* the reactive oxygen species, or ROS, appearance rate in a biological sample, the method comprising:

a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

- the reduced form of Nicotinamide adenine dinucleotide, or NADH,
- the oxidized form of Nicotinamide adenine dinucleotide, or NAD+,
- quinone, or Q
- quinol, or $QH_2$,
- molecular oxygen, or $O_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,
- $[QH_2]$, corresponding to the amount of quinol measured in said biological sample,
- $[O_2]$, corresponding to the amount of molecular oxygen measured in said biological sample,
- $[CytC_{Red}]$, corresponding to the amount of reduced form of cytochrome C measured in said biological sample,
- $[CytC_{ox}]$, corresponding to the amount of oxidized form of cytochrome C measured in said biological sample,

b) calculating a first ROS appearance rate $V_{1\,Ros}$ of ROS in said sample

$$V_{1\,Ros} = 1727.2 \ * f_{NADH} * f_{O_2}$$

wherein

$$f_{NADH}([NADH],[NAD^+]) = \frac{\frac{[NADH]}{K_M^{NADH}}}{1+\frac{[NADH]}{K_M^{NADH}}+\frac{[NAD^+]}{1028.6}} \text{ in which } K_M^{NADH} = \frac{10.6}{1+e^{-\frac{f_Q-174.1}{681.2}}} \ ;$$

$$f_Q([Q],[QH_2]) = \frac{\frac{[Q]}{11.6}}{1+\frac{[Q]}{11.6}*I_{S,Q,QH_2}+\frac{[QH_2]}{16.3}*I_{S,Q,QH_2}} \text{ in which } I_{S,Q,QH_2} = 1 + \frac{[Q]}{719128.5} + \frac{[QH_2]}{36.2} \ ;$$

and

$$fo_2([O_2]) = \frac{\dfrac{[O_2]}{4871.0}}{\left(1 + \dfrac{[NAD^+]}{26.4}\right)\left(1 + 5f_Q\right)}$$

c) calculating a second ROS appearance rate $V_{2\,Ros}$ of ROS in said sample

$$V_{2\,Ros} = 3579.29 * f_{QH_{2o}} * f_{cytC_{ox}} * (1 + \frac{[Q]}{K_M^{Q_o}}) * f_{O_2}$$

wherein

$$f_{QH_{2o}}([QH_2], [Q]) = \frac{\dfrac{[QH_2]}{K_M^{QH_{2o}}}}{1 + \dfrac{[QH_2]}{K_M^{QH_{2o}}} + \dfrac{[Q]}{43.08}} \text{ in which } K_M^{QH_{2o}} = \frac{184.77}{1 + e^{-\dfrac{f_{cytC_{ox}} - 23.05}{472.12}}}$$

$$f_{cytC_{ox}}([cytC_{ox}], [cytC_{red}]) = \frac{\dfrac{[cytC_{ox}]}{K_M^{cytC_{ox}}}}{1 + \dfrac{[cytC_{ox}]}{8.11} + \dfrac{[cytC_{red}]}{419.16}} \text{ and } f_{Q_i}([Q], [QH_2]) = \frac{\dfrac{[Q]}{K_M^{Q_i}}}{1 + \dfrac{[Q]}{225.21} + \dfrac{[QH_2]}{397.14}},$$

and

$$fo_2([O_2]) = \frac{\dfrac{[O_2]}{914.51}}{1 + 22.02 f_{Q_i} + 173.54 f_{cytC_{ox}}}$$

d) obtaining the ROS appearance rate such that

$$V_{Ros} = V_{1Ros} + V_{2\,Ros}.$$

In other words, the invention relates to a method for quantifying, preferably invitro, the reactive oxygen species, or ROS, appearance rate in a biological sample, the method comprising:

a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

- the reduced form of Nicotinamide adenine dinucleotide, or NADH,
- the oxidized form of Nicotinamide adenine dinucleotide, or NAD+,
- quinone, or Q
- quinol, or $QH_2$,
- molecular oxygen, or $O_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,

- [QH$_2$], corresponding to the amount of quinol measured in said biological sample,
- [O$_2$], corresponding to the amount of molecular oxygen measured in said biological sample,
- [CytC$_{Red}$], corresponding to the amount of reduced form of cytochrome C measured in said biological sample,
- [CytC$_{ox}$], corresponding to the amount of oxidized form of cytochrome C measured in said biological sample,

b) calculating a first ROS appearance rate $V_{1\,Ros}$ of ROS in said sample

$$V_{1\,Ros} = 1727.2 \; * f_{NADH} * f_{O_2}$$

wherein

$$f_{NADH}([NADH], [NAD^+]) = \frac{\dfrac{[NADH]}{10.6}}{1 + \dfrac{\frac{[NADH]}{10.6}}{1 + e^{-\frac{f_Q - 174.1}{681.2}}} + \dfrac{[NAD^+]}{1028.6}}$$

$$f_Q([Q], [QH_2]) = \frac{\dfrac{[Q]}{11.6}}{1 + \dfrac{[Q]}{11.6} * (1 + \dfrac{[Q]}{719128.5} + \dfrac{[QH_2]}{36.2}) + \dfrac{[QH_2]}{16.3} * (1 + \dfrac{[Q]}{719128.5} + \dfrac{[QH_2]}{36.2})}$$

and

$$f_{O_2}([O_2]) = \frac{\dfrac{[O_2]}{4871.0}}{\left(1 + \dfrac{[NAD^+]}{26.4}\right)\left(1 + 5f_Q\right)}$$

c) calculating a second ROS appearance rate $V_{2\,Ros}$ of ROS in said sample

$$V_{2\,Ros} = 3579.29 * f_{QH_{2o}} * f_{cytC_{ox}} * (1 + \frac{[Q]}{K_M^{Q_o}}) * f_{O_2}$$

wherein

$$f_{QH_{2o}}([QH_2], [Q]) = \frac{\dfrac{[QH_2]}{184,77}}{1 + \dfrac{\frac{[QH_2]}{184.77}}{1 + e^{-\frac{f_{cytC_{ox}} - 23.05}{472.12}}} + \dfrac{[Q]}{43.08}}$$

$$f_{cytC_{ox}}([cytC_{ox}], [cytC_{red}]) = \frac{\dfrac{[cytC_{ox}]}{K_M^{cytC_{ox}}}}{1 + \dfrac{[cytC_{ox}]}{8.11} + \dfrac{[cytC_{red}]}{419.16}}$$

$$f_{Q_i}([Q],[QH_2]) = \frac{\dfrac{[Q]}{K_M^{Q_i}}}{1 + \dfrac{[Q]}{225.21} + \dfrac{[QH_2]}{397.14}}$$

and

$$f_{O_2}([O_2]) = \frac{\dfrac{[O_2]}{914.51}}{1 + 22.02 f_{Q_i} + 173.54 f_{cytC_{ox}}}$$

d) obtaining the ROS appearance rate such that

$$V_{Ros} = V_{1Ros} + V_{2\,Ros.}$$

Advantageously, the invention relates to the method as defined above, wherein the method comprising:

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH],[NAD^+]) = \frac{\dfrac{[NADH]}{K_M^{NADH}}}{1 + \dfrac{[NADH]}{K_M^{NADH}} * I_{s,NADH} + \dfrac{[NAD^+]}{K_M^{NAD^+}} * I_{s,NADH}}$$

in which

$$K_M^{NADH} = \frac{K_{M,0}^{NADH}}{1 + e^{-\frac{f_Q - k_1}{k_2}}} \text{ and } I_{s,NADH} = 1 + \frac{[NADH]}{K_{is}^{NADH}};$$

$$f_Q([Q],[QH_2]) = \frac{\dfrac{[Q]}{K_M^Q}}{1 + \dfrac{[Q]}{K_M^Q} * I_{s,Q,QH_2} + \dfrac{[QH_2]}{K_M^{QH_2}} * I_{s,Q,QH_2}} \text{ in which } I_{s,Q,QH_2} = 1 + \frac{[Q]}{K_{is}^Q} + \frac{[QH_2]}{K_{is}^{QH_2}};$$

$$f_{O_2}([O_2]) = \frac{\dfrac{[O_2]}{K_{O_2}}}{\left(1 + \dfrac{[NAD^+]}{K_i^{NAD^+}}\right)\left(1 + \alpha f_Q\right)}$$

wherein $V^+ = 2121,1$ mmol/min/mg ; $K_{M,0}^{NADH} = 8,1$ μM, $K_1 = 185,2$, $K_2 = 1021,2$, $K_M^{NAD^+} = 625,8$ μM, $K_M^Q = 15,7$ μM, $K_M^{QH2} = 26$ μM, $K_{is}^Q = 1066907,0$ μM, $K_{is}^{QH2} = 48,6$ μM, $K_{is}^{NADH} = 165076,9$ μM $K_i^{NAD^+} = 34,3$ $K_{O2} = 9551,4$ et $\alpha = 3,3$, and
c) calculating a second ROS appearance rate $V_{2\,Ros}$ of ROS in said sample

$$V_{2\,Ros} = V2^+ * f_{QH_{2o}} * f_{cytC_{ox}} * (1 + \frac{[Q]}{K_M^{Qo}}) * f_{O_2}$$

wherein

$$f_{QH_{2o}}([QH_2], [Q]) = \frac{\frac{[QH_2]}{K_M^{QH_{2o}}}}{1 + \frac{[QH_2]}{K_M^{QH_{2o}}} + \frac{[Q]}{K_M^{Qo}}} \text{ in which } K_M^{QH_{2o}} = \frac{K_{M,0}^{QH_{2o}}}{1 + e^{\frac{f_{cytC_{ox}} - k_1}{k_2}}}$$

$$f_{cytC_{ox}}([cytC_{ox}], [cytC_{red}]) = (100 - \%saturation\ Antimycine) \frac{\frac{[cytC_{ox}]}{K_M^{cytC_{ox}}}}{1 + \frac{[cytC_{ox}]}{K_M^{cytC_{ox}}} + \frac{[cytC_{red}]}{K_M^{cytC_{red}}}}$$

and

$$f_{Q_i}([Q], [QH_2]) = (100 - \%saturation\ Antimycine) \frac{\frac{[Q]}{K_M^{Q_i}}}{1 + \frac{[Q]}{K_M^{Q_i}} + \frac{[QH_2]}{K_M^{QH_{2i}}}},$$

and

$$f_{O_2}([O_2]) = (\%saturation\ Antimycine) \frac{\frac{[O_2]}{K'_{O_2}}}{1 + \alpha' f_{Q_i} + \beta f_{cytC_{ox}}}$$

wherein

$V2^+$ = 3909.06 mmol/min/mg ;
$K_1$ = 19.96, $K_2$= 341.15,
$K_{M,0}^{QH20}$ = 157.61 $\mu$M, $K_M^{QH2i}$ = 478.63,
$K_M^{Qo}$= 50.49 $\mu$M, $K_M^{Qi}$ = 280.26$\mu$M,
$K_M^{cytCox}$ = 10.98$\mu$M, $K_M^{cytCred}$= 248.18$\mu$M,
$K'_{O2}$ = 760.78,
$\alpha'$= 38.66 and $\beta$ = 212.03

d) obtaining the ROS appearance rate such that

$$\text{VRos} = V_{1Ros} + V_{2\,Ros.}$$

In other words, advantageously, the invention relates to the method as defined above, wherein the method comprising:

a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

- the reduced form of Nicotinamide adenine dinucleotide, or NADH,

- the oxidized form of Nicotinamide adenine dinucleotide, or NAD+,
- quinone, or Q
- quinol, or $QH_2$,
- molecular oxygen, or $O_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,
- [$QH_2$], corresponding to the amount of quinol measured in said biological sample,
- [$O_2$], corresponding to the amount of molecular oxygen measured in said biological sample,
- [$CytC_{Red}$], corresponding to the amount of reduced form of cytochrome C measured in said biological sample,
- [$CytC_{ox}$], corresponding to the amount of oxidized form of cytochrome C measured in said biological sample,

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH],[NAD^+]) = \frac{\frac{[NADH]}{K_M^{NADH}}}{1+\frac{[NADH]}{K_M^{NADH}}*I_{s,NADH}+\frac{[NAD^+]}{K_M^{NAD^+}}*I_{s,NADH}}$$

in which

$$K_M^{NADH} = \frac{K_{M,0}^{NADH}}{1+e^{-\frac{f_Q-k_1}{k_2}}} \text{ and } I_{s,NADH} = 1 + \frac{[NADH]}{K_{is}^{NADH}}\;;$$

$$f_Q([Q],[QH_2]) = \frac{\frac{[Q]}{K_M^Q}}{1+\frac{[Q]}{K_M^Q}*I_{s,Q,QH_2}+\frac{[QH_2]}{K_M^{QH_2}}*I_{s,Q,QH_2}} \text{ in which } I_{s,Q,QH_2} = 1 + \frac{[Q]}{K_{is}^Q} + \frac{[QH_2]}{K_{is}^{QH_2}};$$

$$f_{O_2}([O_2]) = \frac{\frac{[O_2]}{K_{O_2}}}{\left(1+\frac{[NAD^+]}{K_i^{NAD^+}}\right)\left(1+\alpha f_Q\right)}$$

wherein $V^+$ = 2121.1 mmol/min/mg ; $K_{M,0}^{NADH}$ = 8.1 $\mu$M, $K_1$ = 185.2, $K_2$= 1021.2, $K_M^{NAD^+}$ = 625.8 $\mu$M, $K_M^Q$= 15.7 $\mu$M, $K_M^{QH2}$ = 26 $\mu$M, $K_{is}^Q$= 1066907.0 $\mu$M, $K_{is}^{QH2}$ = 48.6 $\mu$M, $K_{is}^{NADH}$ = 165076.9 $\mu$M $K_i^{NAD^+}$= 34.3 $K_{O2}$ = 9551.4 et $\alpha$= 3.3,

c) calculating a second ROS appearance rate $V_{2\,Ros}$ of ROS in said sample

$$V_{2\,Ros} = V2^+ * f_{QH_{2o}} * f_{cytC_{ox}} * \left(1 + \frac{[Q]}{K_M^{Q_o}}\right) * f_{O_2}$$

wherein

$$f_{QH_{2o}}([QH_2], [Q]) = \frac{\frac{[QH_2]}{K_M^{QH_{2o}}}}{1 + \frac{[QH_2]}{K_M^{QH_{2o}}} + \frac{[Q]}{K_M^{Q_o}}} \text{ in which } K_M^{QH_{2o}} = \frac{K_{M,0}^{QH_{2o}}}{1 + e^{\frac{f_{cytC_{ox}} - k_1}{k_2}}}$$

$$f_{cytC_{ox}}([cytC_{ox}], [cytC_{red}]) = (100 - \%saturation\,Antimycine)\frac{\frac{[cytC_{ox}]}{K_M^{cytC_{ox}}}}{1 + \frac{[cytC_{ox}]}{K_M^{cytC_{ox}}} + \frac{[cytC_{red}]}{K_M^{cytC_{red}}}}$$

and

$$f_{Q_i}([Q], [QH_2]) = (100 - \%saturation\,Antimycine)\frac{\frac{[Q]}{K_M^{Q_i}}}{1 + \frac{[Q]}{K_M^{Q_i}} + \frac{[QH_2]}{K_M^{QH_{2i}}}},$$

and

$$f_{O_2}([O_2]) = (\%saturation\,Antimycine)\frac{\frac{[O_2]}{K'_{O_2}}}{1 + \alpha' f_{Q_i} + \beta f_{cytC_{ox}}}$$

wherein

$V2^+$ = 3909.06 mmol/min/mg ;
$K_1$ = 19.96, $K_2$= 341.15,
$K_{M,0}^{QH2O}$ = 157.61 µM, $K_M^{QH2i}$ = 478.63,
$K_M^{Qo}$= 50.49 µM, $K_M^{Qi}$ = 280.26µM,
$K_M^{cytCox}$ = 10.98µM, $K_M^{cytCred}$= 248.18µM,
$K'_{O2}$ = 760.78,
$\alpha'$= 38.66 and $\beta$ = 212.03

d) obtaining the ROS appearance rate such that

$$\text{VRos} = V_{1Ros} + V_{2\,Ros.}$$

In this advantageous embodiment, the inventors obtain better results when the formula for obtaining the ROS apparition rate is ameliorated by using the above mentioned formulas. Here for model 3 of complex III, the inventors have an optional input, the % of saturation of Antimycin.

More advantageously, the invention relates to the method as defined above, wherein the activity of CI is determined by the following formula:

$$V_{NAD^+} = V^+ * f_{NADH} * f_Q.$$

In addition to the apparition of ROS, the method proposed by the inventors allows to evaluate the activity of the Complex I. By determining the activity of the complex, it is possible to assess a possible dysfunction in the complex and thus possibly propose a therapy or cure for correcting the default.

More advantageously, the invention relates to the method as defined above, wherein the activity of CIII is determined by the following formula:

$$V_{cytC_{ox}} = V_{cytC_{ox},1} + V_{cytC_{ox},2} = V^+ * f_{QH_{2o}} * f_{cytC_{ox}} * \left( f_{Q_i}^{1/2} + \frac{f_{cytC_{ox}}}{1 + \alpha f_{Q_i}} \right).$$

Similarly, in addition to the apparition of ROS, the method proposed by the inventors allows to evaluate the activity of the Complex III. By determining the activity of the complex, it is possible to assess a possible dysfunction in the complex and thus possibly propose a therapy or cure for correcting the default.

The Invention also relates to a computer program comprising instructions which, when the program is executed by a computer, causes the computer to carry out steps b) to d) of the method as defined above.

The computer program according to the invention is able, when implemented on a computer to calculate the ROS appearance rate and the activities of both complexes, by implementing the above mentioned formulas, based on the data relating to the concentrations of

- [NADH], corresponding to the amount of NADH measured in said biological sample,
- [NAD+], corresponding to the amount of NAD+ measured in said biological sample,
- [Q], corresponding to the amount of quinone measured in said biological sample,
- [QH$_{2}$], corresponding to the amount of quinol measured in said biological sample, and
- [O$_{2}$], corresponding to the amount of molecular oxygen measured in said biological sample.

Every support can be used such as personal computer, portable computer, tablet smartphone, or any dice available for computing the data according to the above mentioned formulas.

The invention also relates to a method, for determining, preferably *in vitro,* ROS detoxifying enzyme activity in a sample of an individual, the method comprising:

- quantifying the ROS appearance rate in the biological sample by carrying out the method as defined above, in order to obtain a calculated rate,
- comparing the calculated rate with a reference ROS appearance rate, said reference ROS appearance rate being obtained from a reference sample, the reference sample being of the same nature as the sample, in order to obtain a ratio R between the ROS appearance rate and the reference ROS appearance rate, and
- concluding that

    * if R is lower than 0.85, then the sample contains efficient ROS detoxifying enzyme activity, in order to detoxify the sample during an oxidative stress,
    * otherwise the sample does not contain efficient ROS detoxifying enzyme activity, in order to detoxify the sample during an oxidative stress.

With the method as defined above, it is possible to evaluate the detoxifying activity of an individual, and therefore to predict if, when submitted to an oxidative stress, the individual will be able to efficiently detoxify cells, or if an accumulation of ROS is expected, with the drawbacks thereof.

in other words, the invention also relates to a method, for determining, preferably *in vitro,* ROS detoxifying enzyme activity in a sample of an individual, the method comprising:

- quantifying the ROS appearance rate in the biological sample by carrying out the method as defined above, in order to obtain a calculated rate, i.e.

    a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

    - the reduced form of Nicotinamide adenine dinucleotide, or NADH,
    - the oxidized form of Nicotinamide adenine dinucleotide, or NAD+,
    - quinone, or Q

- quinol, or $QH_2$,
- molecular oxygen, or $O_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,
- [$QH_2$], corresponding to the amount of quinol measured in said biological sample,
- [$O_2$], corresponding to the amount of molecular oxygen measured in said biological sample,
- [$CytC_{Red}$], corresponding to the amount of reduced form of cytochrome C measured in said biological sample,
- [$CytC_{ox}$], corresponding to the amount of oxidized form of cytochrome C measured in said biological sample,

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH],[NAD^+]) = \frac{\frac{[NADH]}{K_M^{NADH}}}{1+\frac{[NADH]}{K_M^{NADH}}+\frac{[NAD^+]}{K_M^{NAD^+}}} \text{ in which } K_M^{NADH} = \frac{K_{M,0}^{NADH}}{1+e^{-\frac{f_Q-k_1}{k_2}}} \; ;$$

$$f_Q([Q],[QH_2]) = \frac{\frac{[Q]}{K_M^Q}}{1+\frac{[Q]}{K_M^Q}*I_{s,Q,QH_2}+\frac{[QH_2]}{K_M^{QH_2}}*I_{s,Q,QH_2}} \text{ in which } I_{s,Q,QH_2} = 1 + \frac{[Q]}{K_{is}^Q} + \frac{[QH_2]}{K_{is}^{QH_2}} \; ;$$

and

$$f_{O_2}([O_2]) = \frac{\frac{[O_2]}{K_{O_2}}}{\left(1+\frac{[NAD^+]}{K_i^{NAD^+}}\right)\left(1+\alpha f_Q\right)}$$

wherein

$V^+$ = 1727.2 mmol/min/mg ;
$K_{M,0}^{NADH}$ = 10,6
$K_1$ = 174.1, $K_2$ = 681.2,
$K_M^{NAD+}$ = 1028.6 $\mu$M,
$K_M^Q$= 11.6 $\mu$M, $K_{is}^Q$= 719128.5 $\mu$M,
$K_M^{QH2}$ = 16.3 $\mu$M, $K_{is}^{QH2}$ = 36.2 $\mu$M,
$K_i^{NAD+}$ = 26.4
$K_{O2}$ = 4871.0 and
$\alpha$= 5.

c) calculating a second ROS appearance rate $V_{2\,Ros}$ of ROS in said sample

$$V_{2\,Ros} = V2^+ * f_{QH_{2o}} * f_{cytC_{ox}} * (1 + \frac{[Q]}{K_M^{Q_o}}) * f_{O_2}$$

wherein

$$f_{QH_{2o}}([QH_2],[Q]) = \frac{\frac{[QH_2]}{K_M^{QH_{2o}}}}{1+\frac{[QH_2]}{K_M^{QH_{2o}}}+\frac{[Q]}{K_M^{Q_o}}} \text{ in which } K_M^{QH_{2o}} = \frac{K_{M,0}^{QH_{2o}}}{1+e^{\frac{f_{cytC_{ox}}-k_1}{k_2}}}$$

$$f_{cytC_{ox}}([cytC_{ox}],[cytC_{red}]) = \frac{\frac{[cytC_{ox}]}{K_M^{cytC_{ox}}}}{1+\frac{[cytC_{ox}]}{K_M^{cytC_{ox}}}+\frac{[cytC_{red}]}{K_M^{cytC_{red}}}} \text{ and } f_{Q_i}([Q],[QH_2]) = \frac{\frac{[Q]}{K_M^{Q_i}}}{1+\frac{[Q]}{K_M^{Q_i}}+\frac{[QH_2]}{K_M^{QH_{2i}}}},$$

and

$$f_{O_2}([O_2]) = \frac{\frac{[O_2]}{K'_{O_2}}}{1+\alpha' f_{Q_i}+\beta f_{cytC_{ox}}}$$

wherein

$V2^+$ = 3579.29 mmol/min/mg ;
$K_1$ = 23.05, $K_2$= 472.12,
$K_{M,0}^{QH2o}$ =184.77 $\mu$M, $K_M^{QH2i}$ = 397,14,
$K_M^{Qo}$= 43.08 $\mu$M, $K_M^{Qi}$ = 225.21 $\mu$M,
$K_M^{cytCox}$ = 8.11 $\mu$M, $K_M^{cytCred}$= 419.16$\mu$M,
$K'_{O2}$ = 914.51,
$\alpha'$= 22.02 and $\beta$=173.54

d) obtaining the ROS appearance rate such that

$$VRos = V_{1Ros} + V_{2\,Ros,}$$

- comparing the calculated rate with a reference ROS appearance rate, said reference ROS appearance rate being obtained from a reference sample, the reference sample being of the same nature as the sample, in order to obtain a ratio R between the ROS appearance rate and the reference ROS appearance rate, and
- concluding that

    * if R is lower than 0.85, then the sample contains efficient ROS detoxifying enzyme activity, in order to detoxify the sample during an oxidative stress,
    * otherwise the sample does not contain efficient ROS detoxifying enzyme activity, in order to detoxify the sample during an oxidative stress.

Advantageously, the invention relates the above mentioned method, the method comprising:

- quantifying the ROS appearance rate in the biological sample by carrying out the method as defined above, in order to obtain a calculated rate,
- comparing the calculated rate with a reference ROS appearance rate, said reference ROS appearance rate being obtained from a reference sample, the reference sample being of the same nature as the sample, in order to obtain a ratio R between the ROS appearance rate and the reference ROS appearance rate, and
- concluding that

* if R is lower than 0.85, then the sample contains efficient ROS detoxifying enzyme activity, in order to detoxify the sample during an oxidative stress,
* otherwise the sample does not contain efficient ROS detoxifying enzyme activity, in order to detoxify the sample during an oxidative stress.

With the method as defined above, it is possible to evaluate the detoxifying activity of an individual, and therefore to predict if, when submitted to an oxidative stress, the individual will be able to efficiently detoxify cells, or if an accumulation of ROS is expected, with the drawbacks thereof.

In other words, the invention also relates to a method, for determining, preferably in vitro, ROS detoxifying enzyme activity in a sample of an individual, the method comprising:

- quantifying the ROS appearance rate in the biological sample by carrying out the method as defined above, in order to obtain a calculated rate, i.e.

a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

- the reduced form of Nicotinamide adenine dinucleotide, or NADH,
- the oxidized form of Nicotinamide adenine dinucleotide, or NAD+,
- quinone, or Q
- quinol, or $QH_2$,
- molecular oxygen, or $O_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,
- [$QH_2$], corresponding to the amount of quinol measured in said biological sample,
- [$O_2$], corresponding to the amount of molecular oxygen measured in said biological sample,
- [$CytC_{Red}$], corresponding to the amount of reduced form of cytochrome C measured in said biological sample,
- [$CytC_{ox}$], corresponding to the amount of oxidized form of cytochrome C measured in said biological sample,

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH],[NAD^+]) = \frac{\frac{[NADH]}{K_M^{NADH}}}{1+\frac{[NADH]}{K_M^{NADH}}*I_{s,NADH}+\frac{[NAD^+]}{K_M^{NAD^+}}*I_{s,NADH}}$$

in which

$$K_M^{NADH} = \frac{K_{M,0}^{NADH}}{1+e^{-\frac{f_Q-k_1}{k_2}}} \text{ and } I_{s,NADH} = 1+\frac{[NADH]}{K_{is}^{NADH}} \; ;$$

$$f_Q([Q],[QH_2]) = \frac{\frac{[Q]}{K_M^Q}}{1+\frac{[Q]}{K_M^Q}*I_{s,Q,QH_2}+\frac{[QH_2]}{K_M^{QH_2}}*I_{s,Q,QH_2}} \text{ in which } I_{s,Q,QH_2} = 1 + \frac{[Q]}{K_{is}^Q} + \frac{[QH_2]}{K_{is}^{QH_2}};$$

$$f_{O_2}([O_2]) = \frac{\frac{[O_2]}{K_{O_2}}}{\left(1 + \frac{[NAD^+]}{K_i^{NAD^+}}\right)\left(1 + \alpha f_Q\right)}$$

wherein $V^+$ = 2121,1 mmol/min/mg ; $K_{M,0}^{NADH}$ = 8,1 μM, $K_1$ = 185,2, $K_2$= 1021,2, $K_M^{NAD^+}$ = 625,8 μM, $K_M^Q$= 15,7 μM, $K_M^{QH2}$ = 26 μM, $K_{is}^Q$= 1066907,0 μM, $K_{is}^{QH2}$ = 48,6 μM, $K_{is}^{NADH}$ = 165076,9 μM $K_i^{NAD+}$= 34,3 $K_{O2}$ = 9551,4 et α= 3,3.

c) calculating a second ROS appearance rate $V_{2\,Ros}$ of ROS in said sample

$$V_{2\,Ros} = V2^+ * f_{QH_{2o}} * f_{cytC_{ox}} * (1 + \frac{[Q]}{K_M^{Q_o}}) * f_{O_2}$$

wherein

$$f_{QH_{2o}}([QH_2],[Q]) = \frac{\frac{[QH_2]}{K_M^{QH_{2o}}}}{1+\frac{[QH_2]}{K_M^{QH_{2o}}}+\frac{[Q]}{K_M^{Q_o}}} \text{ in which } K_M^{QH_{2o}} = \frac{K_{M,0}^{QH_{2o}}}{1+e^{\frac{f_{cytC_{ox}}-k_1}{k_2}}}$$

$$f_{cytC_{ox}}([cytC_{ox}],[cytC_{red}]) = \frac{\frac{[cytC_{ox}]}{K_M^{cytC_{ox}}}}{1+\frac{[cytC_{ox}]}{K_M^{cytC_{ox}}}+\frac{[cytC_{red}]}{K_M^{cytC_{red}}}} \text{ and } f_{Q_i}([Q],[QH_2]) = \frac{\frac{[Q]}{K_M^{Q_i}}}{1+\frac{[Q]}{K_M^{Q_i}}+\frac{[QH_2]}{K_M^{QH2i}}},$$

and

$$f_{O_2}([O_2]) = \frac{\frac{[O_2]}{K'_{O_2}}}{1 + \alpha' f_{Q_i} + \beta f_{cytC_{ox}}}$$

wherein

$V2^+$ = 3909.06 mmol/min/mg ;
$K_1$ = 19.96, $K_2$= 341.15,
$K_{M,0}^{QH2o}$ = 157.61 μM, $K_M^{QH2i}$ = 478.63,
$K_M^{Qo}$= 50.49 μM, $K_M^{Qi}$ = 280.26μM,
$K_M^{cytCox}$ = 10.98μM, $K_M^{cytCred}$= 248.18μM,
$K'_{O2}$ = 760.78,
α'= 38.66 and β = 212.03

d) obtaining the ROS appearance rate such that

$$VRos = V_{1Ros} + V_{2Ros,}$$

- comparing the calculated rate with a reference ROS appearance rate, said reference ROS appearance rate being obtained from a reference sample, the reference sample being of the same nature as the sample, in order to obtain a ratio R between the ROS appearance rate and the reference ROS appearance rate, and
- concluding that

  * if R is lower than 0.85, then the sample contains efficient ROS detoxifying enzyme activity, in order to detoxify the sample during an oxidative stress,
  * otherwise the sample does not contain efficient ROS detoxifying enzyme activity, in order to detoxify the sample during an oxidative stress.

More advantageously, the invention relates to the above-described method, wherein

* if R is lower than 0,8, then the sample contains efficient ROS detoxifying enzyme activity, in order to detoxify the sample during an oxidative stress,
* otherwise the sample does not contain efficient ROS detoxifying enzyme activity, in order to detoxify the sample during an oxidative stress.

Advantageously, the invention also relates to a method, for determining, preferably *in vitro*, ROS detoxifying enzyme activity in a sample of an individual, the method comprising:

- quantifying the ROS appearance rate in the biological sample by carrying out the method as defined above, in order to obtain a calculated rate, i.e.

  a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

  - the reduced form of Nicotinamide adenine dinucleotide, or NADH,
  - the oxidized form of Nicotinamide adenine dinucleotide, or NAD+,
  - quinone, or Q
  - quinol, or $QH_2$,
  - molecular oxygen, or $O_2$,
  - the reduced form of cytochrome C, or cytCred, and
  - the oxidized form of cytochrome C, or cytCox,

  in order to obtain a respective concentration value

  - [NADH], corresponding to the amount of NADH measured in said biological sample,
  - [NAD+], corresponding to the amount of NAD+ measured in said biological sample,
  - [Q], corresponding to the amount of quinone measured in said biological sample,
  - $[QH_2]$, corresponding to the amount of quinol measured in said biological sample,
  - $[O_2]$, corresponding to the amount of molecular oxygen measured in said biological sample,
  - $[CytC_{Red}]$, corresponding to the amount of reduced form of cytochrome C measured in said biological sample,
  - $[CytC_{ox}]$, corresponding to the amount of oxidized form of cytochrome C measured in said biological sample,

  b) calculating a first ROS appearance rate $V_{1\,Ros}$ of ROS in said sample

$$V_{1\,Ros} = V^+ * f_{NADH} * f_{O_2}$$

  wherein

$$f_{NADH}([NADH],[NAD^+]) = \frac{\frac{[NADH]}{K_M^{NADH}}}{1+\frac{[NADH]}{K_M^{NADH}}*I_{s,NADH}+\frac{[NAD^+]}{K_M^{NAD^+}}*I_{s,NADH}} \text{ in which}$$

$$K_M^{NADH} = \frac{K_{M,0}^{NADH}}{1+e^{-\frac{f_Q-k_1}{k_2}}} \text{ and } I_{s,NADH} = 1+\frac{[NADH]}{K_{is}^{NADH}} \; ;$$

$$f_Q([Q],[QH_2]) = \frac{\frac{[Q]}{K_M^Q}}{1+\frac{[Q]}{K_M^Q}*I_{s,Q,QH_2}+\frac{[QH_2]}{K_M^{QH_2}}*I_{s,Q,QH_2}} \text{ in which } I_{s,Q,QH_2} = 1+\frac{[Q]}{K_{is}^Q}+\frac{[QH_2]}{K_{is}^{QH_2}};$$

$$f_{O_2}([O_2]) = \frac{\frac{[O_2]}{K_{O_2}}}{\left(1+\frac{[NAD^+]}{K_i^{NAD^+}}\right)\left(1+\alpha f_Q\right)}$$

wherein $V^+$ = 2121,1 mmol/min/mg ; $K_{M,0}^{NADH}$ = 8,1 μM, $K_1$ = 185,2, $K_2$= 1021,2, $K_M^{NAD^+}$ = 625,8 μM, $K_M^Q$= 15,7 μM, $K_M^{QH2}$ = 26 μM, $K_{is}^Q$= 1066907,0 μM, $K_{is}^{QH2}$ = 48,6 μM, $K_{is}^{NADH}$ = 165076,9 μM $K_i^{NAD+}$= 34,3 $K_{O2}$ = 9551,4 et α= 3,3.

c) calculating a second ROS appearance rate $V_{2\,Ros}$ of ROS in said sample

$$V_{2\,Ros} = V2^+ * f_{QH_{2o}} * f_{cytC_{ox}} * (1+\frac{[Q]}{K_M^{Q_o}}) * f_{O_2}$$

wherein

$$f_{QH_{2o}}([QH_2],[Q]) = \frac{\frac{[QH_2]}{K_M^{QH_{2o}}}}{1+\frac{[QH_2]}{K_M^{QH_{2o}}}+\frac{[Q]}{K_M^{Q_o}}} \text{ in which } K_M^{QH_{2o}} = \frac{K_{M,0}^{QH_{2o}}}{1+e^{-\frac{f_{cytC_{ox}}-k_1}{k_2}}}$$

$$f_{cytC_{ox}}([cytC_{ox}],[cytC_{red}]) = \frac{\frac{[cytC_{ox}]}{K_M^{cytC_{ox}}}}{1+\frac{[cytC_{ox}]}{K_M^{cytC_{ox}}}+\frac{[cytC_{red}]}{K_M^{cytC_{red}}}} \text{ and } f_{Q_i}([Q],[QH_2]) = \frac{\frac{[Q]}{K_M^{Q_i}}}{1+\frac{[Q]}{K_M^{Q_i}}+\frac{[QH_2]}{K_M^{QH_{2i}}}},$$

and

$$f_{o_2}([O_2]) = \frac{\frac{[O_2]}{K'_{O_2}}}{1+\alpha' f_{Q_i}+\beta f_{cytC_{ox}}}$$

wherein

$V2^+$ = 3909.06 mmol/min/mg ;
$K_1$ = 19.96, $K_2$= 341.15,
$K_{M,0}^{QH20}$ = 157.61 $\mu$M, $K_M^{QH2i}$ = 478.63,
$K_M^{Qo}$= 50.49 $\mu$M, $K_M^{Qi}$ = 280.26$\mu$M,
$K_M^{cytCox}$ = 10.98$\mu$M, $K_M^{cytCred}$= 248.18$\mu$M,
$K'_{O2}$ = 760.78,
$\alpha'$= 38.66 and $\beta$ = 212.03

d) obtaining the ROS appearance rate such that

$$VRos = V_{1\,Ros} + V_{2\,Ros,}$$

- comparing the calculated rate with a reference ROS appearance rate, said reference ROS appearance rate being obtained from a reference sample, the reference sample being of the same nature as the sample, in order to obtain a ratio R between the ROS appearance rate and the reference ROS appearance rate, and
- concluding that

  * if R is lower than 0.8, then the sample contains efficient ROS detoxifying enzyme activity, in order to detoxify the sample during an oxidative stress,
  * otherwise the sample does not contain efficient ROS detoxifying enzyme activity, in order to detoxify the sample during an oxidative stress.

In one other aspect, the invention relates to a method for determining *in vitro* the response of a patient to a compound liable to reduce ROS amount in a biological sample, the method comprising:

- quantifying the ROS appearance rate in the biological sample, after the administration of the compound to the patient, by carrying out the method as defined in anyone of claims 1 to 4, in order to obtain a final ROS rate,
- comparing the initial ROS rate and the final ROS rate in order to obtain a ratio R between the final ROS and the initial ROS rate,
- concluding that

  * if R is lower than 0.85, then the compound is able to reduce ROS amount in the sample,
  * otherwise the sample is not able to reduce ROS amount in the sample.

With the above mentioned method, it is possible to evaluate the effect of a compound in the modulation of ROS production in a cell or a sample of an individual.

By measuring the ROS production by implementing the method according to the invention at a determined time (before any treatment) and after a period of time during which a treatment is administered, it is possible to evaluate if said treatment is efficient or not on modulating ROS production, preferably ROS reduction. Any difference of about 15% or more compared to the initial value will be considered as significantly different and associated with an effect of the treatment. By contrast, if the difference between the initial value of ROS amount and the final value of the ROS amount (after treatment) is lower than 15%, it is considered that he treatment has no effect on ROS production.

Advantageously, the invention relates to a method for determining in vitro the response of a patient to a compound liable to reduce ROS amount in a biological sample, the method comprising:

- quantifying the ROS appearance rate in the biological sample, before the administration of the compound to the patient, by carrying out the method as defined above, in order to obtain an initial ROS rate, the method comprising:

  - quantifying the ROS appearance rate in the biological sample by carrying out the method as defined above, in order to obtain a calculated rate, i.e.

    a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

    - the reduced form of Nicotinamide adenine dinucleotide, or NADH,
    - the oxidized form of Nicotinamide adenine dinucleotide, or NAD+,

- quinone, or Q
- quinol, or QH$_2$,
- molecular oxygen, or O$_2$,
- the reduced form of cytochrome C, or cytCred, and
- the oxidized form of cytochrome C, or cytCox,

in order to obtain a respective concentration value

- [NADH], corresponding to the amount of NADH measured in said biological sample,
- [NAD+], corresponding to the amount of NAD+ measured in said biological sample,
- [Q], corresponding to the amount of quinone measured in said biological sample,
- [QH$_2$], corresponding to the amount of quinol measured in said biological sample,
- [O$_2$], corresponding to the amount of molecular oxygen measured in said biological sample,
- [CytC$_{Red}$], corresponding to the amount of reduced form of cytochrome C measured in said biological sample,
- [CytC$_{ox}$], corresponding to the amount of oxidized form of cytochrome C measured in said biological sample,

b) calculating a first ROS appearance rate V$_{1\,Ros}$ of ROS in said sample

$$V_{1\,Ros} = V^+ * f_{NADH} * f_{O_2}$$

wherein

$$f_{NADH}([NADH],[NAD^+]) = \frac{\frac{[NADH]}{K_M^{NADH}}}{1 + \frac{[NADH]}{K_M^{NADH}} * I_{s,NADH} + \frac{[NAD^+]}{K_M^{NAD^+}} * I_{s,NADH}}$$

in which

$$K_M^{NADH} = \frac{K_{M,0}^{NADH}}{1 + e^{-\frac{f_Q - k_1}{k_2}}} \text{ and } I_{s,NADH} = 1 + \frac{[NADH]}{K_{is}^{NADH}} \; ;$$

$$f_Q([Q],[QH_2]) = \frac{\frac{[Q]}{K_M^Q}}{1 + \frac{[Q]}{K_M^Q} * I_{s,Q,QH_2} + \frac{[QH_2]}{K_M^{QH_2}} * I_{s,Q,QH_2}} \text{ in which } I_{s,Q,QH_2} = 1 + \frac{[Q]}{K_{is}^Q} + \frac{[QH_2]}{K_{is}^{QH_2}};$$

$$f_{O_2}([O_2]) = \frac{\frac{[O_2]}{K_{O_2}}}{\left(1 + \frac{[NAD^+]}{K_i^{NAD^+}}\right)\left(1 + \alpha f_Q\right)}$$

wherein $V^+$ = 2121,1 mmol/min/mg ; $K_{M,0}^{NADH}$ = 8,1 µM, $K_1$ = 185,2, $K_2$= 1021,2, $K_M^{NAD^+}$ = 625,8 µM, $K_M^Q$= 15,7 µM, $K_M^{QH2}$ = 26 µM, $K_{is}^Q$= 1066907,0 µM, $K_{is}^{QH2}$ = 48,6 µM, $K_{is}^{NADH}$ = 165076,9 µM $K_i^{NAD^+}$= 34,3 $K_{O2}$ = 9551,4 et $\alpha$= 3,3.

c) calculating a second ROS appearance rate V$_{2\,Ros}$ of ROS in said sample

$$V_{2\,Ros} = V2^+ * f_{QH_{2o}} * f_{cytC_{ox}} * (1 + \frac{[Q]}{K_M^{Q_o}}) * f_{O_2}$$

wherein

$$f_{QH_{2o}}([QH_2], [Q]) = \frac{\frac{[QH_2]}{K_M^{QH_{2o}}}}{1 + \frac{[QH_2]}{K_M^{QH_{2o}}} + \frac{[Q]}{K_M^{Q_o}}} \text{ in which } K_M^{QH_{2o}} = \frac{K_{M,0}^{QH_{2o}}}{1 + e^{\frac{f_{cytC_{ox}} - k_1}{k_2}}}$$

$$f_{cytC_{ox}}([cytC_{ox}], [cytC_{red}]) = \frac{\frac{[cytC_{ox}]}{K_M^{cytC_{ox}}}}{1 + \frac{[cytC_{ox}]}{K_M^{cytC_{ox}}} + \frac{[cytC_{red}]}{K_M^{cytC_{red}}}} \text{ and } f_{Q_i}([Q], [QH_2]) = \frac{\frac{[Q]}{K_M^{Q_i}}}{1 + \frac{[Q]}{K_M^{Q_i}} + \frac{[QH_2]}{K_M^{QH_{2i}}}},$$

and

$$f_{O_2}([O_2]) = \frac{\frac{[O_2]}{K'_{O_2}}}{1 + \alpha' f_{Q_i} + \beta f_{cytC_{ox}}}$$

wherein

$V2^+$ = 3909.06 mmol/min/mg ;
$K_1$ = 19.96, $K_2$ = 341.15,
$K_{M,0}^{QH20}$ = 157.61 $\mu$M, $K_M^{QH2i}$ = 478.63,
$K_M^{Qo}$ = 50.49 $\mu$M, $K_M^{Qi}$ = 280.26$\mu$M,
$K_M^{cytCox}$ = 10.98$\mu$M, $K_M^{cytCred}$ = 248.18$\mu$M,
$K'_{O2}$ = 760.78,
$\alpha'$ = 38.66 and $\beta$ = 212.03

d) obtaining the ROS appearance rate such that

$$\text{VRos} = \text{V}_{1\,Ros} + \text{V}_{2\,Ros,}$$

- quantifying the ROS appearance rate in the biological sample, after the administration of the compound to the patient, by carrying out the method as defined in anyone of claims 1 to 4, in order to obtain a final ROS rate,
- comparing the initial ROS rate and the final ROS rate in order to obtain a ratio R between the final ROS and the initial ROS rate,
- concluding that

   * if R is lower than 0.85, then the compound is able to reduce ROS amount in the sample,
   * otherwise the sample is not able to reduce ROS amount in the sample.

More advantageously, the invention relates to the above-described method, wherein

* if R is lower than 0.8, then the compound is able to reduce ROS amount in the sample,
* otherwise the sample is not able to reduce ROS amount in the sample.

Advantageously, the invention relates to a method for determining in vitro the response of a patient to a compound liable to reduce ROS amount in a biological sample, the method comprising:

- quantifying the ROS appearance rate in the biological sample, before the administration of the compound to the patient, by carrying out the method as defined above, in order to obtain an initial ROS rate, the method comprising:

  - quantifying the ROS appearance rate in the biological sample by carrying out the method as defined above, in order to obtain a calculated rate, i.e.

    a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

    - the reduced form of Nicotinamide adenine dinucleotide, or NADH,
    - the oxidized form of Nicotinamide adenine dinucleotide, or NAD+,
    - quinone, or Q
    - quinol, or $QH_2$,
    - molecular oxygen, or $O_2$,
    - the reduced form of cytochrome C, or cytCred, and
    - the oxidized form of cytochrome C, or cytCox,

    in order to obtain a respective concentration value

    - [NADH], corresponding to the amount of NADH measured in said biological sample,
    - [NAD+], corresponding to the amount of NAD+ measured in said biological sample,
    - [Q], corresponding to the amount of quinone measured in said biological sample,
    - $[QH_2]$, corresponding to the amount of quinol measured in said biological sample,
    - $[O_2]$, corresponding to the amount of molecular oxygen measured in said biological sample,
    - $[CytC_{Red}]$, corresponding to the amount of reduced form of cytochrome C measured in said biological sample,
    - $[CytC_{ox}]$, corresponding to the amount of oxidized form of cytochrome C measured in said biological sample,

    b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH],[NAD^+]) = \frac{\frac{[NADH]}{K_M^{NADH}}}{1+\frac{[NADH]}{K_M^{NADH}}*I_{s,NADH}+\frac{[NAD^+]}{K_M^{NAD^+}}*I_{s,NADH}}$$

in which

$$K_M^{NADH} = \frac{K_{M,0}^{NADH}}{1+e^{-\frac{f_Q-k_1}{k_2}}} \text{ and } I_{s,NADH} = 1 + \frac{[NADH]}{K_{is}^{NADH}} \;;$$

$$f_Q([Q],[QH_2]) = \frac{\frac{[Q]}{K_M^Q}}{1+\frac{[Q]}{K_M^Q}*I_{s,Q,QH_2}+\frac{[QH_2]}{K_M^{QH_2}}*I_{s,Q,QH_2}} \text{ in which } I_{s,Q,QH_2} = 1 + \frac{[Q]}{K_{is}^Q} + \frac{[QH_2]}{K_{is}^{QH_2}};$$

$$f_{O_2}([O_2]) = \frac{\dfrac{[O_2]}{K_{O_2}}}{\left(1 + \dfrac{[NAD^+]}{K_i^{NAD^+}}\right)\left(1 + \alpha f_Q\right)}$$

wherein $V^+$ = 2121,1 mmol/min/mg ; $K_{M,0}^{NADH}$ = 8,1 μM, $K_1$ = 185,2, $K_2$= 1021,2, $K_M^{NAD^+}$ = 625,8 μM, $K_M^Q$= 15,7 μM, $K_M^{QH2}$ = 26 μM, $K_{is}^Q$= 1066907,0 μM, $K_{is}^{QH2}$ = 48,6 μM, $K_{is}^{NADH}$ = 165076,9 μM $K_i^{NAD+}$= 34,3 $K_{O2}$ = 9551,4 et α= 3,3.

c) calculating a second ROS appearance rate $V_{2\,Ros}$ of ROS in said sample

$$V_{2\,Ros} = V2^+ * f_{QH_{2o}} * f_{cytC_{ox}} * \left(1 + \frac{[Q]}{K_M^{Q_o}}\right) * f_{O_2}$$

wherein

$$f_{QH_{2o}}([QH_2],[Q]) = \frac{\dfrac{[QH_2]}{K_M^{QH_{2o}}}}{1 + \dfrac{[QH_2]}{K_M^{QH_{2o}}} + \dfrac{[Q]}{K_M^{Q_o}}} \text{ in which } K_M^{QH_{2o}} = \frac{K_{M,0}^{QH_{2o}}}{1 + e^{\frac{f_{cytC_{ox}} - k_1}{k_2}}}$$

$$f_{cytC_{ox}}([cytC_{ox}],[cytC_{red}]) = \frac{\dfrac{[cytC_{ox}]}{K_M^{cytC_{ox}}}}{1 + \dfrac{[cytC_{ox}]}{K_M^{cytC_{ox}}} + \dfrac{[cytC_{red}]}{K_M^{cytC_{red}}}} \text{ and } f_{Q_i}([Q],[QH_2]) = \frac{\dfrac{[Q]}{K_M^{Q_i}}}{1 + \dfrac{[Q]}{K_M^{Q_i}} + \dfrac{[QH_2]}{K_M^{QH_{2i}}}},$$

and

$$f_{O_2}([O_2]) = \frac{\dfrac{[O_2]}{K'_{O_2}}}{1 + \alpha' f_{Q_i} + \beta f_{cytC_{ox}}}$$

wherein

$V2^+$ = 3909.06 mmol/min/mg ;
$K_1$ = 19.96, $K_2$= 341.15,
$K_{M,0}^{QH2o}$ = 157.61 μM, $K_M^{QH2i}$ = 478.63,
$K_M^{Qo}$= 50.49 μM, $K_M^{Qi}$ = 280.26 μM,
$K_M^{cytCox}$ = 10.98 μM, $K_M^{cytCred}$= 248.18 μM,
$K'_{O2}$ = 760.78,
α'= 38.66 and β = 212.03

d) obtaining the ROS appearance rate such that

$$VRos = V_{1\,Ros} + V_{2\,Ros,}$$

- quantifying the ROS appearance rate in the biological sample, after the administration of the compound to the patient, by carrying out the method as defined in anyone of claims 1 to 4, in order to obtain a final ROS rate,
- comparing the initial ROS rate and the final Ros rate in order to obtain a ratio R between the final ROS and the initial ROS rate,

- concluding that

    * if R is lower than 0.8, then the compound is able to reduce ROS amount in the sample,
    * otherwise the sample is not able to reduce ROS amount in the sample.

In one other aspect, the invention relates to a method for determining *in vitro* in a biological sample if a compound is a pro- or an anti-oxidant compound, the method comprising

- quantifying the Ros appearance rate in the biological sample by carrying out the method as defined in anyone of claims 1 to 4, in order to obtain a calculated rate,
- comparing the calculated rate with a reference Ros appearance rate, said reference Ros appearance rate being obtained from a reference sample, the reference sample being of the same nature as the sample, in order to obtain a ratio R between the Ros appearance rate and the reference Ros appearance rate, and
- concluding that

    * if R is lower than 0.85, then the compound is an anti-oxidant compound,
    * if R is higher than 1.15, then the compound is a pro-oxidant compound, and
    * if R is comprised from 0.85 to 1.15, then the compound has neither anti- nor pro-oxidant properties.

Advantageously the invention relates to the method defined above, the method comprising

- quantifying the Ros appearance rate in the biological sample by carrying out the method as defined in anyone of claims 1 to 4, in order to obtain a calculated rate,
- comparing the calculated rate with a reference Ros appearance rate, said reference Ros appearance rate being obtained from a reference sample, the reference sample being of the same nature as the sample, in order to obtain a ratio R between the Ros appearance rate and the reference Ros appearance rate, and
- concluding that

    * if R is lower than 0.8, then the compound is an anti-oxidant compound,
    * if R is higher than 1.2, then the compound is a pro-oxidant compound, and
    * if R is comprised from 0.8 to 1.2, then the compound has neither anti- nor pro-oxidant properties.

## Brief description of the drawings

The invention will be better understood in light of the following examples and the following figures:

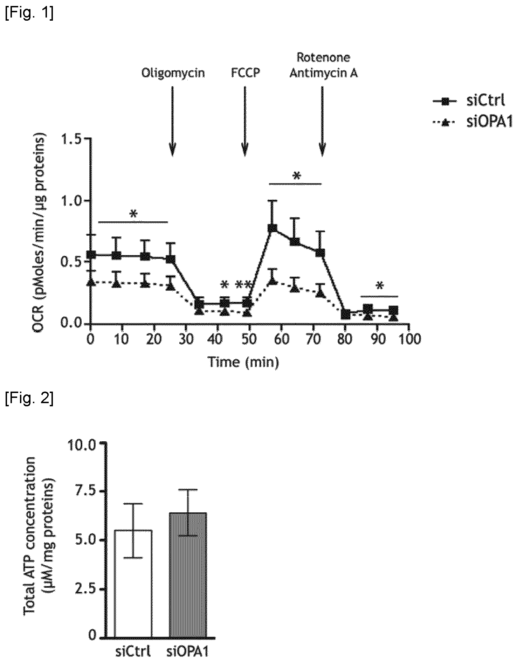

[Fig. 1] Oxygen consumption rates (OCR) were measured in siOPA1- and siCtrl-transfected HeLa cells. Spontaneous mitochondrial respiration was significantly lower in siOPA-transfected cells ($0.33 \pm 0.02$ pMoles/min/mg protein) than control cells ($0.53 \pm 0.04$ pMoles/min/mg protein). After oligomycin (1 $\mu$M) injection, cell respiration was also significantly lower in siOPA1-transfected cells ($0.11 \pm 0.01$ pMoles/min/mg protein) than control cells ($0.17 \pm 0.02$ pMoles/min/mg protein). After FCCP (1 $\mu$M) injection, maximal respiratory was significantly lower in siOPA-transfected cells ($0.30 \pm 0.04$ pMoles/min/mg protein) than control cells ($0.67 \pm 0.10$ pMoles/min/mg protein). Finally, rotenone (1 $\mu$M) plus antimycin A (1 $\mu$M) injections inhibited mitochondrial respiration. Results are expressed as the mean $\pm$ SEM (n = 3). P values were determined by Student's unpaired t-test p < 0.05* and p < 0.01**.
[Fig. 2] The ATP concentration was unchanged in siOPA1 Hela cells (grey bar) relative to siCtrl-treated (white bar) cells. Results are expressed as the mean $\pm$ SEM (n = 3). P values were determined by Student's-paired t-test.

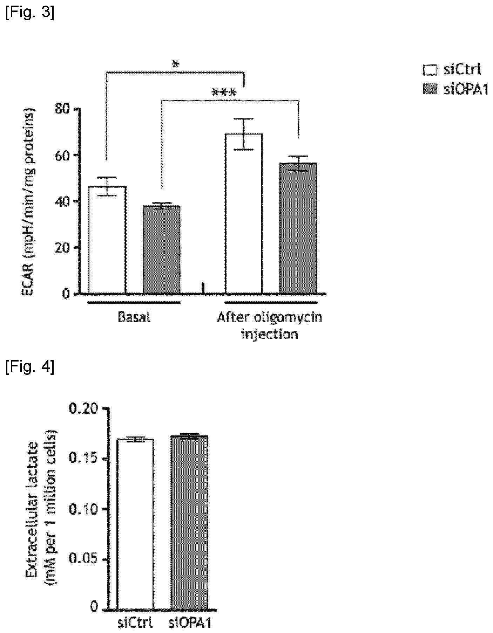

[Fig. 3] Extracellular acidification rates (ECAR) were unchanged in OPA1-treated cells (basal: $37.93 \pm 1.33$ pMoles/min/mg protein, oligomycin: $56.42 \pm 3.08$ pMoles/min/mg protein) relative to siCtrl-treated cells (basal: $46.40 \pm 3.93$ pMoles/min/mg protein, oligomycin: $69.14 \pm 6.72$ pMoles/min/mg protein) under basal conditions and after 1 $\mu$M oligomycin injection. As expected, ECAR increased in both siCtrl- and siOPA1-transfected cells after oligomycin injection. Results are expressed as the mean $\pm$ SEM (n = 3). P values were determined by Student's unpaired t-test p < 0.05* p < 0.001***
[Fig. 4] Extracellular lactate was measured by colorimetric analysis in HeLa cells with (grey bar) or without (white bar) siOPA1 (Mean $\pm$ SEM, n = 2). P values were determined by Student's-paired t-test.

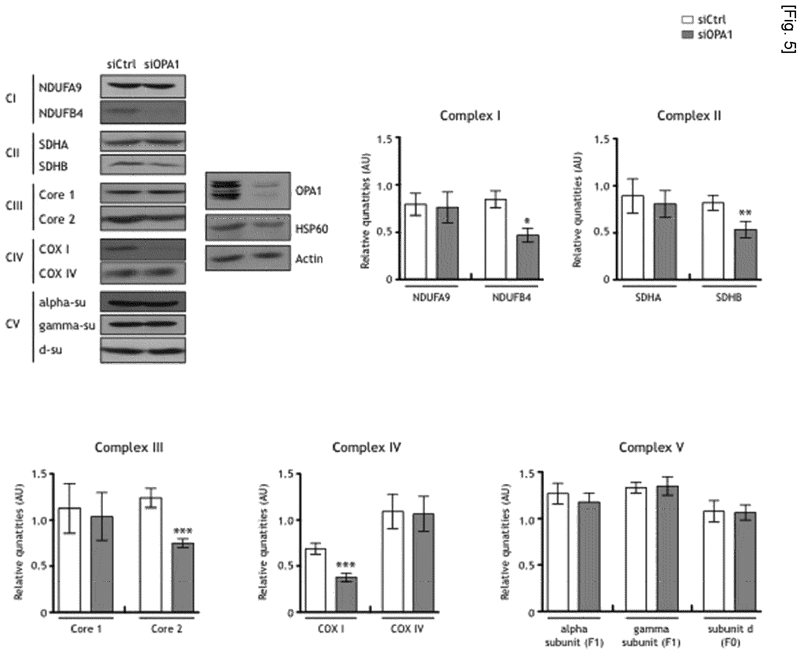

[Fig. 5] Representative immunoblots and histograms showing the effect of OPA1 downregulation in HeLa cells on the levels of two subunits of the MRC complexes (complex I-IV) and three subunits of ATP synthase (complex V) relative to actin. The quantity of NDUFB4 (n = 10) was significantly lower in siOPA1-transfected HeLa cells (0.47 ± 0.07) than siCtrl-transfected cells (0.85 ± 0.09). The quantity of SDHB (n =1 0) was significantly lower in siOPA1-treated cells (0.68 ± 0.16) than control cells (0.96 ± 0.16). The level of Core 2 (n = 10) was significantly lower in siOPA1-treated cells (0.75 ± 0.05) than control cells (1.238 ± 0.10). The level of COX I (n = 10) was lower in siOPA1-treated cells (0.37 ± 0.05) than control cells (0.69 ± 0.06). P values were determined by Student's-paired t-test (p < 0.05*, p < 0.01**, and p < 0.001***).

[Fig. 6] Activity of complex I (n = 3), II (n = 16), III (n = 3), and IV (n = 16) measured *in vitro* in both siOPA1- and siCtrl-treated HeLa cells. Succinate dehydrogenase (complex II) activity was lower in siOPA1-treated cells (6.438 ± 0.701) than control cells (8.688 ± 0.7229). Results are expressed as the mean ± SEM. P values were determined by Student's paired t-test (p < 0.05*, p < 0.01**, and p < 0.001***).

[Fig. 7] Total ROS measured by the $H_2$-DCFDA probe in siOPA1-transfected HeLa cells (72.63 ± 4.6) was lower than in siCtrl-treated cells (95.48 ± 9.1).

[Fig. 8] Aconitase activity was lower in siOPA1-treated cells (3.185 ± 1.41) than siCtrl-treated cells (4.811 ± 1.361).

[Fig. 9] Representative immunoblots and histograms showing that OPA1 downregulation in HeLa cells has no effect on the expression of aconitase relative to actin.

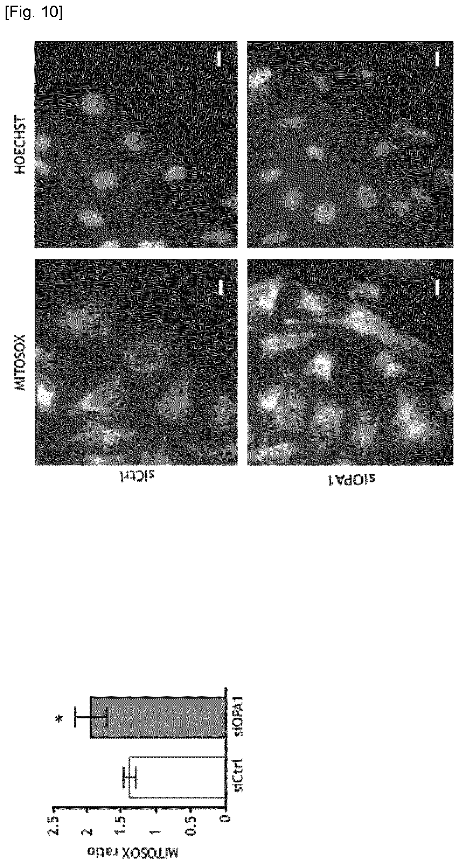

[Fig. 10] Mitochondrial superoxide production was measured using MitoSOX Red (mitochondria-targeted superoxide indication) with fluorescent microscopy (X40). MitoSOX measurement was performed after 72h siCtrl (white) or siOPA1 (grey) transfection. Representative histogram of quantitative fluorescence intensity was analysed by imaged software. The ROS level was higher in siOPA1-tranfected cells (1,9444 ± 0,2278) than control cells (1,386 ± 0,08754) Representative micrographs of mitochondrial MitoSOX immunocytofluorescence and DNA Hoechst staining in siCtrl- or siOPA1-treated HeLa cells 72h after transfection. Scale bar represents 10 μm. Results are expressed as the mean ± SEM with n = 8 (A), n = 6 (B), and n = 8 (C) and n=3 (D) with more than 200 cells. Statistical analysis was performed using a nonparametric test (Mann-Whitney) for A and D and Student's paired t-test for B (p < 0.05*).

[Fig. 11] Representative micrographs of NRF2 (green) immunocytofluorescence and DNA Hoechst staining (blue) or Merge (green+blue) in siOPA1- or siCtrl-treated HeLa cells 72 h after transfection.

[Fig. 12] Representative histogram showing the percentage of cells with NRF2 nuclear translocation 66, 67, 68, 69, 70, and 72 h after siOPA1 (grey) or siCtrl (white) transfection. Nuclear immunostaining of NRF2 was observed in 15.18 ± 1.86% of control HeLa cells and 51.68 ± 5.57% of siOPA1-treated cells 72 h after transfection. Results are expressed as the mean ± SEM with n =4 to 14 (400 cells per condition). P values were determined by a nonparametric test (Mann-Whitney test), p < 0.001***. Scale bar: 10 μm.

[Fig. 13] Representative immunoblots and relative quantities of SOD1, SOD2, Catalase, NQO1, GSTP1, FHC, and FLC protein in both siCtrl- and siOPA1-treated HeLa cells. The quantity of SOD1 was higher in siOPA1-treated cells (1.05 ± 0.03) than control cells (0.79 ± 0.05). The quantity of GSTP1 was higher in siOPA1-treated cells (1.07 ± 0.06) than control cells (0.82 ± 0.11).

[Fig. 14] Total SOD activity (SOD1 and SOD2) was higher in siOPA1-treated cells (1.01 ± 0.12) than control cells (0.73 ± 0.08).

[Fig. 15] Catalase activity was the same in siOPA1- and siCtrl-treated cells. Results are expressed as the mean ± SEM with n = 8 (A), n = 5 (B) n =7 (C) n=5 (D) and n=7. Statistical significance was determined by Student's-paired t-test, p<0.01**.

[Fig. 16] Simulations of Complex I activity with the mathematical model in a context of SiOPA1 or SiCtrl-treated cells.

[Fig. 17] Simulations of ROS production by complex I with the mathematical model in a context of SiOPA1 or SiCtrl-treated cells.

[Fig. 18] Representative immunoblots and histograms showing protein levels of OPA1 (inner membrane), citrate synthase (matrix), HSP60 (matrix), VDAC (outer membrane) and TOM20 (outer membrane) relative to actin, in OPA1 down-regulated cells and control cells. OPA1 protein level is drastically decreased in siOPA1-transfected HeLa cells (92%). In OPA1 downregulated HeLa cells, there are no differences of all proteins quantities compared to control cells.

[Fig. 19] Citrate synthase activity, a TCA cycle enzyme, is unchanged in both conditions. Results are expressed as mean +/- SEM with n=8 (A), n=16 (B). P values were determined by Student's-paired t-test p<0.001***.

[Fig. 20] The mitochondrial network in siOPA1-transfected HeLa cells is punctuated and filamentous in siCtr-transfected HeLa cells. Nuclei are stained with DAPI and the mitochondrial network is coloured with mitotracker. Scale bar represents 10 μm.

[Fig. 21] Ratio of $NAD^+$/NADH, $H^+$ and total intracellular levels of $NAD^+$ and NADH, $H^+$ were unchanged in HeLa cells transfected with siOPA1, when compared to siCtrl cells. Results are expressed as mean +/- SEM n=5. P values were determined by Student's-paired t-test.

[Fig. 22] Reduced (GSH) and oxidized (GSSG) glutathione levels are measured in HeLa cells transfected with siOPA1 (grey), compared to control cells (white).

[Fig. 23] Oxidized and reduced quinones are evaluated. Quinone redox state is the ratio of oxidized form of quinone on total forms. Results are expressed as mean +/- SEM n=4 (A), n=8 (B). P values were determined by Student's-paired t-test.

[Fig. 24] Representative micrographs of NRF2 (green) immunocytofluorescence and DNA Hoechst staining (blue) in siOPA1- or siCtrl-treated HeLa cells 72 h after transfection. (The scale bar represents 5 $\mu$m)

[Fig. 25] **Graphical Abstract.** In OPA1 depleted cells, some subunits of the first four complexes of the Mitochondrial Respiratory Chain are decreased without altered total ATP production. The cells fall in a pro-oxidative state as underlined by the decreased aconitase activity, the NRF2 transcription factor which is translocated into the nucleus and the GSTP1 and SOD1 protein quantities that are significantly increased and in turn decrease the intracellular ROS levels.

[Fig. 26] Complex I activity : simulation of Catalytic speed of $NAD^+$ in several configurations indicated on the graph with the different models from 1 to 5 with biological data from Heiske et al.(2014) (* represents biological data from Heiske et al.(2014) named literature ; lines with different colors are the results of simulation from model 1 to 5)

> **A - I :** For different concentrations of *NADH,* and with constant concentration of quinone Q (from 1.1 to 70 $\mu$M).
> **J - R :** For different concentrations of quinone Q and with constant concentration of *NADH* (from 1 to 47.5 $\mu$M)
> **S - U :** For different concentrations of quinol $QH_2$ or a ratio of $QH_2/Q_{tot}$ with constant concentration of NADH and quinone Q
> **V - X :** For different concentration of $NAD^+$ and with constant concentration of *NADH* and quinone Q.

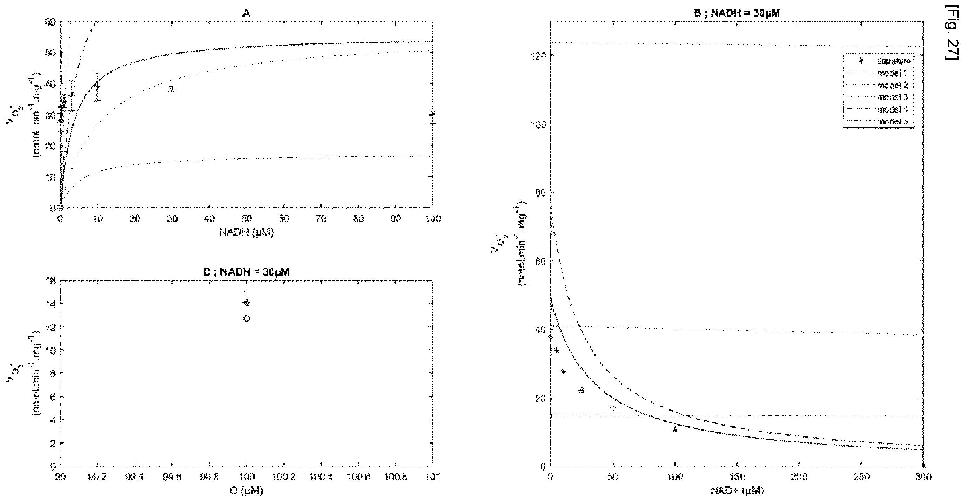

[Fig. 27] Complex I ROS production : Catalytic speed of $O_2^{\cdot-}$ in several configurations indicated on the graph with the different models from 1 to 5 with biological data from Kussmaul and Hirst (2006) and Grivennikova and Vinogradov(2006) (* represents biological data from Kussmaul and Hirst (2006) and Grivennikova and Vinogradov(2006) named literature ; solid line or with dashes are the results of simulation from model 1 to 5)

> **A :** For different concentrations of *NADH*
> **B :** For different concentrations of $NAD^+$
> **C :** For a concentration of NADH = 30 $\mu$M and Q = 100 $\mu$M

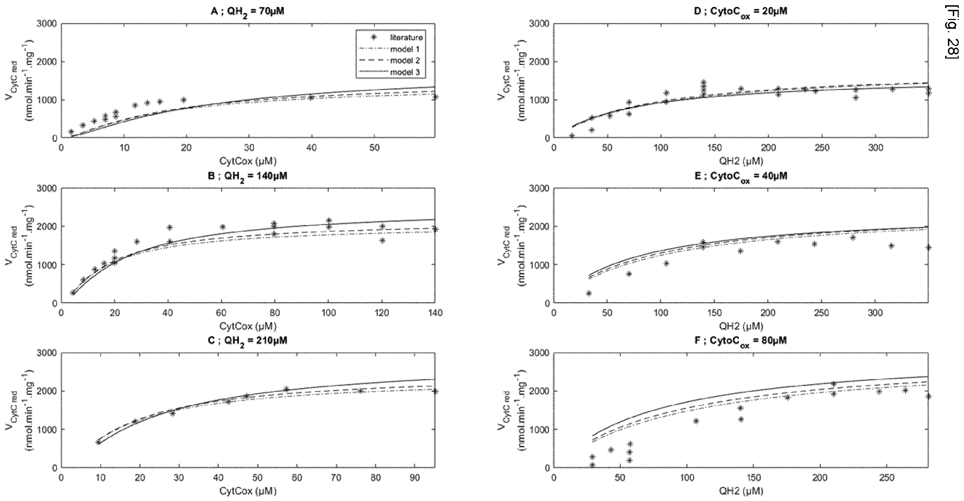

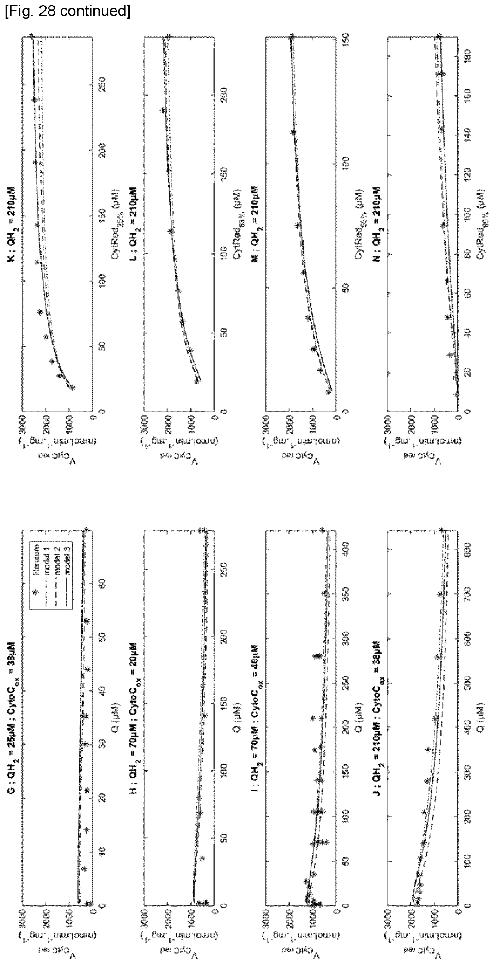

[Fig. 28] Complex III activity: simulation of Catalytic speed of $NAD^+$ in several configurations indicated on the graph with the different models from 1 to 3 with biological data from Heiske et al.(2017) (* represents biological data from Heiske et al.(2017) named literature ; solid line or with dashes are the results of simulation from model 1 to 3)

> **A - C :** For different concentrations of $CytoC_{ox}$ and with constant concentration of quinol $QH_2$ (from 70 to 210 $\mu$M).
> **D - F :** For different concentrations of quinole $QH_2$, and with constant concentration of $CytoC_{ox}$ (from 20 to 80 $\mu$M).
> **G - J :** For different concentrations of quinone Q, and with constant concentration of quinol $QH_2$ and of $CytoC_{ox}$ (from 20 to 80 pM)
> **K - N :** For different concentrations and percentage of $CytoC_{red}$ and with constant concentration of quinol $QH_2$ (210 $\mu$M).

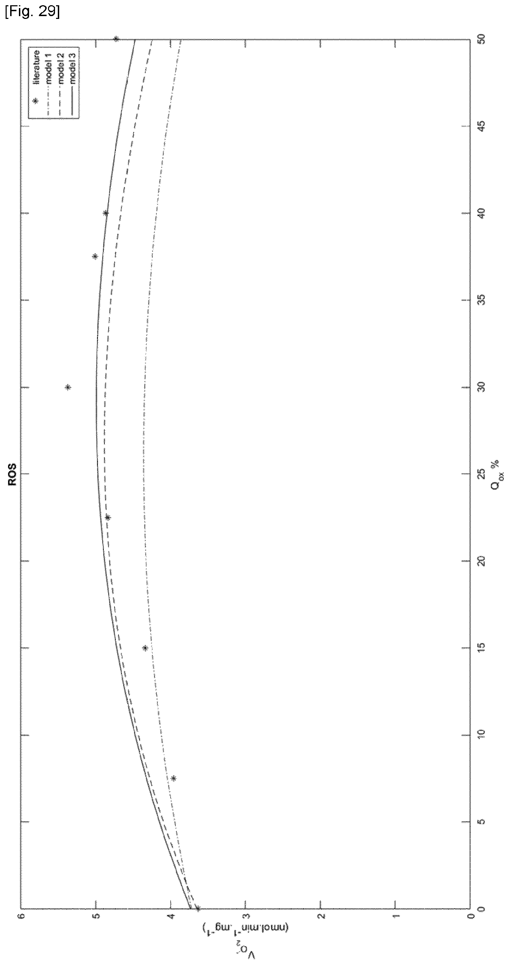

[Fig. 29] Complex I ROS production : Catalytic speed of $O_2^{\cdot-}$ for different percentages of quinone Q indicated on the graph with the different models from 1 to 3 with biological data from Dröse and Brandt (2008) (* represents biological data from Dröse and Brandt (2008) named literature ; solid line or with dashes are the results of simulation from model 1 to 3)

## Examples

### Example 1 -

Dominant optic atrophy (DOA) is characterized by moderate to severe loss of visual acuity with insidious onset in early childhood (Amati-Bonneau et al., 2009; Lenaers et al., 2012; Yu-Wai-Man et al., 2011). This disease primarily affects retinal ganglion cells (RGCs), of which the axons forming the optic nerve degenerate. The estimated prevalence is between 1:10,000 in Denmark and 1:50,000 worldwide. There is considerable inter- and intra-familial variability and penetrance may be as low as 40%. Different studies have shown certain OPA1 mutations to be associated with a severe multi-systemic disorder (DOA+ syndrome)(Amati-Bonneau et al., 2008; Cohn et al., 2007; Yu-Wai-Man et al., 2010; Zeviani, 2008). DOA+ patients present additional neurological complications, such as ataxia, sensorineural deafness, sensory-motor neuropathy, progressive external ophthalmoplegia, and parkinsonism, as well as myopathy. Altogether, these findings highlight the widespread deleterious consequences of OPA1 mutations, not only for RGCs but also for other cell populations (Barboni et al., 2013; Chao de la Barca et al., 2016; Mackey and Trounce, 2010; Spinazzi et al., 2008; Zeviani, 2008). There is currently no effective treatment for this complex disease.

Most DOA patients (-75%) harbour mutations in the OPA1 gene, which encodes a mitochondrial GTPase (Delettre et al., 2000) localized in the inter-membrane space (IMS) and anchored to the mitochondrial inner membrane (Griparic et al., 2004; Ishihara et al., 2006; Olichon et al., 2002; Satoh et al., 2003). Most OPA1 mutations result in premature termination, with the ensuing OPA1 haploinsufficiency as a major pathogenic mechanism (Amati-Bonneau et al., 2009). OPA1 protein has been shown to be involved in fusion of the mitochondrial inner membrane and the structure of the cristae in various cell lines. Fusion and fission of mitochondria control mitochondrial morphology and regulate the major mitochondrial functions (Bertholet et al., 2016). These processes also contribute to the quality control of the organelle. Through its role in organizing the structure of the cristae, OPA1 sequesters cytochrome c inside the cristae and thus exerts an anti-apoptotic function. Data on skin fibroblasts, muscle, and lymphoblasts from DOA patients globally show altered mitochondrial morphology and energetics, as well as increased sensitivity to apoptosis (Alavi et al., 2009; Chevrollier et al., 2008; Olichon et al., 2006; Spinazzi et al., 2008; Yu-Wai-Man et al., 2011; Zanna et al., 2008). However, there are numerous contradictory reports concerning the existence and nature of energetic defects in DOA patients and thus, the extent to which these processes contribute to the pathogenesis of DOA is still unknown. Furthermore, two invertebrate DOA models and, more recently, a mammalian DOA model have linked the critical generation of ROS to OPA1 dysfunction (Millet A. et al., 2017; Millet et al., 2016; Shahrestani et al., 2009; Tang et al., 2009).

The inventors previously developed a deterministic mathematical model able to predict The ROS production of Complex I of the MRC as well as The complex I catalytic activity . The method used is based on in vitro data referring to the activity (Heiske et al., 2014)(Heiske et al., 2014) and production of ROS (Grivennikova and Vinogradov, 2006; Kussmaul and Hirst, 2006) in several operating configurations of Complex I. These data are introduced in algorithms with normalization to be able to score the future sets of parameter. The molecular behavior is transcribed with the Michaelis and Menten equations of enzymatic kinetics of Complex I reactions. Several models are created with different levels of accuracy, thus increasing the number of parameters to be optimized. After having randomly generated a population of parameters, each solution is passed in a differential evolution algorithm in order to generate an optimized solution population. The models are connected through a cascade structure and are called successively from the simplest model to the most accurate. According to the algorithm, the selected set of parameters can simulate the activity of Complex I and the production of ROS at the same time with results qualitatively and quantitatively close to the input data.

The inventors thus addressed the question of the involvement of OPA1 in oxidative metabolism. As haploinsufficiency is primarily responsible for DOA and the effects of OPA1 inactivation are not restricted to RGCs, the inventors assessed the general impact of OPA1 inactivation on oxidative phosphorylation and the redox state by downregulating OPA1 in HeLa cells using an RNA interference strategy. The inventors found cellular respiration to be diminished when OPA1 levels were decreased. This was accompanied by an increase in mitochondrial ROS production, which was buffered by the activation of antioxidant defences, leading to a pro-oxidative state. The inventors' algorithm of Complex I is able to simulate its activity that fits with the inventors' in vitro data and refine hypothesis for the ROS production

2. Results

2.1. OPA1 downregulation affects cellular oxygen consumption and the quantity and activity of mitochondrial respiratory chain complexes

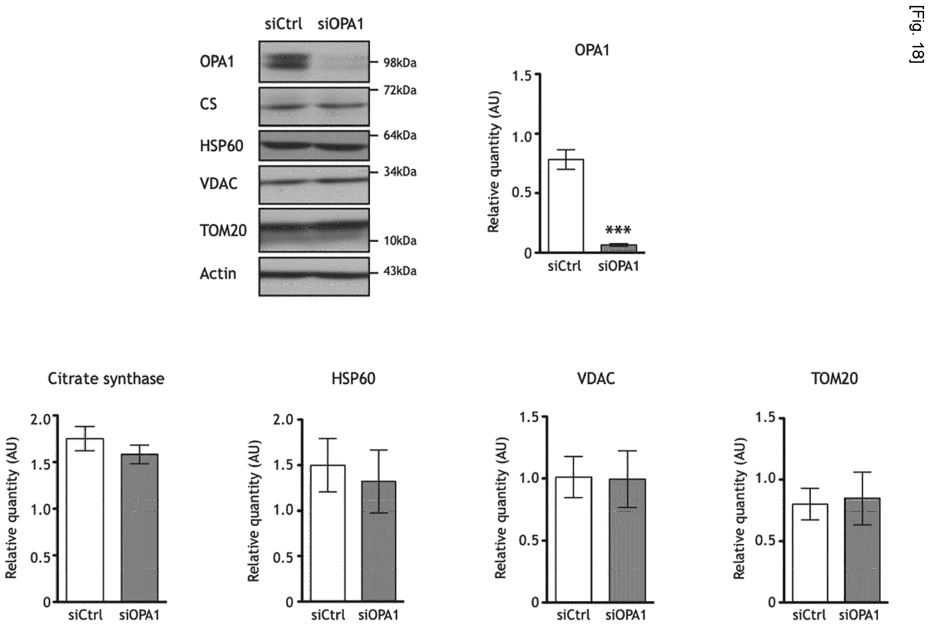

The inventors evaluated the effect of OPA1 downregulation on respiration and glycolysis in HeLa cells transfected with siRNA against OPA1 (siOPA1) using siRNA against Luciferase (siCtrl) as a control. Seventy-two hours after transfection, the quantity of OPA1 was 92% lower in siOPA1 treated cells than siCrtl cells but actin levels were not altered (**Figure 18**).

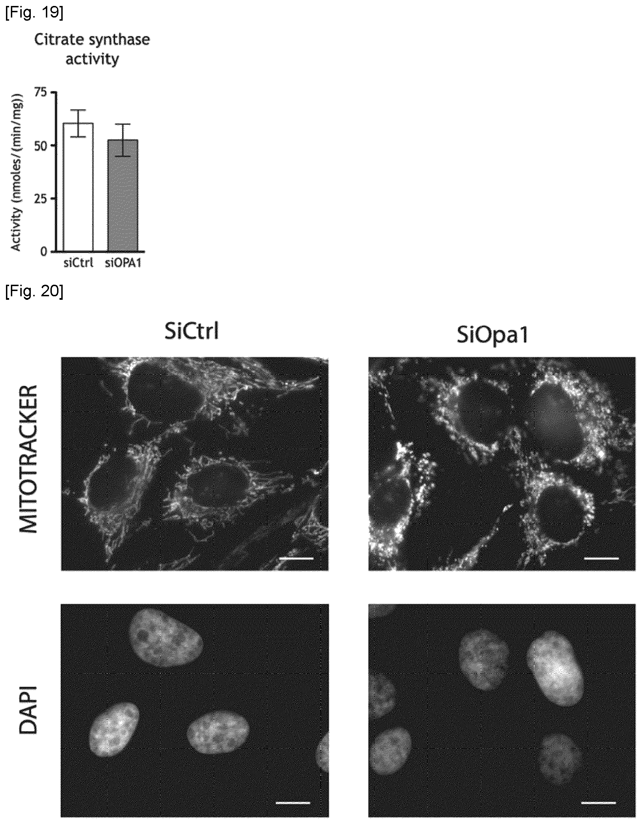

The inventors assessed oxygen consumption rates (OCR), a direct measure of oxidative phosphorylation activity, and extracellular acidification rates (ECAR), representative of glycolysis, since it accounts for approximately 80% of acidification (Wu et al., 2007, Xie et al., 2009), using the Seahorse XF24 flux analyser (Seahorse Bioscience Inc, North Billerica, MA, USA). Basal respiration was 38% lower in siOPA1-transfected HeLa cells than siCtrl-treated cells (**Figure 1**). Furthermore, ATP-linked respiration measured in the presence of oligomycin, which inhibits ATP synthase, was reduced by 40%. The maximal OCR measured in the presence of the protonophore FCCP, which uncouples oxidation and phosphorylation, was reduced by 58%. The maximal OCR in siOPA1-treated cells was not significantly different from the basal OCR, in contrast to siCtrl-transfected cells. Rotenone (complex I inhibitor) and antimycin (complex III inhibitor) considerably decreased the OCR in both siOPA1- and siCtrl-transfected Hela cells, showing that more than 95% of oxygen consumption was due to mitochondrial respiration (**Figure 1**). Of note, the decrease of mitochondrial respiration in siOPA1-treated cells did not correlate with a diminution in mitochondrial biomass. Indeed, citrate synthase activity and quantity and HSP60, VDAC, and TOM20 levels were unchanged (**Figures 18 and 19**). The inventors examined the mitochondrial network of SiCtrl- and SiOPA1-treated Hela cells; the SiOPA1 treated cells showed a fragmented mitochondrial network, in contrast to the filamentous mitochondrial network observed in SiCtrl-treated cells (**Figure 20**).

Although OPA1 downregulation induced a decrease in mitochondrial respiration, total intracellular ATP levels remained unchanged (**Figure 2**). Furthermore, the decrease in OPA1 levels did not induce a shift towards glycolysis in HeLa cells, as measured by ECAR (**Figure 3**) and extracellular lactate levels in the culture media (**Figure 4**). Similarly, oligomycin treatment increased acidification rates in both siCtrl- and siOPA1-treated HeLa cells (**Figure 3**).

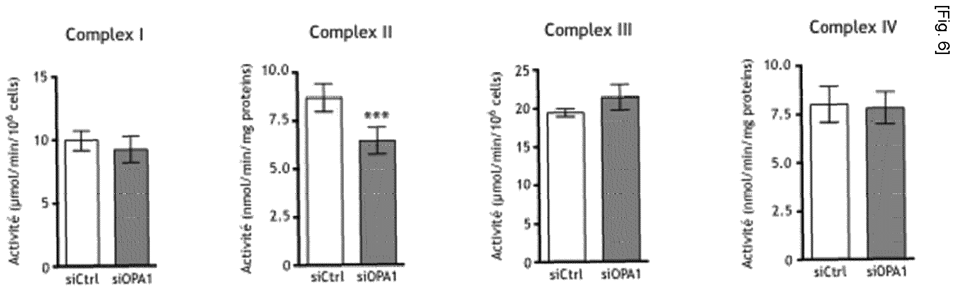

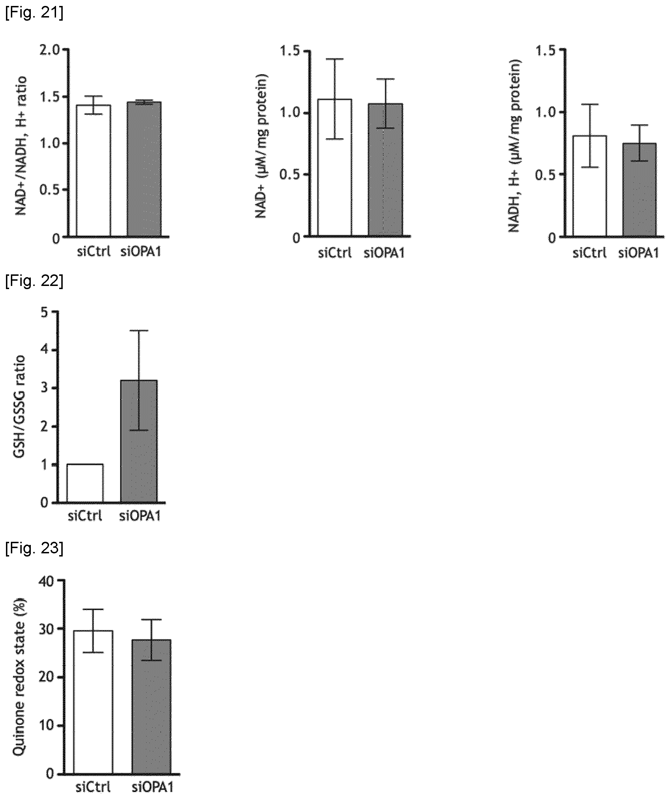

The effect of OPA1 downregulation on cellular respiration could be explained by a decrease in the level or activity of mitochondrial respiratory chain (MRC) complexes. The levels of the subunits NDUFB4 (Complex I), SDHB (Complex II), Core2 (Complex III), and COX I (Complex IV) were 44, 29, 39, and 46% lower, respectively, in siOPA1-treated HeLa cells than siCtrl treated cells (**Figure 5**). However, the levels of other subunits, such as NDUFA9 (Complex I), SDHA (Complex II), Core1 (Complex III), and COX IV (Complex IV), were unchanged (**Figure 5**). The levels of three subunits of ATP synthase (alpha and gamma for F1 complex and d for F0 complex) were unaffected by OPA1 silencing in HeLa cells (**Figure 5**). The inventors then assessed the in vitro activities of complexes I to IV by spectrophotometry. Although the activity of complexes I, III, and IV was not affected in OPA1-downregulated HeLa cells, complex II activity was reduced by approximately 25% (**Figure 6**). Succinate dehydrogenase is also a tricarboxylic acid (TCA) cycle enzyme. The inventors thus assessed the level of NADH, H+, the major TCA cycle product, in HeLa cells. The total intracellular level of NADH, H+/NAD+ was unchanged in siOPA1-treated HeLa cells (**Figure 21**). Accordingly, the levels of two other TCA cycle enzyme activities, fumarase and malate dehydrogenase, were also unchanged (data not shown).

2.2. OPA1 downregulation results in an imbalanced intracellular redox state

The loss of OPA1 results in impaired mitochondrial respiratory chain function, without disruption of the supply of NADH, H+. This can lead to an increase in electron leaks and thus increased reactive oxygen species production. OPA1 downregulation could thus lead to an imbalance of intracellular redox homeostasis in HeLa cells.

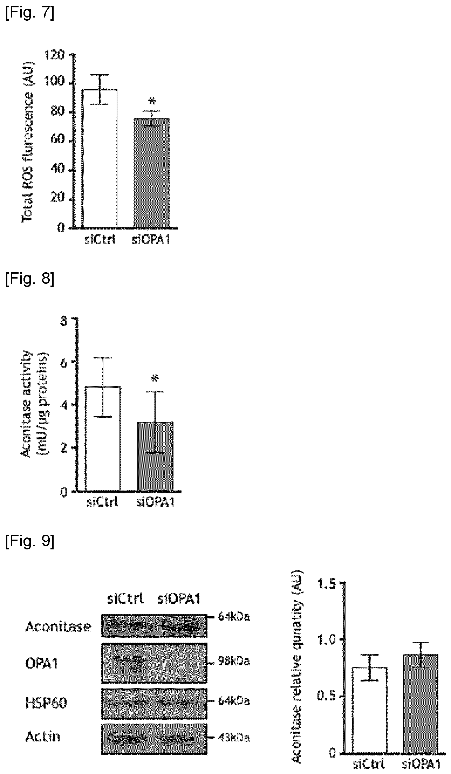

The inventors investigated this possibility by first measuring total ROS content using an H2-DCFDA probe. Surprisingly, ROS levels decreased by 23% in OPA1 downregulated HeLa cells 72 h after transfection (**Figure 7**). The inventors then investigated aconitase activity, which has been shown to be highly sensitive to oxidation due to a damaged FeS core. Inhibition of its activity is thus widely used as a signature of increased mitochondrial ROS production (Gardner et al., 1994; Kelly et al., 2010; Vincent et al., 2005). Aconitase activity was significantly lower (by 34%) in siOPA1-treated HeLa cells than control cells (**Figure 8**). This decrease could not be attributed to differences in protein quantity, which was unchanged (**Figure 9**).

2.3. OPA1 downregulation activates a major antioxidant pathway: the NRF2 transcription factor

Although the total intracellular level of ROS was reduced, the inhibition of aconitase activity provided evidence of an increase in intra-mitochondrial ROS production. This suggests that intracellular antioxidant responses were activated upon OPA1 downregulation. The inventors thus first analysed the levels of two redox state markers, glutathione and quinone. The ratio between reduced (GSH) and oxidized (GSSG) glutathione in siOPA1-treated HeLa cells increased by 78%, but this increase was not significant (**Figure 22**). The quinone redox state was not altered by the loss of OPA1 (**Figure 23**).

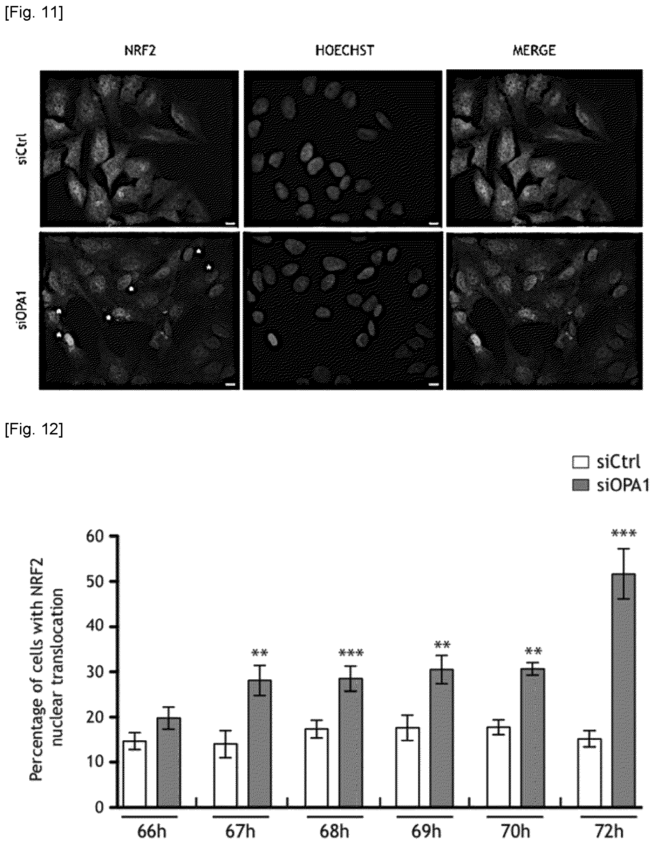

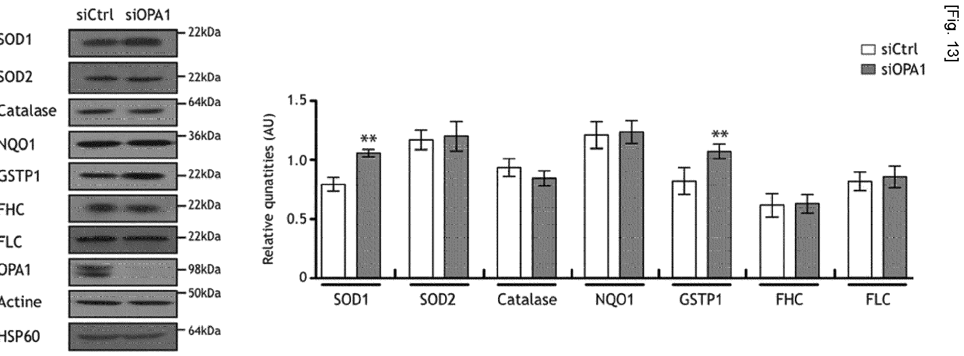

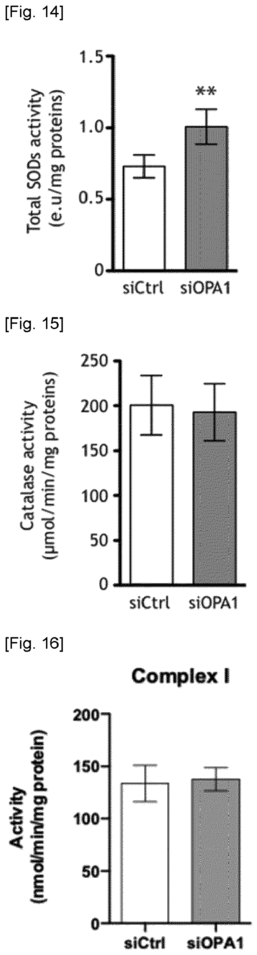

The inventors then investigated the nuclear factor (erythroid-derived 2)-like 2 (NRF2) pathway, which accounts for a large part of antioxidative responses. The inventors assessed the intracellular localisation of NRF2, which indicates its activation, by immunocytofluorescence in HeLa cells from 66 to 72 h post-transfection (**Figures 11 and 12**). The kinetics of NRF2 immunostaining showed significant NRF2 nuclear relocalisation starting 67 h after OPA1 downregulation (**Figure 12**). The inventors already knew that the level of OPA1 protein was reduced by 50% 48 h and 90% 72 h post-transfection (data not shown). The inventors thus hypothesized that NRF2 translocation would occur between 48 and 72 h post-transfection. Seventy-two hours post-transfection, 51% of siOPA1-treated HeLa cells showed NRF2 nuclear localisation, whereas only 15% of siCtrl-treated cells showed NRF2 in the nucleus **(Figures 11, 12 and 25)**. The inventors thus assessed the levels of several NRF2 target proteins, such as superoxide dismutases 1 and 2 (SOD1 and SOD2), catalase, NQO1, GSTP1, and ferritin heavy (FHC) and light (FLC) chains. The quantity of SOD1 protein was significantly higher (33%) in siOPA1-treated HeLa cells than control cells **(Figure 13)**. This increase correlated with a 37% increase in total SOD (SOD1 and SOD2) activity **(Figure 14)**. There was also an increase of 30% in the quantity of GSTP1 protein in siOPA1-treated HeLa cells **(Figure 13)**. Neither the quantity nor activity of catalase were altered upon downregulation of OPA1 **(Figures 13 and 15)**. Furthermore, there was no change in NQO1, FHC, or FLC protein levels in siOPA1-transfected HeLa cells. The data are summarized in Table 1.

[Table 1]

|  | OPA1 deficiency |
| --- | --- |
| H2DCFDA= total cellular ROS | Decrease |
| Aconitase activity | Decrease |
| Mitosox= mitochondrial ROS | Increase |
| Nuclear translocation of NRF2 | Increase |
| SOD1 | Increase |
| GSTP1 | Increase |
| Total SODs activity | Increase |

Table 1: Summary table of performed tests of oxidative metabolism for OPA1 deficiency in HeLa cells. Altogether, these results are showing that OPA1 down regulation increases mitochondrial ROS levels, activating the nuclear translocation of NRF2 which in turn upregulates some antioxidant defenses decreasing the total cellular ROS. This generates a pro-oxidative stress sensitizing cells to subsequent stress.

2.4. A deterministic mathematical model of complex I is able to predict the results obtained with OPA1 down regulation.

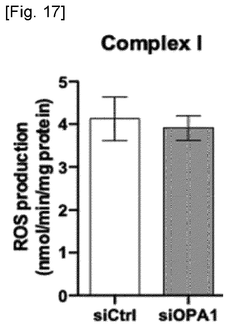

The data presented in **Figure 16** show that the inventors' algorithm fed with data from the inventors' analysis didn't find any difference in complex I activity while OPA1 is down-regulated or not as well as the inventors presented *in vitro* activities in **Figure 6.** the algorithm doesn't express the results in the same units as the *in vitro* results of **Figure 6** (micromole per min per milligram of proteins instead of micromole per min per million of cells). The inventors verified the stability of the protein contents in OPA1 down regulated and WT cells (SiLuc treated cells 99,23 +/- 22,56 n=6 and SiOPA1 treated cells 77,38 +/- 16,20 p value= 0,4516 with an unpaired T test) as they are strictly identical the inventors can compare the two types of results. Moreover, the inventors' algorithm presents no differences in ROS production by Complex I (**Figure 17**).

3. Discussion

Mitochondrial respiration was impaired upon OPA1 downregulation in HeLa cells, as previously shown in several cell types (Millet et al., 2016). This, however, did not trigger a shift towards glycolysis. Overall, the mitochondrial biomass did not change, as estimated both by measurement of the levels of several mitochondrial proteins and citrate synthase activity. However, the level of specific subunits of the first four complexes of the mitochondrial respiratory chain decreased. Therefore, the decrease in mitochondrial respiration measured in siOPA1-treated cells did not correlate with a decrease in the quantity of mitochondria but may be the consequence of impaired activity of the MRC complexes. Only the activity of complex II was decreased.

3.1. Cellular respiration is impaired in OPA1 downregulated cells

Oxygen consumption rates (OCR) were significantly decreased in OPA1 downregulated cells. This phenomenon, also analysed in vitro in OPA1 downregulated neurons, was not accompanied by a shift towards glycolysis (Millet et al., 2016). The decrease in OCR shown in siOPA1-treated cells could not be linked to a decrease in mitochondrial biomass, as the quantities of citrate synthase, HSP60, VDAC, and TOM 20 and the activity of citrate synthase remained unchanged in both siCtrl and siOPA1 treated cells. This phenomenon is similar to that observed in our previous study on OPA1 downregulated rat neurons in primary culture (Millet et al., 2016). In addition, the level of subunits of the first four complexes of the MRC were found to be similar in both OPA1 downregulated HeLa cells and rat neurons in vitro, with a marked decrease in the level of certain subunits. However, the quantity of complex V (ATP synthase) was unchanged upon OPA1 inactivation in both cellular models (Bertholet et al., 2013; Millet et al., 2016). Such a similar mitochondrial metabolism pattern in two very different cell types suggests that OPA1 downregulation triggers mitochondrial dysfunction possibly involved in both neuronal and extra-neuronal degeneration, as seen in DOA+ patients.

3.2. The redox state is imbalanced in OPA1-downregulated cells

A marked decrease in the quantity of MRC subunits and/or dysfunction of the activities of the complexes, while the level of NADH remains stable, results in increased mitochondrial ROS production. The fact that the NADH, H+, NAD+, and NAD+/NADH ratio were unchanged in the siCtrl and siOPA1 treated HeLa cells implies that the amounts of substrates provided to the MRC were also unchanged in both populations. As already demonstrated in siOPA1-treated rat neurons in primary culture, total intracellular ROS levels decreased. However, aconitase activity also decreased, suggesting an increase in mitochondrial ROS levels. Hence, the increase in intra-mitochondrial ROS production, which did not correlate with the total intracellular level of ROS, suggests the activation of antioxidant responses upon OPA1 downregulation. We verified this possibility by first analysing two redox state markers, glutathione and quinone. The ratio between reduced (GSH) and oxidized (GSSG) glutathione in siOPA1-treated HeLa cells increased by 121% relative to control cells, but was not statistically different (**Figure 22**), and by 78.1% in siOPA1-transfected neurons ($p < 0.05$), as previously described (Millet et al., 2016). The quinone redox state (oxidized quinones/(oxidized + reduced quinones)) was not altered by the loss of OPA1 (**Figure 23**). Altogether, these results suggest that OPA1 downregulation activates antioxidant responses by increasing the reduced form of glutathione, the major non-enzymatic antioxidant compound in the cells. Quinones appear not to be involved in mitochondrial ROS detoxification under our conditions. Once again, the similarities between the data obtained with neurons and HeLa cells is striking. These data suggest activation of the antioxidant signaling pathway to buffer the increase in mitochondrial ROS production.

3.3. Antioxidant defenses are activated in OPA1 downregulated cells.

We analysed the intra-nuclear level of NRF2, a major transcription factor involved in antioxidant defenses (Ma, 2013), in a time-course experiment to assess its activation upon OPA1 downregulation. There was a significant increase in the nuclear translocation of NRF2 in siOPA1-transfected cells from 67 to 72 h post-transfection. Of note, the level of OPA1 decreased by 50% and 90% 48 and 72 h post-siOPA1 transfection, respectively. This correlation between the decrease in OPA1 content and the nuclear translocation of NRF2 is particularly intriguing, as it underscores the link between OPA1 and the redox state.

We measured the levels of various NRF2 targets in the two conditions; the quantity of SOD 1 and GSTP1 increased upon OPA downregulation, as well as SOD activity. Similarly, both the level and activity of catalase, another NRF2 target, were elevated in siOPA1-treated neurons (Millet et al., 2016). The detoxification of the superoxide anion was clearly activated in both cellular types, underlying the importance of the phenomenon and the universal involvement of OPA1 in redox signaling.

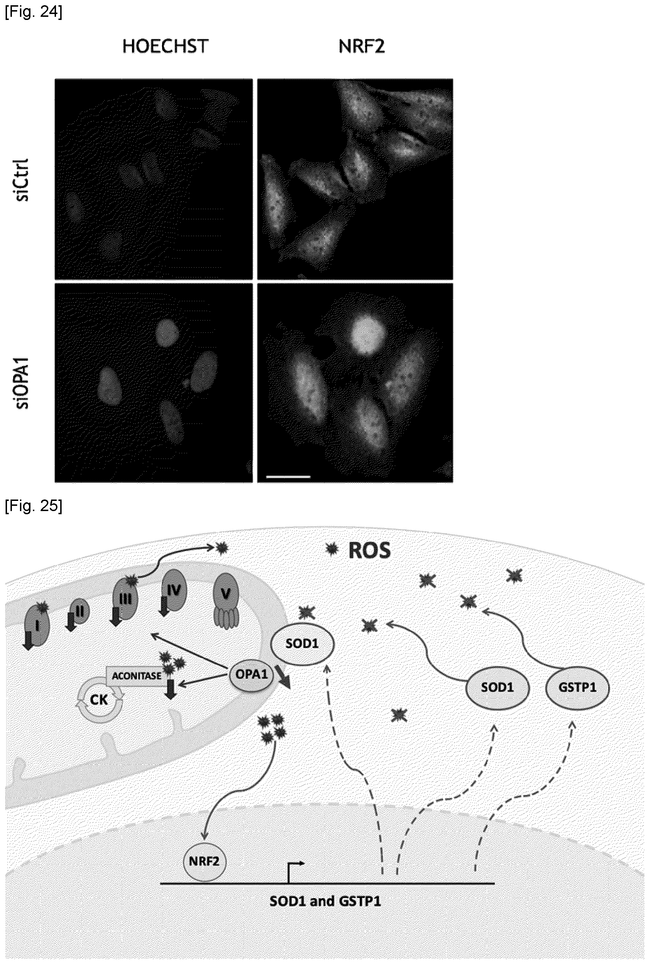

Our study is clearly in accordance with the literature linking OPA1 with the redox state in invertebrates (Kanazawa et al., 2008; Yarosh et al., 2008) and also confirms our results in neurons and transgenic mice cortices (Daloyau et al., 2018; Millet et al., 2016), with better identification of the actors. Moreover, this paper underlines the ubiquitous effect of OPA1 deficiency on oxidative metabolism in different cell types. A recent study analysing the expression of OPA1 during $H_2O_2$ treatment showed a large decrease in the long form of OPA1 (Garcia et al., 2018). The destabilization of the long form of OPA1 could, in turn, disorganize the inner membrane, emphasizing the effect of the reorganisation of the MRC and increasing ROS production. In other words, this phenomenon may represent a vicious circle (**Figure 25**).

3.4. Replicative cells as biological material to predict and treat OPA1 gene mutation-related disorders

The high convergence of the results based on two biological models of DOA that are so strikingly different paves the way towards novel ways of analysing, predicting, and possibly treating OPA1 dysfunctions. Moreover, the use of a mathematical model for the complex I activity and ROS production is interesting. In this work our algorithm simulates perfectly the complex I activity whether there is or not OPA1 expression. The activities are stable in both cases with in silico simulations (**Figure 16**) and in vivo analysis (**Figure 6**). In term of ROS production, the simulations suggest that Complex I wouldn't be the principal ROS producer. In this context, Complex III would be the most probable actor of the increase in mitochondrial ROS production. In silico simulations with our mathematical model of Complex I activity combined with ROS production will become a powerful tool to integrate different biological data and the predict the evolution of the described processes.

This is a real proof of concept for translational medicine in the area of neurodegenerative diseases. We plan to analyse the oxidative metabolism of DOA and DOA+ patients using fibroblasts from patient biopsies (Millet et al., 2016) and epithelial cells to predict the evolution of the disease, which is currently impossible to control. We will combine experimental and mathematical models to enhance the power of wet-lab investigations and more accurately predict the evolution of the disease by considering mitochondrial ROS production and the detoxifying pathways of ROS (Merabet et al., 2017; Millet A. et al., 2017).

Mitochondria have long been proposed to play a key role in aging (Green et al., 2011). As a consequence of their central role in ATP formation via the MRC, mitochondria are the major source of ROS and are thus highly involved in oxidative stress processes (Brookes et al., 2004). However, mitochondria are also targets of these molecules (Brookes et al., 2004). Under physiological conditions, approximately 1 to 3% of molecular oxygen is incompletely reduced during redox reactions in the MRC, leading to the production of ROS superoxide anion ($O_2^{\cdot -}$) by-products. In this scenario, complex interactions in antioxidant defence systems repress oxidative stress within mitochondria (Kienhofer et al., 2009). Cellular systems that protect against oxidants involve antioxidant defence enzymes (superoxide dismutase [SOD], glutathione peroxidase [GPx], and catalase) (Kienhofer et al., 2009), oxidant scavengers (vitamin E, vitamin C, carotenoids, uric acid, and polyphenols), and mechanisms that repair oxidant-induced damage to lipids, proteins, or DNA. Despite these protective mechanisms, the uncontrolled generation of ROS can overwhelm the capacity of antioxidant protection, causing mitochondrial dysfunction (for example Parkinson disease directly involves Complex I dysfunction). In vivo studies in transgenic mice have shown that the overexpression of catalase targeted to mitochondria reduces age-associated diseases and increases their lifespan (Lopez-Armada et al., 2013; Schriner et al., 2005). Based on these observations, our entire body of work clearly suggests that the downregulation of OPA1 in cellular and animal models induces an imbalance of the redox state, which could lead to premature cellular ageing.

## 4. Materials and Methods

### 4.1. Cell culture

HeLa cells from the American Type Culture Collection (Manassas, VA) were cultured in Dulbecco's Modified Eagle's Medium with 4.5 g/l glucose (DMEM, Invitrogen), supplemented with 10% FCS, penicillin (100 units/ml) and streptomycin (100 mg/ml), in an incubator at 37°C and 5% CO2. HeLa cells were electroporated using the Cell Line Kit R (Amaxa, Lonza) with 1.5 $\mu$g control siRNA (D-001210-02, Dharmacon Research) or human OPA1 siRNA (D-005273-03, target sequence **AAAGAAGGCUGUACCGUUA (SEQ ID NO: 1)**, Dharmacon Research) per $10^6$ cells.

### 4.2. Measurement of oxygen consumption rates, extracellular acidification rates and ATP levels

Oxygen consumption rates (OCR) and extracellular acidification rates (ECAR) were measured using the XF24 Extracellular Flux Analyser (Seahorse Bioscience, North Billerica, MA). HeLa cells (15.103) transfected with siCtrl or siOPA1 were plated on XF24 microplates three days before OCR measurements. Dual-analyte sensor cartridges were soaked in XF Calibrant Solution (Seahorse Biosciences) in 24-well cell-culture microplates overnight at 37°C to hydrate. Approximately one hour prior to experimentation, injection ports on the sensor cartridge were filled with oligomycin (1 $\mu$M), Carbonyl cyanide 4-(trifluoromethoxy) phenylhydrazone (FCCP) (1 $\mu$M), and rotenone (1 $\mu$M) plus antimycin A (1 $\mu$M). Plates were then loaded into the XF24 instrument for calibration. For measuring oxygen consumption, the DMEM growth media of HeLa cells was replaced by DMEM supplemented with NaCl (143 mM), Phenol Red (3 mg/ml), glucose (10 mM), glutamine (2 mM), and pyruvate (2 mM) at pH 7.4, and the plates maintained at 37°C 1 h prior to experimentation. Plates were then loaded into the Seahorse XF24 analyser following the manufacturer's instructions.

ATP measurements in HeLa cells were determined using the ATP Colorimetric/Fluorometric Assay kit (Abcam). Intracellular ATP levels were determined using the colorimetric assay, following the manufacturer's instructions, on 1.106 HeLa cells transfected with control siRNA or OPA1 siRNA. ATP content was measured in duplicate (570 nm) and calculated per microgram of protein.

### 4.3. *In vitro* activities of respiratory chain complexes

The activity of respiratory complexes II and IV and citrate synthase was measured as previously described (Agier et al., 2012). The activity of respiratory complexes I and III was measured as previously described (Spinazzi et al., 2011).

### 4.4. Extracellular lactate levels

Extracellular lactate levels were measured using a colorimetric assay (BioMérieux). Lactate was measured in media supernatants (1/10 dilution) at 505 nm 72 h after transfection of HeLa cells with siCtrl or siOPA1, following the manufacturer's instructions.

### 4.5. Immunoblot analysis

Transfected HeLa cells were lysed for 30 min in a RIPA buffer containing 50 mM Tris-HCL pH 7.5, 250 mM NaCl, 5 mM EDTA, 5 mM EGTA, 1 mM Dithiothreitol, 0.1% Triton X-100, 0.1% SDS, 1% Deoxycholate, 1% NP40, and protease inhibitors (« Complete » protease inhibitor mixture, Roche Applied Science). Cell lysates were then centrifuged at 14,000 g at 4°C for 10 min. The total protein concentration was determined in the supernatant using the Bradford Protein-assay (Bio-Rad).

Proteins (100-200 $\mu$g) were separated by SDS-PAGE (8-15%) and transferred onto nitrocellulose membranes (Whatman, Protran). Free binding sites were blocked with 5% nonfat dry milk in 1X Tris Buffered Saline pH 7.6 containing 0.2% Tween 20 (blocking buffer). The membranes were probed with various primary antibodies (anti-OPA1 (1/300, BD-Biosciences), anti-actin (1/25,000, Chemicon), anti-HSP60 (1/8,000, Sigma), anti-OXPHOS (1/200, Mitosciences), anti-NDUFB4 (1/500, Mitosciences), anti-NDUFA9 (1/100, Mitosciences), anti-SDHA (1/1,000, Abcam), anti-Core 1 (1/500, Invotrogen), anti-COXIV (1/250, Cell Signaling Technology), anti-ATP5C1 (1/500, Abgent), anti-ATP5H (1/5,000, Abcam), anti-aconitase (1/500, Abcam), anti-SOD1 and anti-SOD2 (1/2,000, Epitomics), anti-catalase (1/3,000, Abcam), anti-NQO1 (1/3,000, Abcam), anti-GSTP1 (1/8,000, Oxford Biochemical Research), anti-ferritin heavy chain (1/500, Abcam), and anti-ferritin light chain (1/4000, Abcam) and incubated overnight at 4°C in blocking buffer. After chemiluminescent detection of horseradish peroxidase-conjugated secondary antibody (1/50,000, Abcam), scanned photographic films were analysed using imaged software.

4.6. Immunocytochemistry or mitotracker staining

HeLa cells were fixed for 20 min with PBS containing 3.7% formaldehyde and permeabilized for 5 min in 1X PBS, 0.25 % TritonTM X-100 and incubated for 10 min at -20°C with methanol prior to nuclear NRF2 detection. Non-specific binding sites were blocked with 3% BSA in 1X PBS for 15 to 30 min at room temperature. Cells were immunostained with rabbit polyclonal anti-NRF2 antibody (1/50, Santa Cruz Biotechnology) for 1 h at 37°C. HeLa cells were then incubated with Alexa fluor 488-conjugated secondary antibodies (1/300, Molecular Probes), labelled with 0.25 $\mu$g/ml Hoechst in 1X PBS for 5 min, and mounted in Mowiol. Immunolabelling of HeLa cells 66, 67, 68, 69, 70, and 72 h after siCtrl or siOPA1 transfection was visualized under a fluorescence microscope (Nikon Eclipse 80i) and the images acquired using an NIS-Element (Nikon Digital Sight DUS2 camera). HeLa cells showing accumulation of NRF2 staining in the nucleus were counted by stack with Hoechst labelling of the nucleus using imaged software.

Mitochondrial network was stained with Mitotracker Red (Invitrogen molecular probes FM Invitrogen M22425) in culture medium during 15 min at 37°C and 5% CO2. Then cells were fixed for 20 min with PBS containing 3.7% formaldehyde and permeabilized for 5 min in 1X PBS, 0.25 % TritonTM X-100 and the mounted in DAPI-mounted medium.

4.7. Measurement of reactive oxygen species levels and aconitase activity

ROS levels in HeLa cells were measured using the fluorescent dye 2',7'-dichlorodihydrofluorescein diacetate (CM-H2-DCFDA, Molecular Probes) at 4 $\mu$M for 30 min at 37°C. Fluorescence intensities were measured at 493 nm in a WALLAC VICTOR 1480 Multilabel Counter.

Mitochondrial superoxide production was measured using MitoSOX Red (mitochondria-targeted superoxide indication) 5 $\mu$m upon the manufacturer's protocol with fluorescent microscopy (X40). MitoSOX measurement was performed after 72h siCtrl or siOPA1 transfection. Quantification of fluorescence intensity was analysed by imaged software.

Aconitase activity in HeLa cells was measured at 525 nm (UVIKON Spectrophotometer 922) using a protocol already described in (Colombani et al., 2009).

4.8. Enzymatic antioxidant activities

Superoxide dismutase (SOD) activities (Mn SOD and Cu/Zn SOD) were assayed by inhibition of pyrogallol auto-oxidation in HeLa cell extracts, as performed for aconitase activity. One enzymatic unit of SOD activity was defined as the amount of enzyme that inhibited pyrogallol auto-oxidation by 50% (Galinier et al., 2006). Catalase activity was determined by measuring the decomposition of H2O2 at 240 nm (Galinier et al., 2006).

4.9. Statistical analysis

Most of the experiments were statistically treated using Student's paired t-test because of the systematic comparison between siCtrl and siOPA1-treated HeLa cells. Oxygen consumption rates between siCtrl and siOPA1-treated HeLa cells were investigated using a nonparametric test (Mann-Whitney). P values: $p < 0.05^*$, $p < 0.01^{**}$, $p < 0.001^{***}$.

4.10. Immunoblot analysis

The membranes were probed with various primary antibody (anti-OPA1 (1/300, BD-Biosciences), anti-actin (1/25000, Chemicon), anti-HSP60 (1/8000, Sigma), anti-citrate synthase (1/3000, Abcam), anti-VDAC (1/1000, Abcam) and anti-TOM20 (1/3000, Abcam)) and incubated overnight at 4°C in blocking buffer. After chemiluminescent detection of horseradish peroxidase-conjugated secondary antibody (1/50000, Abcam), scanned photographic films were analysed using imaged software.

4.11. Citrate synthase activity

Citrate synthase activity was evaluated in HeLa cells transfected with siCtrl or siOPA1 lysates. Citrate synthase activity was determined by measurement of TNB produced by the reaction of CoA-SH and 5'5 dithiobis 2 nitrobenzoic acid (DTNB). 930 $\mu$l of reaction media (100 $\mu$M DTNB, 100 mM TrisHCL pH 8.0, 300 $\mu$M acetyl-CoA, 500 $\mu$M oxaloacetate, 0,1 % Triton X-100) and 40 $\mu$g of proteins were measured at 412 nm (37°C). Reactions start after adding 50 $\mu$l of 10 mM oxaloacetic acid diluted in 100 mM Tris HCl pH 8.1.

4.12. NAD$^+$ and NADH, H$^+$ levels

Intracellular NAD$^+$ and NADH levels in HeLa cells were measured with an NAD$^+$/NADH Assay kit (Abcam) according to the manufacturer's instructions. Briefly, $2.10^6$ HeLa cells were transfected with small interfering RNA control or against OPA1. Cells were washed with cold PBS and extracted with NADH/NAD extraction buffer by two freeze/thaw cycles (20 min on dry ice and then 10 min at room temperature). Total NAD (NADt) and NADH levels were detected in a 96-well plate, and color was developed and read at 450 nm. NAD+/NADH ratio was calculated as (NADt - NADH)/NADH.

4.13. Glutathione and quinone levels

HeLa cells were lysed with 200 $\mu$l of 5 % metaphosphoric acid and then centrifuged 1,500 g at 4°C during 10 min. Final supernatant was used for glutathione assay (reduced GSH and oxidized GSSG measurements) performed by reverse-phase high-perforance liquid chromatography (HPLC) as previously described (Galinier *et al.,* 2006). Quinone reduced and oxidized levels were performed as described (Galinier *et al.,* 2006).

**References**

Alavi, M.V., Fuhrmann, N., Nguyen, H.P., Yu-Wai-Man, P., Heiduschka, P., Chinnery, P.F., and Wissinger, B. (2009). Subtle neurological and metabolic abnormalities in an Opa1 mouse model of autosomal dominant optic atrophy. Experimental neurology 220, 404-409. 10.1016/j.expneurol.2009.09.026.

Amati-Bonneau, P., Milea, D., Bonneau, D., Chevrollier, A., Ferre, M., Guillet, V., Gueguen, N., Loiseau, D., de Crescenzo, M.A., Verny, C., et al. (2009). OPA1-associated disorders: phenotypes and pathophysiology. The international journal of biochemistry & cell biology 41, 1855-1865. 10.1016/j.biocel.2009.04.012.

Amati-Bonneau, P., Valentino, M.L., Reynier, P., Gallardo, M.E., Bornstein, B., Boissiere, A., Campos, Y., Rivera, H., de la Aleja, J.G., Carroccia, R., et al. (2008). OPA1 mutations induce mitochondrial DNA instability and optic atrophy 'plus' phenotypes. Brain : a journal of neurology 131, 338-351.

Barboni, P., Valentino, M.L., La Morgia, C., Carbonelli, M., Savini, G., De Negri, A., Simonelli, F., Sadun, F., Caporali, L., Maresca, A., et al. (2013). Idebenone treatment in patients with OPA1-mutant dominant optic atrophy. Brain : a journal of neurology 136, e231. 10.1093/brain/aws280.

Bertholet, A.M., Delerue, T., Millet, A.M., Moulis, M.F., David, C., Daloyau, M., Arnaune-Pelloquin, L., Davezac, N., Mils, V., Miquel, M.C., et al. (2016). Mitochondrial fusion/fission dynamics in neurodegeneration and neuronal plasticity. Neurobiology of disease 90, 3-19. 10.1016/j.nbd.2015.10.011.

Bertholet, A.M., Millet, A.M., Guillermin, O., Daloyau, M., Davezac, N., Miquel, M.C., and Belenguer, P. (2013). OPA1 loss of function affects in vitro neuronal maturation. Brain : a journal of neurology 136, 1518-1533. 10.1093/brain/awt060.

Brookes, P.S., Yoon, Y., Robotham, J.L., Anders, M.W., and Sheu, S.S. (2004). Calcium, ATP, and ROS: a mitochondrial love-hate triangle. American journal of physiology. Cell physiology 287, C817-833. 10.1152/ajpcell.00139.2004.

Chao de la Barca, J.M., Prunier-Mirebeau, D., Amati-Bonneau, P., Ferre, M., Sarzi, E., Bris, C., Leruez, S., Chevrollier, A., Desquiret-Dumas, V., Gueguen, N., et al. (2016). OPA1-related disorders: Diversity of clinical expression, modes of inheritance and pathophysiology. Neurobiology of disease 90, 20-26. 10.1016/j.nbd.2015.08.015.

Chevrollier, A., Guillet, V., Loiseau, D., Gueguen, N., de Crescenzo, M.A., Verny, C., Ferre, M., Dollfus, H.,

Odent, S., Milea, D., et al. (2008). Hereditary optic neuropathies share a common mitochondrial coupling defect. Annals of neurology 63, 794-798.

Cohn, A.C., Toomes, C., Potter, C., Towns, K.V., Hewitt, A.W., Inglehearn, C.F., Craig, J.E., and Mackey, D.A. (2007). Autosomal dominant optic atrophy: penetrance and expressivity in patients with OPA1 mutations. American journal of ophthalmology 143, 656-662.

Colombani, A.L., Carneiro, L., Benani, A., Galinier, A., Jaillard, T., Duparc, T., Offer, G., Lorsignol, A., Magnan, C., Casteilla, L., et al. (2009). Enhanced hypothalamic glucose sensing in obesity: alteration of redox signaling. Diabetes 55, 2189-2197. 10.2337/db09-0110.

Coustham, C., Merabet, N., Bordeneuve-guibe, J., and Davezac, N. (2021). Modeling of Complex I catalytic activity and ROS production: a multi-objective optimization for personalized medicine. GFB 21-24th september 2021.

Daloyau, M., Millet, A., Miquel, M.C., Mils, V., Wissinger, B., Belenguer, P., and Davezac, N. (2018). Brains from aged opa1+/-(B6;C3-Opa1 329-355del) Mouse Strain Are in a Pro-Oxidative State. ROS 6, 1-10.

Delettre, C., Lenaers, G., Griffoin, J.M., Gigarel, N., Lorenzo, C., Belenguer, P., Pelloquin, L., Grosgeorge, J., Turc-Carel, C., Perret, E., et al. (2000). Nuclear gene OPA1, encoding a mitochondrial dynamin-related protein, is mutated in dominant optic atrophy [In Process Citation]. Nat Genet 26, 207-210.

Galinier, A., Carriere, A., Fernandez, Y., Carpene, C., Andre, M., Caspar-Bauguil, S., Thouvenot, J.P., Periquet, B., Penicaud, L., and Casteilla, L. (2006). Adipose tissue proadipogenic redox changes in obesity. The Journal of biological chemistry 281, 12682-12687. 10.1074/jbc.M506949200.

Garcia, I., Innis-Whitehouse, W., Lopez, A., Keniry, M., and Gilkerson, R. (2018). Oxidative insults disrupt OPA1-mediated mitochondrial dynamics in cultured mammalian cells. Redox Rep 23, 160-167. 10.1080/13510002.2018.1492766.

Gardner, P.R., Nguyen, D.D., and White, C.W. (1994). Aconitase is a sensitive and critical target of oxygen poisoning in cultured mammalian cells and in rat lungs. Proceedings of the National Academy of Sciences of the United States of America 91, 12248-12252.

Green, D.R., Galluzzi, L., and Kroemer, G. (2011). Mitochondria and the autophagyinflammation-cell death axis in organismal aging. Science 333, 1109-1112. 10.1126/science. 1201940.

Griparic, L., van der Wel, N.N., Orozco, I.J., Peters, P.J., and van der Bliek, A.M. (2004). Loss of the intermembrane space protein Mgm1/OPA1 induces swelling and localized constrictions along the lengths of mitochondria. The Journal of biological chemistry 279, 18792-18798. 10.1074/jbc. M400920200.

Grivennikova, V.G., and Vinogradov, A.D. (2006). Generation of superoxide by the mitochondrial Complex I. Biochimica et biophysica acta 1757, 553-561. 10.1016/j.bbabio.2006.03.013.

Heiske, M., Nazaret, C., and Mazat, J.P. (2014). Modeling the respiratory chain complexes with biothermokinetic equations - the case of complex I. Biochimica et biophysica acta 1837, 1707-1716. 10.1016/j.bbabio.2014.07.013.

Ishihara, N., Fujita, Y., Oka, T., and Mihara, K. (2006). Regulation of mitochondrial morphology through proteolytic cleavage of OPA1. The EMBO journal 25, 2966-2977. 10.1038/sj.emboj.7601184.

Kanazawa, T., Zappaterra, M.D., Hasegawa, A., Wright, A.P., Newman-Smith, E.D., Buttle, K.F., McDonald, K., Mannella, C.A., and van der Bliek, A.M. (2008). The C. elegans Opa1 homologue EAT-3 is essential for resistance to free radicals. PLoS genetics 4, e1000022.

Kelly, M., Trudel, S., Brouillard, F., Bouillaud, F., Colas, J., Nguyen-Khoa, T., Ollero, M., Edelman, A., and Fritsch, J. (2010). Cystic fibrosis transmembrane regulator inhibitors CFTR(inh)-172 and GlyH-101 target mitochondrial functions, independently of chloride channel inhibition. The Journal of pharmacology and experimental therapeutics 333, 60-69. 10.1124/jpet. 109.162032.

Kienhofer, J., Haussler, D.J., Ruckelshausen, F., Muessig, E., Weber, K., Pimentel, D., Ullrich, V., Burkle, A., and Bachschmid, M.M. (2009). Association of mitochondrial antioxidant enzymes with mitochondrial DNA as integral nucleoid constituents. FASEB journal : official publication of the Federation of American Societies for Experimental Biology 23, 2034-2044. 10.1096/fj.08-113571.

Kussmaul, L., and Hirst, J. (2006). The mechanism of superoxide production by NADH:ubiquinone oxidoreductase (complex I) from bovine heart mitochondria. Proceedings of the National Academy of Sciences of the United States of America 103, 7607-7612. 10.1073/pnas.0510977103.

Lenaers, G., Hamel, C., Delettre, C., Amati-Bonneau, P., Procaccio, V., Bonneau, D., Reynier, P., and Milea, D. (2012). Dominant optic atrophy. Orphanet journal of rare diseases 7, 46. 10.1186/1750-1172-7-46.

Lopez-Armada, M.J., Riveiro-Naveira, R.R., Vaamonde-Garcia, C., and Valcarcel-Ares, M.N. (2013). Mitochondrial dysfunction and the inflammatory response. Mitochondrion 13, 106-118. 10.1016/j.mito.2013.01.003.

Ma, Q. (2013). Role of nrf2 in oxidative stress and toxicity. Annual review of pharmacology and toxicology 53, 401-426. 10.1146/annurev-pharmtox-011112-140320.

Mackey, D.A., and Trounce, !. (2010). Genetics: Optic nerve genetics--more than meets the eye. Nature reviews. Neurology 6, 357-358. 10.1038/nrneurol.2010.77.

Merabet, N., Bordeneuve-Guibe, J., and Davezac, N. (2017). Modelling the redox imbalance in Dominant Optic Atrophy: the case of respiratory Complex I. IFAC-PapersOnLine 50.

Millet A., M.N., Bertholet A., Daloyau M.,, Reynier P., G.A., Devin A., Wissinger B.,, and Bordeneuve-guibe J., B.P., Davezac N. (2017). Imbalance of the REDOX state in dominant Optic Atrophy: the way of mathematical modeling. Archives of the International Society of Antioxidants in Nutrition and Health 5, 21-24.

Millet, A.M., Bertholet, A.M., Daloyau, M., Reynier, P., Galinier, A., Devin, A., Wissinguer, B., Belenguer, P., and Davezac, N. (2016). Loss of functional OPA1 unbalances redox state: implications in dominant optic atrophy pathogenesis. Annals of clinical and translational neurology 3, 408-421. 10.1002/acn3.305.

Olichon, A., Emorine, L.J., Descoins, E., Pelloquin, L., Brichese, L., Gas, N., Guillou, E., Delettre, C., Valette, A., Hamel, C.P., et al. (2002). The human dynamin-related protein OPA1 is anchored to the mitochondrial inner membrane facing the inter-membrane space. FEBS letters 525, 171-176.

Olichon, A., Guillou, E., Delettre, C., Landes, T., Arnaune-Pelloquin, L., Emorine, L.J., Mils, V., Daloyau, M., Hamel, C., Amati-Bonneau, P., et al. (2006). Mitochondrial dynamics and disease, OPA1. Biochimica et biophysica acta 1763, 500-509.

Satoh, M., Hamamoto, T., Seo, N., Kagawa, Y., and Endo, H. (2003). Differential sublocalization of the dynamin-related protein OPA1 isoforms in mitochondria. Biochemical and biophysical research communications 300, 482-493.

Schriner, S.E., Linford, N.J., Martin, G.M., Treuting, P., Ogburn, C.E., Emond, M., Coskun, P.E., Ladiges, W., Wolf, N., Van Remmen, H., et al. (2005). Extension of murine life span by overexpression of catalase targeted to mitochondria. Science 308, 1909-1911. 10.1126/science.1106653.

Shahrestani, P., Leung, H.T., Le, P.K., Pak, W.L., Tse, S., Ocorr, K., and Huang, T. (2009). Heterozygous mutation of Drosophila Opa1 causes the development of multiple organ abnormalities in an age-dependent and organ-specific manner. PloS one 4, e6867. 10.1371/journal. pone.0006867.

Spinazzi, M., Cazzola, S., Bortolozzi, M., Baracca, A., Loro, E., Casarin, A., Solaini, G., Sgarbi, G., Casalena, G., Cenacchi, G., et al. (2008). A novel deletion in the GTPase domain of OPA1 causes defects in mitochondrial morphology and distribution, but not in function. Human molecular genetics 17, 3291-3302.

Tang, S., Le, P.K., Tse, S., Wallace, D.C., and Huang, T. (2009). Heterozygous mutation of Opa1 in Drosophila shortens lifespan mediated through increased reactive oxygen species production. PloS one 4, e4492.

Vincent, A.M., McLean, L.L., Backus, C., and Feldman, E.L. (2005). Short-term hyperglycemia produces oxidative damage and apoptosis in neurons. FASEB journal : official publication of the Federation of American Societies for Experimental Biology 19, 638-640. 10.1096/fj.04-2513fje.

Yarosh, W., Monserrate, J., Tong, J.J., Tse, S., Le, P.K., Nguyen, K., Brachmann, C.B., Wallace, D.C., and Huang, T. (2008). The molecular mechanisms of OPA1-mediated optic atrophy in Drosophila model and prospects for antioxidant treatment. PLoS genetics 4, e6.

Yu-Wai-Man, P., Griffiths, P.G., Gorman, G.S., Lourenco, C.M., Wright, A.F., Auer-Grumbach, M., Toscano, A., Musumeci, O., Valentino, M.L., Caporali, L., et al. (2010). Multisystem neurological disease is common in patients with OPA1 mutations. Brain : a journal of neurology 133, 771-786. 10.1093/brain/awq007.

Yu-Wai-Man, P., Trenell, M.I., Hollingsworth, K.G., Griffiths, P.G., and Chinnery, P.F. (2011). OPA1 mutations impair mitochondrial function in both pure and complicated dominant optic atrophy. Brain : a journal of neurology 134, e164. 10.1093/brain/awq288.

Zanna, C., Ghelli, A., Porcelli, A.M., Karbowski, M., Youle, R.J., Schimpf, S., Wissinger, B., Pinti, M., Cossarizza, A., Vidoni, S., et al. (2008). OPA1 mutations associated with dominant optic atrophy impair oxidative phosphorylation and mitochondrial fusion. Brain : a journal of neurology 131, 352-367.

Zeviani, M. (2008). OPA1 mutations and mitochondrial DNA damage: keeping the magic circle in shape. Brain : a journal of neurology 131, 314-317. 10.1093/brain/awm339.

## Example 2 - Multi objective optimization of mitochondrial process model : a novel tool for precision medicine

### 1. INTRODUCTION

In the context of precision medicine adapted to different pathologies (neurodegenerated diseases, metabolic diseases or inflammation processes) traditional biological approaches are still not sufficient. A system biology methodology associated to engineering techniques are necessary. This procedure is used to build a knowledge model of biological mechanisms. In a cellular system, mitochondria arc the main provider of energy: Adenosine Tri Phosphate (ATP) via the oxidative phosphorylation. The oxidative phosphorylation system consists of five enzyme complexes anchored in the inner membrane of the mitochondria. Four of these complexes (complexes I to IV) make up the respiratory chain. By moving protons (H+), complexes !, VI and IV establish an electrochemical gradient. This process is powered by the redox energy released during the successive electron transfers from the Nicotine of electrons from reduced Nicotinamide adenine dinucleotide (NADH) and succinate to oxygen. The fifth complex uses this electrochemical gradient created by complexes I, III and IV to catalyse the phosphorylation of adenosine diphosphate (ADP) to ATP (Mitchell, 1961; Mitchell, 1966).

During a normal function of the MRC, Reactive Oxygen Species (ROS) are basically produced essentially by the first and third complexes. ROS arc derivatives of dioxygen with sur numerar electrons. However, complex 1 and complex III dysfunctions, caused by different factors (genetic, environmental or pathologies) result in a dramatic increase of their production of ROS. This increase, if it is not balanced by antioxidant defences, induces lipids, proteins and DNA destruction 1. This phenomenon finally ends in cell death most of the time associated with inflammation processes. We previously developed a stochastic mechanical model of complex I able to simulate complex I activity and its ROS production (Merabet, 2017). In the present example, the inventors focused on a deterministic model of complex I and complex III.

## 2. BIOLOGICAL MECHANISM OF MITOCHONDRIAL RESPIRATORY CHAIN

Complex I is an electron entry point in the respiratory chain 2. It catalyzes the oxidation of *NADH to NAD+* and the reduction of ubiquinone (Q) into ubiquinol *(QH2}*. This reaction allows to move four protons from the matrix to the intermembrane space.

$$NADH + Q + 5H_x^+ \leftrightarrows NAD^+ + QH_2 + 4H_{is}^+ \quad (1)$$

"x" refers to the mitochondrial matrix and "is" refers to the intermembrane space.

NADH is oxidized by a non-covalently bound flavin mononucleotide (FMN). The hilly reduced flavin FMNH- and the semi-reduced flavin FMNH transfer these electrons one by one to a chain of eight ironsulfur (FrS) clusters constituting the redox centers of the Complex I. The last cluster of the chain is the electron donor to the Q-binding site. It is well accepted that biological electron transfer is a process of tunnelling (Moser et al., 1992).

Complex III oxidizes ubiquinol and reduces cytochrome c. In mammals, complex III is a dimer with each monomer consisting of 11 subunits (Iwata et al., 1998). Among these 11 subunits, three are conserved among species, from bacteria to mammals, and contain four groups involved in the redox function of complex TH: the bH and bL hemes of cytochrome b, cytochrome c1, and the [2Fe-2S] center of the Rieske protein (ISP). The mechanism of the reaction catalyzed by complex III is called cycle Q. It was initially proposed by Mitchell (1975) and subsequently modified (Hunte et al., 2003; Berry et al.,2000). This mechanism is based on the existence of two binding sites for the Q/QH2 redox couple called site Qo site and Qi site. The oxidation of ubiquinol at the Qo site induces the release of two protons and the transfer of two electrons to complex III. The first electron is transferred to the Fb-S group of the PSI before being transferred to cytochrome cl and filially reducing cytochrome c (high potential chain). The other electron enters the low potential chain with the reduction of heme bL and is then transferred to heme bH and filially to the ubiquinone of the Qi site, forming a stabilized semiquinone (Hunte et al., 2003; Berry et al., 2000) that can be detected and released from the positive side of the membrane and a second cytochrome c is reduced ma the high potential chain while a second electron is transferred to the Qi site *via* the low potential chain, reducing the semiquinone to ubiquinol. This second phase is accompanied by the use of two protons (Berry et al., 2000).

Reactive oxygen species (ROS) and more precisely superoxide anion the first reactive oxygen species is mainly produced in mitochondria by complexes I and III of the MRC. Complexes I and III of the respiratory chain are considered to be the main producers of superoxide anion and its derivatives within the mitochondria (Bleier and Drose, 2013; Wong et al., 2017; Pryde and Hirst, 2011; Murphy, 2009). Superoxide production by complex I has been shown using isolated complexes (Kussmaul and Hirst, 2006; Esterliâzy et al., 2008), submitochondrial particles (Vinogradov and (Grivennikova, 2005; Pryde and Hirst, 2011; Genova et al., 2001; Fato et al.,2009; Grivennikova and Vinogradov, 2013; Maranzana et al., 2013; Kim et al.,2018) and intact mitochondria from multiple sources. Two modes of superoxide production by complex I have been identified: a direct production mode in the presence of NADH and a reverse production mode in the presence of succinate.

The production of ROS in direct mode is maximal when the NADH/NAD+ ratio is high (strongly reduced NAD pool) and, in mitochondria and submitochondrial particles, when complex I is inhibited by a Q-site inhibitor (e.g. rotenone) (Kussmaul and Hirst, 2006; Lambert and Brand, 2004). The second mode of production was observed with coupled submitochondrial particles and intact mitochondria. This mode of production relies on the reverse transport of electrons in complex I: this reverse transport requires the presence of a highly reduced quinone pool and a sufficiently high electrochemical gradient. Measured production rates in reverse mode can reach three times those in direct mode, measured under optimal conditions of KOS production by the direct mode (fully reduced NAD pool and presence of Q-site presence of complex I Q-site inhibitors such as rotenone). The reverse mode of production of complex I has been known since the 1960s (Hinkle et al., 1967) but has long been considered as a phenomenon without physiological relevance. However, several studies suggest, that this process, which does not require inhibition or dysfunction of complex 1, may have physiological validity. Indeed, this process could play an important role in redox signalling mechanisms in basal location and in pathological oxidative mechanisms, especially in the case of pathological mechanisms (Chouchani et al.,2016; Pell et al.,2018; Robb et al,2018).

Complex III is capable of producing large amounts of superoxide when inhibited by antimycin A at the Qi site. This production takes place at the Qo site and the superoxide produced is released oil both sides of the inner membrane of the mitochondria. In the absence of antimycin A the production of $O_2{\cdot}^-$ by complex III is low to undetectable (T^irrens et al., 1985; Muller et al,2002; Muller et al.,2003; Drose and Brandt, 2008; Borek et al., 2008; Chen et al., 2003b; Ksenzenko et al., 1983). But, in the presence of a physiological membrane potential, purified yeast complex III recoustituted iii phospholipid vesicles, also produce non-negligible amounts of ROS (Rottenberg et al.,2009). The rate of produced ROS would increase exponentially as a function of membrane potential and could reach values similar to those obtained lining antimycin A. Moreover, the absence of production by complex 111 under basal conditions does not mean that this production could not increase under pathological conditions (Bleier and Drose, 2013). This oxygen electron donor would be a semiquinone produced at the Qo site (Drose and Brandt, 2008; Fisher et al., 2016).

## 3. MODELLING OF ENZYME KINETICS

### 3.1 Complex I

Complex I is considered as a system that can transfer electrons from a donor *(NADH)* to several recipients $(Q, O_2)$ depending on the circumstances : The inventors use the Michaelis and Menten equation to relate observations to formulas. At the 2 active sites, the substrates need to bind to complex I to allow electron transfer (donor or recipient). In addition, once the products are formed, they access the active site, blocking the following substrates. The products become competitive inhibitions for their own substrate. The functions of active sites take the following form

$$(x, y) \longmapsto \frac{x/k_{Mx}}{1+x/k_{Mx}+y/k_{My}}$$ . The inventors identify *x* and *y* which are equivalent to molecule concentrations and $k_{Mx}$ and $k_{My}$ which are the Michaelis constants and are to be determined by all the inventor's algorithms.

For $f_{NADH}$

$$f_{NADH}([NADH],[NAD^+]) = \frac{\frac{[NADH]}{K_M^{NADH}}}{1 + \frac{[NADH]}{K_M^{NADH}} + \frac{[NAD^+]}{K_M^{NAD^+}}} \quad (2)$$

And for $f_Q$

$$f_{NQ}([NQ],[QH_2]) = \frac{\frac{[Q]}{K_M^Q}}{1 + \frac{[Q]}{K_M^Q} + \frac{[QH_2]}{K_M^{QH_2}}} \quad (3)$$

There is also a phenomenon of inhibition by substrates. These ones could physically prevent the products from leaving the active site, slowing down the resulting activity. Complex I has the ability to accumulate a limited number of electrons within itself. Thus, the more electrons the complex stores, the more the bottleneck effect will be felt as the reaction rate of electron donation from *NADH* to the complex is reduced. Thus, if the capacity of the quinone Qto accept electrons is low, the complex empties slowly, the bottleneck is consequent and the *NADH* can donate its electrons less quickly, thus $K_M^{NADH}$ decreases.

The production of the superoxide anion $O_2^{\cdot-}$ is proportionally linked to the quantity of oxygen but is also inhibited by two phenomena:

- *NAD⁺* blocks access to oxygen when donating an electron to the complex, this effect is modelled by competitive inhibition;

- the complex functions properly when the availability of the quinone $Q$ is sufficiently high.

The inventors add the empirical parameter alpha to control this phenomenon.

As the two types of inhibition do not operate at the same sites, they are multiplied ui the following formula:

$$f_{O_2}([O_2]) = \frac{\frac{[O_2]}{K_{O_2}}}{\left(1+\frac{[NAD^+]}{K_i^{NAD^+}}\right)(1+\alpha f_Q)} \quad (4)$$

By using the mechanisms of Complex I in direct mode, the inventors can transcribe its activity for each electronic pathway. When all the substrates are present at their site, a *NADH* molecule will donate 2 electrons, both of them for quinone site or directly for the oxygen reduction.

$$V_{NAD^+,Q} = V^+ * f_{NADH} * \left(f_Q + f_{O_2}\right) \quad (5)$$

The inventors use the constant $V^+$ to model the Complex I capacity to accept the electron transfer. The Inventors can separate the different pathways to have the normal pathway with quinone reduction.

$$V_{NAD^+,Q} = V^+ * f_{NADH} * f_Q \quad (6)$$

And the ROS production

$$V_{NAD^+,O_2^-} = V^+ * f_{NADH} * f_{O_2} \quad (7)$$

A second operating mode can be added to the direct mode :the reverse mode. In reverse mode, quinol $QH_2$ becomes the electron donor and $NAD^+$ or $O_2$ the recipients. The $f_{NAD^+}$ and $f_{QH_2}$ functions are then used with a mirror construction of their direct mode equivalent. For the $O_2^{\cdot-}$ production, the inventors assume that the electron donor is flavin as before but the presence or absence of quinone inhibiting $O_2^{\cdot-}$ production in direct mode has no impact in reverse mode, so wc remove it from the equation 4.

Once all these equations have been established, we need to find the values that the constants $K_M^{NADH}$, $K_M^{NAD^+}$, $K_M^Q$, $K_M^{QH_2}$, $K_{O_2}$, $\alpha$, $K_i^{NAD^+}$, and $V^+$ should take. For this purpose, the concentrations *NADH, NAD^+,* QH2, Q and $O_2$ are considered to be known arid are taken from the literature.

the inventors use a total of 5 direct models of complex I. The first one (numbered 1) is the simplest of all and uses the presented equations before 2, 3, 4, 6 and 7. *The model N*2 uses a sigmoid function to represent one of the Michaelis constants.* Models 3 and 4 take into account the inhibition of $f_{O_2}^2$ by $f_Q$ and $NAD^+$ respectively. The 5th and last model implements the steric inhibition of NADH.

Each model improves the previous model, so model 5 takes into account, all the additions mentioned and is the most complete.

3.2 Complex III

Complex III is considered as a system that can transfer electrons from a donor *(QH_2)* to several recipients (Q, $Cyt_c$, $O_2$) depending on the circumstances:

- In the absence of quinone al the *Qi* site, cytochrome C will be the recipient;

- In the presence of antimycin cytochrome C and oxygen will receive the electrons.

The inventor's use the Michaelis and Menten equations to relate observations to formulas. At the 3 active sites, the substrates need to bind to complex III to allow electron transfer (donor or recipient), in addition, once the products are formed, they access the active site, blocking the following substrates. These products become competitive inhibitions for their own substrate. The functions of active sites take following form $(x, y) \longmapsto \frac{x/k_{Mx}}{1+x/k_{Mx}+y/k_{My}}$ :

For $f_{QH_{2o}}$,

$$f_{QH_{2o}}([QH_2], [Q]) = \frac{\frac{[QH_2]}{K_M^{QH_{2o}}}}{1 + \frac{[QH_2]}{K_M^{QH_{2o}}} + \frac{[Q]}{K_M^{Q_o}}} \quad (8)$$

for $f_{cytc_{ox}}$

$$f_{cytc_{ox}}([cytc_{ox}], [cytc_{red}]) = \frac{\frac{[cytc_{ox}]}{K_M^{cytc_{ox}}}}{1 + \frac{[cytc_{ox}]}{K_M^{cytc_{ox}}} + \frac{[cytc_{red}]}{K_M^{cytc_{red}}}} \quad (9)$$

And for $f_Q$

$$f_Q([Q]) = \frac{\frac{[Q]}{K_M^{Q_i}}}{1 + \frac{[Q]}{K_M^{Q_i}} + \frac{[QH_2]}{K_M^{QH_{2i}}}} \quad (10)$$

The effects of antimycin on complex III and the *QH2* oxidation rate allow the inventors to speculate that a dependence between the *QH2* oxidation rate and the ability to receive electrons from cytochrome C exists. If the capacity of cytochrome C to accept electrons is high then $QH_2$ can donate them faster, (n) therefore $K_M^{QH_2}$ increases; and vice versa. $K_M^{QH_2}$ can therefore be modeled as a function of $f_{Cytc_{ox}}$ as a sigmoid to enrich the model.

The electrons evolving in complex III will be accepted in priority by the 2 substrates Q and cytochrome C, so

$$f_{O_2}([O_2]) = \frac{\frac{[O_2]}{K_{O_2}}}{1 + \alpha f_{Q_i} + \beta f_{cytC_{ox}}} \quad (11)$$

The inventors can modify the functions of $f_{cytcox}$, $f_Q$ and $f_{O2}$ to include antimycin inhibition.

By using the mechanisms of complex III, the inventors can transcribe its activity in each configuration. When all the substrates are present at their site, a quinole molecule will donate 2 electrons, 1 for each site. Cytochrome C will be reduced and quinone will be partially reduced, giving:

$$V_{cytc_{ox},1} = V^+ * f_{QH_2} * f_{cytc_{ox}} * f_{Q_i}^{\frac{1}{2}} \quad (12)$$

The inventors use the constant $V^+$ to model the complex III capacity to accept the electron transfer. When there is no quinone, the 2 electrons in the quinole reduce 2 cytochromes. The presence of quinone is considered to be an inhibition here.

$$f_{cytc_{ox},2} = V^+ * f_{QH_{2o}} * \frac{f^2_{cytc_{ox}}}{1 + \alpha f_{Qi}} \quad (13)$$

In the presence of antimycin, the electrons of the quinole will partition between tlie cytochrome and the heme bL. A quinone molecule at the $Q_o$ site collects the electron from the heme bL by reverse transfer and gives it to oxygen.

$$V_{cytc_{ox},O_2} = V^+ * f_{QH_{2o}} * f_{cytc_{ox}} * \left(1 + \frac{Q}{K_M^{Q_o}}\right) * f_{O_2} \quad (14)$$

The QH2, Q, $cytc_{ox}$, $cytc_{red}$, $O_2$ and *antimycin* concentrations are input data from literature. The inventors have to find the value of the other constant $K_M^{QH_{2o}}$, $K_M^{Q_o}$, $K_M^{Qcytc_{ox}}$, $K_M^{Qcytc_{red}}$, $K_M^{Q_i}$, $K_M^{QH_{2i}}$, $K_{O_2}$, $\alpha$, $\beta$ and $V^+$ to use the complex III model.

The inventorss use a total of 3 models of complex III. The first one (numbered 1) is the simplest and uses the presented equations. *Model No 2 uses a sigmoid function to represent one of the Michaelis constants.* The 3rd and last model implements the inhibition of complex III by antimycin.

Each model improves the previous model, so model 3 takes into account all the additions mentioned and is the most complete.

4. MULTIOBJECTIVE OPTIMIZATION

*Optimization function* The inventors use experimental data from articles :

- 3 for the complex I, one for the activity (Heiske et al.(2014)) and another for te ROS production (Kussmaul and Hirst (2006) ; Grivennikova and Vinogradov(2006))
- 2 for the complex III, one for the activity (Heiske et. al., 2017) and another for the ROS production (Dröse et Brandt, 2008).

The inventors search to minimize the maximal difference between model evaluation and the experimental data: the optimization is ensured *via* the constant, values.

$$f_i = \max\left(\left|V_{exp,i} - V_{mod,i}\right|\right) \quad (15)$$

*fi* is the i-th objective function, $V_{exp,i}$ is a vector of experimental catalytic speed of the i-th objective and $V_{mod,i}$ is the vector of catalytic speed evaluated with the model. The magnitude order between the catalytic speed and the ROS production speed being too large, we use the (Grodzevich et Romanko, 2006) method.

$$z_i^N = 1 \le j \le dmax\left(f_i(x_j)\right) \quad (16)$$

$$0 \le \frac{f_i - z_i^U}{z_i^N - z_i^U} \le 1 \quad (17)$$

$$z_i^U = f_i(x_i) \quad \text{where} \quad z_i^U = argmin_x\{f_i(x): x \in A\}$$

In order to optimize the parameters, we establish objective functions from experimental data from the literature and from the modeling of these data by our model. Thus the inventors seek to reduce as much as possible the difference between the experimental data and the inventors' model. The optimization domain is bounded by 2 points:

- the Utopia point: this is the ideal objective vector which minimizes each objective function individually,
- the Nadir point: this is the vector referencing the worst optimization of each objective function with the solutions found for the Utopia point.

Normalization allows us to give a score between 0 and 1 to each objective function. A score close to 0 means being close to the Utopia point, and therefore a good multi-objective solution. Conversely, a score close to 1 will result in a solution to be ruled out.

*Dominance* These scores make it possible to sort the most interesting solutions according to their dominance. A solution a dominates a solution b if each of its scores less than or equal to the respective score of b AND if there is at least one score of a lower than respective b score.

$$S_i^{(a)} \leq S_i^{(b)}, and \; \exists i \mid S_i^{(a)} < S_i^{(b)} \quad i \in [1, 2, ..., k] \quad (18)$$

*k* is number of objective function of an optimization. The solutions are arranged by fronts: front 1 contains all the solutions which are not dominated, front 2 contains the solutions dominated only once, and so on until having all the fronts.

The crowding distance (Deb, 2002) allows solutions to be ranked within the same dominance front. For each objective, the solutions with the highest and lowest scores will have an infinite distance. The intermediate solutions will have a crowding distance calculated by the normalised absolute difference of the solutions of the objective functions adjacent to solution i (i+1 and i-1). In other words, for each objective function fj, with $j \in \{1, ..., k\}$, the solutions are sorted by increasing score and the intermediate solutions Xi, $i \in \{2, ..., NP-1\}$ are calculated via :

$$d_{i,j} = \frac{\left|f_j(X_{i+1}) - f_j(X_{i-1})\right|}{\left|f_j(X_{NP}) - f_j(X_1)\right|}$$

with the assumption $f_j(X_{NP}) \neq f_j(X_1)$.

A total crowding distance can be calculated by adding the distances of each objective for a given solution Xi. Thus, the set of solutions is prioritized first by dominance ranks and then within a rank, the solution with the largest clutter distance will be the best solution.

*Initial population* The initial candidate population is usually created randomly to cover a wider field of the study area. However, the experimental data and the objective functions allows up to know a priori the relevant intervals for the different parameters. Nevertheless, this method can make converge towards local minima and skew the continuation of procedures. Therefore, we use Afi's method which generates a population of size NP according to an uniform random distribution while being bounded by the user, a population of opposite candidates is built in parallel to enrich the total population. A candidate

X = $(x_1, x_2, ..., x_n)$ of dimension n is constructed for all $i \in [1, 2, ..., n]$ to have $x_i \in [l_i, u_i]$ with $x_i = l_i + (u_i - l_i) * U(0,1)$. The opposite candidate X' = $(x'_1, x'_2, ..., x'_n)$ is defined by $x'_i = l_i + u_i - x_i \sim$ All these candidates make up a population of 2NP candidates. We use the dominance sorting method explained above for a population of the NP best candidates.

*Evolutionary algorithm* Once the population is formed, each $X_{p,G}$ candidate (p-th candidate of the population generation G) passes through a differential evolution algorithm having 3 operators: mutation, crossing and selection.

*Mutation:* this operator creates a new candidate from 3 candidates from the current population. For a candidate $X_{p,G}$ 3 random candidates are drawn $\{X_{p_1,G}, X_{p_2G}, X_{p_3,G}\}$ with $p, p_1, p_2, p_3 \in \{1,2, ..., NP\}$ and $p \neq p_1 \neq p_2 \neq p_3$. The mutated candidate $M_{p,G}$ is defined by:

$$M_{p,G} = X_{p_1,G} + F * (X_{p_2,G} - X_{p_3,G}) (19)$$

F is a control parameter between 0 and 1.

Crossing : the mutated candidate $M_{p,G} = (m_{p,G,1}, ..., m_{p,G,n})$ is crossed with the initial candidates $X_{p,G} = (x_{p,G,1}, ..., x_{p,G,n})$ create a temporary candidate $C_{p,G} = (c_{p,G,1}, ..., c_{p,G,n})$

$$C_{p,G,i} = \begin{cases} m_{p,G,i} & rand_r \leq C_r \ Vr \leq rr \ \ if \\ x_{p,G,i} & else \end{cases} \quad (20)$$

with $p \in \{1,2,..., NP\}$, $i \in \{l,2,...,n\}$, ra random value between 0 and 1, and rr a random value generated only one time for each p, and $C_r \in [0; 1]$ the crossing rate.

Selection : Usually, the selection is between the initial candidate and the crossed candidate, the best one is selected to incorporate the initial population of the next generation, the other candidate is simply discarded. Here, the unselected candidate is placed in a pool, once the whole population has been run through the evolutionary algorithm, this pool is challenged with the previously selected candidates in order to keep only the best NPs for the next generation's population.

It is possible to stop the evolutionary algorithm prematurely once a certain population convergence has been reached. This convergence is measured with the stability of the density of the non-dominated solutions. In other words, the stopping criterion is based on the evolution of the crowding of solutions: convergence is observed when the crowding distance no longer evolves significantly over several generations, so the search for solutions is stopped. We note $d\ell$ the maximum clutter distance at generation $\ell$. We can measure the stability of the clutter over L generations by calculating the difference :

$$\delta_L = \max_{1 \leq \ell \leq L} d_\ell - \min_{1 \leq \ell \leq L} d_\ell$$

with the condition on the stopping criterion:

$$\delta_L < \delta_{lim}$$

At this stage, a difficulty may arise since $d\ell$ may undergo oscillations even after convergence. In this case, with a too large $\Delta$lim criterion, the oscillations will not be taken into account and the search for a solution will stop too soon. On the contrary, if $\Delta$lim is too small, the stop will be too fast. In this respect, (ROUDENKO, 2004) uses the standard deviation of the dl instead of the stability of the clutter:

$$\sigma_L = \sqrt{\frac{1}{L}\sum_{\ell=1}^{L}(d_\ell - \overline{d_L})^2} < \delta_{lim}$$

wherein $\overline{d_L}$ is the mean of $d_\ell$ during L generations.

5. RESULTS AND DISCUSSION

The inventors have constructed a model of the catalytic activity of complex I and its production of superoxide anion in direct and reverse mode. To build this model we modified classical enzymatic kinetic equations to fit the specificities of complex I. The inventors optimized the parameters of the direct model with a multi-objective algorithm.

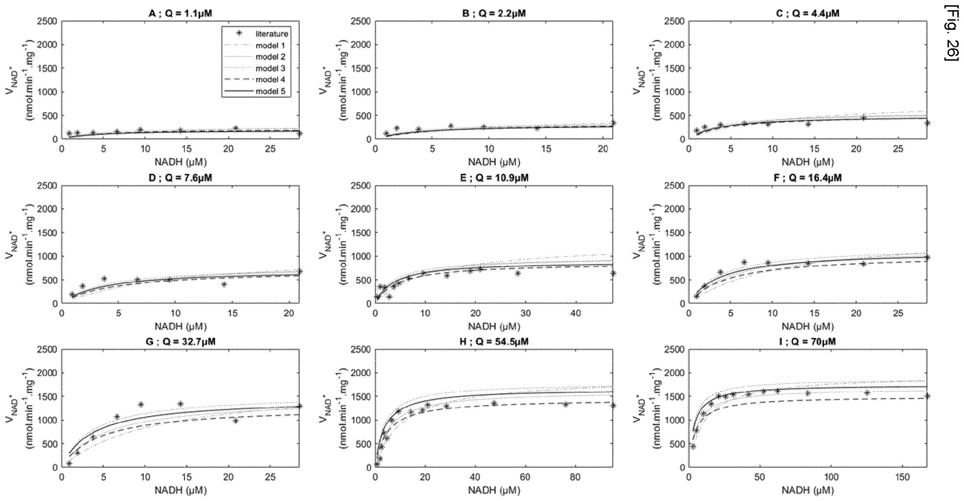

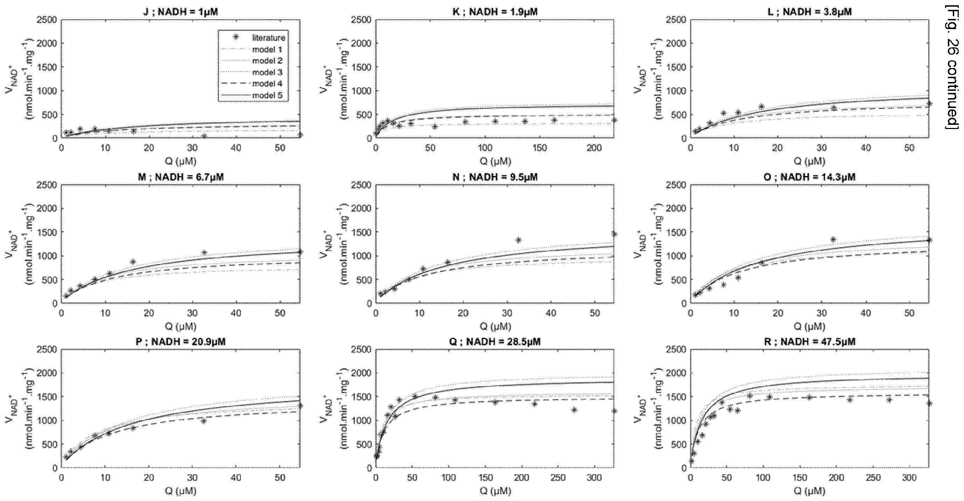

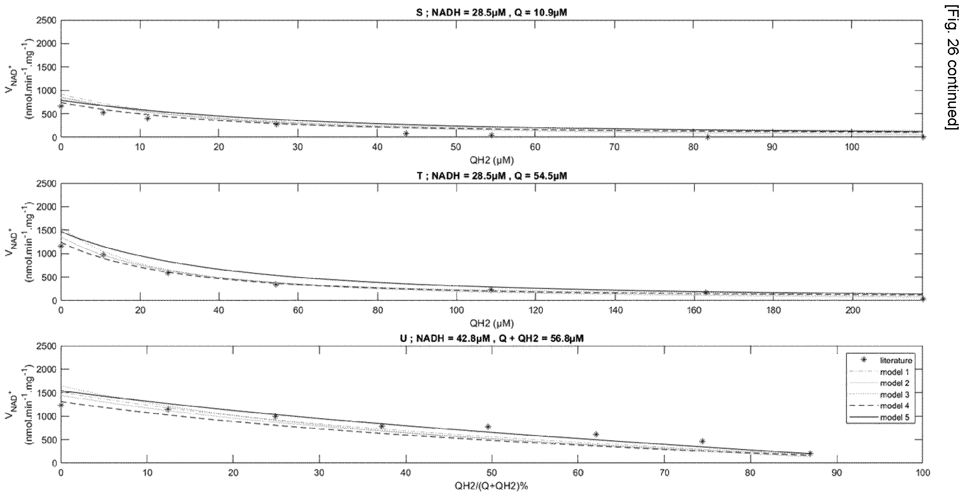

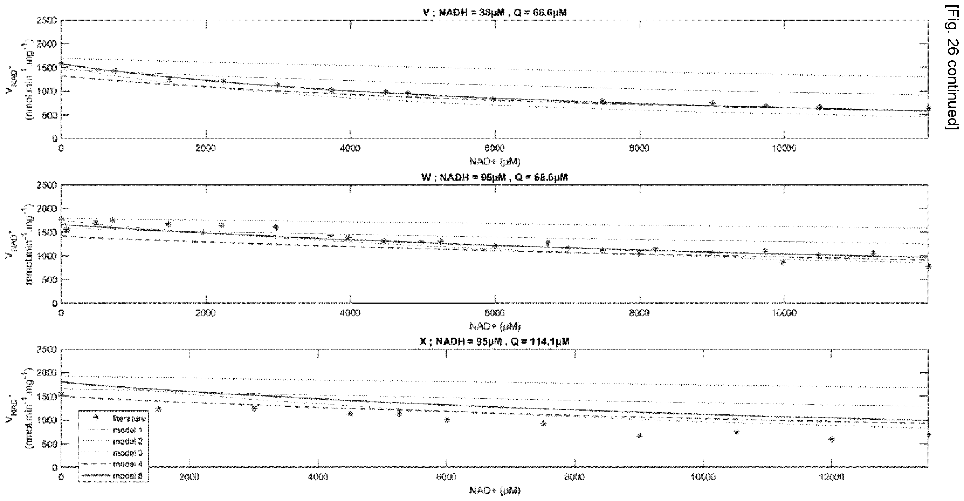

The model of complex I is able to simulate the catalytic activity - **Figure 26** - and production of ROS - **Figure 27** - in direct mode for different configurations and concentration of substrats and products.

For different concentrations of *NADH,* and with constant concentration of quinone $Q$ (from 1.1 to 70 $\mu$M) our model No 5 is the most accurate comparing to the experimental data. The result from model 1 to 5 is shown **Figure 26 A - I.**

For different concentrations of quinone Q and with constant concentration of *NADH* (from 1 to 47.5 $\mu$M) our model No 5 is the most of the time better comparing to the experimental data. The result from model 1 to 5 is shown **Figure 26 J - R.**

For different concentrations of quinol $QH_2$ or a ratio of $QH_2/Q_{tot}$ with constant concentration of *NADH* and quinone Q inventors' model No 5 is the most accurate comparing to the experimental data. The result from model 1 to 5 is shown **Figure 26 S** - **U.**

Finally, for different concentrations of $NAD^+$ and with constant concentration of $NADH$ and quinone Q inventors' model No 5 is the *most* accurate comparing to the experimental data. The result from model 1 to 5 is shown **Figure 26 V** - **X.**

For different concentrations of $NAD^+$ the model No 5 is the best for low concentrations of $NADH$ but it is divergent, for high concentration of $NADH.$ The result from model 1 to 5 is shown **Figure 27 A.**

For different concentrations of $NAD^+$ the model No 5 is the best. The result from model 1 to 5 to shown **Figure 27 B.**

For a concentration of NADH = $30 \mu$M and Q = 100 $\mu$M, all models are acceptable The result from model 1 to 5 to shown **Figure 27 C.**

The inventors can observe a real improvement of the simulations through the iterative process from model 1 to 5.

For different concentrations of $CytoC_{ox}$ and with constant concentration of quinol $QH_2$ (from 70 to 210 $\mu$M) inventors' model No 3 is the most accurate comparing to the experimental data. The result from model 1 to 3 is shown **Figure 28 A** - **C.**

For different concentrations of quinole $QH_2,$ and with constant concentration of $CytoC_{ox}$ (from 20 to 80 $\mu$M) our model No 3 is the most accurate comparing to the experimental data. The result, from model 1 to 3 is shown **Figure 28 D** - **F.**

For different concentrations of quinone Q, and with constant concentration of quinol $QH_2$ and of $CytoC_{ox}$ (from 20 to 80 pM) our model No 3 is the most accurate comparing to the experimental data. The result from model 1 to 3 is shown **Figure 28 G** - **J.**

For different concentrations and percentage of $CytoC_{red}$ and with constant concentration of quinol $QH_2$ (210 $\mu$M) our model No 3 is the most accurate comparing to the experimental data. The result from model 1 to 3 is shown **Figure 28 K** - **N.**

For different percentages of quinone Q the model No 3 is the best. The result from model 1 to 3 is shown **Figure 29.**

We applied the modelling method used for complex I to complex III. We created a model capable of simulating the production of ROS and the catalytic activity of complex III. We also performed a sensitivity analysis in the same way as for complex I.

Gauthier et al (Gauthier et al., 2013a) constructed a mathematical model of the production of ROS by complexes I and III in order to study the production of ROS in cardiac myocytes under different metabolic conditions. However, the model focuses only on the production of ROS and is not tested for the catalytic activity of the respiratory chain which is lacking. Another model for the production of ROS by complexes I and III has been proposed by Bazil el al. (Bazil et al.,2016). This model includes the respiratory chain model developed by Beard et al. (Beard, 2005). The model of Beard et al. models the activity of respiratory complexes by a law of mass action. In the Bazil et al. model (Bazil et al., 2016), the model for complex I *is* the one previously proposed by Bazil et al. (Bazil et al., 2014). In addition, a production of ROS with the semiquinone donor, in disagreement with the experimental data obtained on complex I, is assumed in the model (Bazil el al., 2014). Other models of complex III have also been proposed but focus solely on the mechanism of ROS production or quinol oxidation by complex III (Quinlan et al., 2011; Crofts et al., 2006). These models therefore do not meet the criteria that we set for the creation of our model and that are important to be used in a global model of a more comprehensive model of mitochondrial metabolism for different applications such as neurodegenerative pathologies, metabolic diseases, inflammatory diseases or ageing.

**References**

Bazil, J.N., Beard, D.A., and Vinnakota, K.C. (2016). Catalytic coupling of oxidative phosphorylation, atp demand, and reactive oxygen species generation. Biophysical journal, 110(4), 962-971.

Bazil, J.N., Pannala, V.R., Dash, R.K., and Beard, D.A.(2014). Determining the origins of superoxide and hydrogen peroxide in the mammalian nadh:ubiquinone oxidoreductase. Free radical biology & medicine, 77,121-129.

Beard, D.A. (2005). A biophysical model of the mitochondrial respiratory system and oxidative phosphorylation. PLoS computational biology, 1(4), e36.

Berry, E., Guergova-Kuras, M., Huang, L., and Crofts, A.(2000). Structure and function of cytochrome be complexes. Annual review of biochemistry, 69, 1005-1075.

Bleier, L. and Dröse, S. (2013). Superoxide generation by complex iii: from mechanistic rationales to functional ★ This work is supported by European Union FEDER and INSPIRE. consequences. Biochimica et biophysica acta, 1827(1112), 1320-1331.

Borek, A., Sarewicz, M., and Osyczka, A. (2008). Movement of the ironsulfur head domain of cytochrome bc1 transiently opens the catalytic qo site for reaction with oxygen. Biochemistry, 47(47), 12365-12370.

Chen, H., Detmer, S.A., Ewald, A.J., Griffin, E.E., Fraser, S.E., and Chan, D.C. (2003). Mitofusins mfn1 and mfn2 coordinately regulate mitochondrial fusion and are essential for embryonic development. The Journal of cell biology, 160(2), 189-200.

Chouchani, E.T., Pell, V.R., James, A.M., Work, L.M.,Saeb-Parsy, K., Frezza, C., Krieg, T., and Murphy, M.P. (2016). A unifying mechanism for mitochondrial superoxide production during ischemia-reperfusion injury. Cell Metabolism, 23(2), 254 - 263.

Crofts, A.R., Lhee, S., Crofts, S.B., Cheng, J., and Rose, S. (2006). Proton pumping in the bc1 complex: a new gating mechanism that prevents short circuits. Biochimica et biophysica acta, 1757(8), 1019-1034.

Deb, K., Pratap, A., Agarwal, S., and Meyarivan, T. (2002). A fast and elitist multiobjective genetic algorithm: Nsga-ii. IEEE Transactions on Evolutionary Computation, 6(2), 182-197.

Dröse, S. and Brandt, U. (2008). The mechanism of mitochondrial superoxide production by the cytochrome bc1 complex. The Journal of biological chemistry, 283(31), 21649-21654.

Esterh'azy, D., King, M.S., Yakovlev, G., and Hirst, J. (2008). Production of reactive oxygen species by complex i (nadh:ubiquinone oxidoreductase) from escherichia coli and comparison to the enzyme from mitochondria. Biochemistry, 47(12), 3964-3971.

Fato, R., Bergamini, C., Bortolus, M., Maniero, A.L., Leoni, S., Ohnishi, T., and Lenaz, G. (2009). Differential effects of mitochondrial complex i inhibitors on production of reactive oxygen species. Biochimica et biophysica acta, 1787(5), 384-392.

Fisher, N., Bowman, M.K., and Kramer, D.M. (2016). Electron Transfer Reactions at the Qo Site of the Cytochrome bc1 Complex: The Good, the Bad, and the Ugly, 419-434. Springer Netherlands, Dordrecht.

Genova, M., Ventura, B., Giuliano, G., Bovina, C., Formiggini, G., Parenti Castelli, G., and Lenaz, G. (2001). The site of production of superoxide radical in mitochondrial complex i is not a bound ubisemiquinone but presumably iron-sulfur cluster n2. FEBS letters, 505(3), 364-368.

Grivennikova, V.G. and Vinogradov, A.D. (2006). Generation of superoxide by the mitochondrial complex i. Biochimica et Biophysica Acta (BBA) - Bioenergetics, 1757(5), 553 - 561. 14th European Bioenergetics Conference.

Grivennikova, V.G. and Vinogradov, A.D. (2013). Partitioning of superoxide and hydrogen peroxide production by mitochondrial respiratory complex i. Biochimica et Biophysica Acta (BBA) - Bioenergetics, 1827(3), 446 - 454.

Grodzevich, O. and Romanko, O. (2006). Normalization and other topics in multi-objective optimization. In Fields-MITACS Industrial Problems Workshop.

Heiske, M., Letellier, T., and Klipp, E. (2017). Comprehensive mathematical model of oxidative phosphorylation valid for physiological and pathological conditions. The FEBS journal, 284(17), 2802-2828.

Heiske, M., Nazaret, C., and Mazat, J.P. (2014). Modeling the respiratory chain complexes with biothermokinetic equations - the case of complex i. Biochimica et Biophysica Acta (BBA) - Bioenergetics, 1837(10), 1707 - 1716.

Hinkle, P., Butow, R., Racker, E., and Chance, B. (1967). Partial resolution of the enzymes catalyzing oxidative phosphorylation. xv. reverse electron transfer in the flavin-cytochrome beta region of the respiratory chain of beef heart submitochondrial particles. The Journal of biological chemistry, 242(22), 5169-5173.

Hunte, C., Palsdottir, H., and Trumpower, B.L. (2003). Protonmotive pathways and mechanisms in the cytochrome bc1 complex. FEBS letters, 545(1), 39-46.

Iwata, S., Lee, J., Okada, K., Lee, J., Iwata, M., Rasmussen, B., Link, T., Ramaswamy, S., and Jap, B. (1998). Complete structure of the 11-subunit bovine mitochondrial cytochrome bc1 complex. Science (New York, N.Y.), 281(5373), 64-71.

Kim, M., Stepanova, A., Niatsetskaya, Z., Sosunov, S., Arndt, S., Murphy, M.P., Galkin, A., and Ten, V.S. (2018). Attenuation of oxidative damage by targeting mitochondrial complex i in neonatal hypoxic-ischemic brain injury. Free Radical Biology and Medicine, 124, 517 - 524.

Ksenzenko, M., Konstantinov, A., Khomutov, G., Tikhonov, A., and Ruuge, E. (1983). Effect of electron transfer inhibitors on superoxide generation in the cytochrome bc1 site of the mitochondrial respiratory chain. FEBS letters, 155(1), 19-24.

Kussmaul, L. and Hirst, J. (2006). The mechanism of superoxide production by nadh:ubiquinone oxidoreductase (complex i) from bovine heart mitochondria. Proceedings of the National Academy of Sciences of the United States of America, 103(20), 7607-7612.

Lambert, A.J. and Brand, M.D. (2004). Superoxide production by nadh:ubiquinone oxidoreductase (complex i) depends on the ph gradient across the mitochondrial inner membrane. The Biochemical journal, 382(Pt 2), 511-517.

Maranzana, E., Barbero, G., Falasca, A.I., Lenaz, G., and Genova, M.L. (2013). Mitochondrial respiratory supercomplex association limits production of reactive oxygen species from complex i. Antioxidants & redox signaling, 19(13), 1469-1480.

Merabet, N., Bordeneuve, J., and Davezac, N. (2017). Modelling the redox imbalance in dominant optic atrophy: the case of respiratory complex i. IFAC, 50, 862-867.

Mitchell, P. (1961). Coupling of phosphorylation to electron and hydrogen transfer by a chemi-osmotic type of mechanism. Nature, 191, 144-148.

Mitchell, P. (1966). Chemiosmotic coupling in oxidative and photosynthetic phosphorylation. Biological Reviews, 41(3), 445-501.

Moser, C., M. Keske, J., Warncke, K., Farid, R., and Leslie Dutton, P. (1992). Nature of biological electron transfer. Nature, 355, 796-802.

Muller, F., Crofts, A.R., and Kramer, D.M. (2002). Multiple q-cycle bypass reactions at the qo site of the cytochrome bc1 complex. Biochemistry, 41(25), 7866-7874.

Muller, F.L., Roberts, A.G., Bowman, M.K., and Kramer, D.M. (2003). Architecture of the qo site of the cytochrome bc1 complex probed by superoxide production. Biochemistry, 42(21), 6493-6499. Murphy, M.P. (2009). How mitochondria produce reactive oxygen species. The Biochemical journal, 417(1), 1 - 13.

Pell, V.R., Spiroski, A.M., Mulvey, J., Burger, N., Costa, A.S., Logan, A., Gruszczyk, A.V., Rosa, T., James, A.M., Frezza, C., Murphy, M.P., and Krieg, T. (2018). Ischemic preconditioning protects against cardiac ischemia reperfusion injury without affecting succinate accumulation or oxidation. Journal of Molecular and Cellular Cardiology, 123, 88 - 91.

Pryde, K.R. and Hirst, J. (2011). Superoxide is produced by the reduced flavin in mitochondrial complex i: A single, unified mechanism that applies during both forward and reverse electron transfer. Journal of Biological Chemistry, 286(20), 18056-18065.

Quinlan, C.L., Gerencser, A.A., Treberg, J.R., and Brand, M.D. (2011). The mechanism of superoxide production by the antimycin-inhibited mitochondrial q-cycle. The Journal of biological chemistry, 286(36), 31361-31372.

Robb, E.L., Hall, A.R., Prime, T.A., Eaton, S., Szibor, M., Viscomi, C., James, A.M., and Murphy, M.P. (2018). Control of mitochondrial superoxide production by reverse electron transport at complex i. The Journal of biological chemistry, 293(25), 9869-9879.

Rottenberg, H., Covian, R., and Trumpower, B.L. (2009). Membrane potential greatly enhances superoxide generation by the cytochrome bc1 complex reconstituted into phospholipid vesicles. The Journal of biological chemistry, 284(29), 19203-19210.

Roudenko, O. and Schoenauer, M. (2004). A steady performance stopping criterion for pareto-based evolutionary algorithms. In Proceedings of the 6th international multi-objective programming and goal programming conference.

Turrens, J., Alexandre, A., and Lehninger, A. (1985). Ubisemiquinone is the electron donor for superoxide formation by complex iii of heart mitochondria. Archives of biochemistry and biophysics, 237(2), 408-414.

Vinogradov, A. and Grivennikova, V. (2005). Generation of superoxide-radical by the nadh:ubiquinone oxidoreductase of heart mitochondria. Biochemistry. Biokhimiia, 70(2), 120-127.

Wong, H.S., Dighe, P.A., Mezera, V., Monternier, P.A., and Brand, M.D. (2017). Production of superoxide and hydrogen peroxide from specific mitochondrial sites under different bioenergetic conditions. Journal of Biological Chemistry, 292(41), 16804-16809

## Claims

1. A method for *in vitro* quantifying the reactive oxygen species, or ROS, appearance rate in a biological sample, the method comprising:

   a) determining the amount of 7 components involved in the activity of mitochondrial respiratory chain Complex I, or CI, or involved in the activity of mitochondrial respiratory chain Complex III, or CIII, or in the activity of both the mitochondrial respiratory chain CI and CIII, said 7 components being

   - the reduced form of Nicotinamide adenine dinucleotide, or NADH,
   - the oxidized form of Nicotinamide adenine dinucleotide, or NAD+,
   - quinone, or Q
   - quinol, or $QH_2$,
   - molecular oxygen, or $O_2$,
   - the reduced form of cytochrome C, or cytCred, and
   - the oxidized form of cytochrome C, or cytCox,

   in order to obtain a respective concentration value

   - [NADH], corresponding to the amount of NADH measured in said biological sample,
   - [NAD+], corresponding to the amount of NAD+ measured in said biological sample,
   - [Q], corresponding to the amount of quinone measured in said biological sample,
   - [$QH_2$], corresponding to the amount of quinol measured in said biological sample,

- [$O_2$], corresponding to the amount of molecular oxygen measured in said biological sample,
- [$CytC_{Red}$], corresponding to the amount of reduced form of cytochrome C measured in said biological sample,
- [$CytC_{ox}$], corresponding to the amount of oxidized form of cytochrome C measured in said biological sample,

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH], [NAD^+]) = \frac{\frac{[NADH]}{K_M^{NADH}}}{1 + \frac{[NADH]}{K_M^{NADH}} + \frac{[NAD^+]}{K_M^{NAD^+}}} \text{ in which } K_M^{NADH} = \frac{K_{M,0}^{NADH}}{1 + e^{-\frac{f_Q - k_1}{k_2}}} \; ;$$

$$f_Q([Q], [QH_2]) = \frac{\frac{[Q]}{K_M^Q}}{1 + \frac{[Q]}{K_M^Q} * I_{s,Q,QH_2} + \frac{[QH_2]}{K_M^{QH_2}} * I_{s,Q,QH_2}} \text{ in which } I_{s,Q,QH_2} = 1 + \frac{[Q]}{K_{is}^Q} + \frac{[QH_2]}{K_{is}^{QH_2}} \; ;$$

and

$$f_{O_2}([O_2]) = \frac{\frac{[O_2]}{K_{O_2}}}{\left(1 + \frac{[NAD^+]}{K_i^{NAD^+}}\right)(1 + \alpha f_Q)}$$

wherein

$V^+$ = 1727.2 mmol/min/mg ;
$K_{M,0}^{NADH}$ = 10,6
$K_1$ = 174.1, $K_2$ = 681.2,
$K_M^{NAD+}$ = 1028.6 $\mu$M,
$K_M^Q$ = 11.6 $\mu$M, $K_{is}^Q$ = 719128.5 $\mu$M,
$K_M^{QH2}$ = 16.3 $\mu$M, $K_{is}^{QH2}$ = 36.2 $\mu$M,
$K_i^{NAD+}$ = 26.4
$K_{O2}$ = 4871.0 and
$\alpha$ = 5.

c) calculating a second Ros appearance rate $V_{2\,Ros}$ of ROS in said sample

$$V_{2\,Ros} = V2^+ * f_{QH_{2o}} * f_{cytC_{ox}} * (1 + \frac{[Q]}{K_M^{Q_o}}) * f_{O_2}$$

wherein

$$f_{QH_{2o}}([QH_2], [Q]) = \frac{\frac{[QH_2]}{K_M^{QH_{2o}}}}{1 + \frac{[QH_2]}{K_M^{QH_{2o}}} + \frac{[Q]}{K_M^{Q_o}}} \text{ in which } K_M^{QH_{2o}} = \frac{K_{M,0}^{QH_{2o}}}{1 + e^{-\frac{f_{cytC_{ox}} - k_1}{k_2}}}$$

$$f_{cytC_{ox}}([cytC_{ox}],[cytC_{red}]) = \frac{\frac{[cytC_{ox}]}{K_M^{cytC_{ox}}}}{1+\frac{[cytC_{ox}]}{K_M^{cytC_{ox}}}+\frac{[cytC_{red}]}{K_M^{cytC_{red}}}} \text{ and } f_{Q_i}([Q],[QH_2]) = \frac{\frac{[Q]}{K_M^{Q_i}}}{1+\frac{[Q]}{K_M^{Q_i}}+\frac{[QH_2]}{K_M^{QH_{2i}}}},$$

and

$$f_{O_2}([O_2]) = \frac{\frac{[O_2]}{K'_{O_2}}}{1+\alpha' f_{Q_i} + \beta f_{cytC_{ox}}}$$

wherein

$V2^+$ = 3579.29 mmol/min/mg ;
$K_1$ = 23.05, $K_2$= 472.12,
$K_{M,0}^{QH20}$ =184.77 $\mu$M, $K_M^{QH2i}$ = 397,14,
$K_M^{Qo}$= 43.08 $\mu$M, $K_M^{Qi}$ = 225.21$\mu$M,
$K_M^{cytCox}$ = 8.11 $\mu$M, $K_M^{cytCred}$= 419.16$\mu$M,
$K'_{O2}$ = 914.51,
$\alpha'$= 22.02 and $\beta$=173.54,

d) obtaining the Ros appearance rate such that

$$VRos = V_{1Ros} + V_{2\,Ros.}$$

2. The method according to claim 1, wherein the method comprising:

b) calculating a first ROS appearance rate $V_{1\,Ros}$ of ROS in said sample

$$V_{1\,Ros} = V^+ * f_{NADH} * f_{O_2}$$

wherein

$$f_{NADH}([NADH],[NAD^+]) = \frac{\frac{[NADH]}{K_M^{NADH}}}{1+\frac{[NADH]}{K_M^{NADH}}*I_{s,NADH}+\frac{[NAD^+]}{K_M^{NAD^+}}*I_{s,NADH}}$$

in which

$$K_M^{NADH} = \frac{K_{M,0}^{NADH}}{1+e^{-\frac{f_Q-k_1}{k_2}}} \text{ and } I_{s,NADH} = 1+\frac{[NADH]}{K_{is}^{NADH}} \ ;$$

$$f_Q([Q],[QH_2]) = \frac{\frac{[Q]}{K_M^Q}}{1+\frac{[Q]}{K_M^Q}*I_{s,Q,QH_2}+\frac{[QH_2]}{K_M^{QH_2}}*I_{s,Q,QH_2}} \text{ in which } I_{s,Q,QH_2} = 1+\frac{[Q]}{K_{is}^Q}+\frac{[QH_2]}{K_{is}^{QH_2}};$$

$$f_{O_2}([O_2]) = \frac{\dfrac{[O_2]}{K_{O_2}}}{\left(1 + \dfrac{[NAD^+]}{K_i^{NAD^+}}\right)\left(1 + \alpha f_Q\right)}$$

wherein $V^+$ = 2121,1 mmol/min/mg ; $K_{M,0}^{NADH}$ = 8,1 μM, $K_1$ = 185,2, $K_2$= 1021,2, $K_M^{NAD+}$ = 625,8 μM, $K_M^Q$= 15,7 μM, $K_M^{QH2}$ = 26 μM, $K_{is}^Q$= 1066907,0 μM, $K_{is}^{QH2}$ = 48,6 μM, $K_{is}^{NADH}$ = 165076,9 μM $K_i^{NAD+}$ = 34,3 $K_{O2}$ = 9551,4 et $\alpha$= 3,3, and

c) calculating a second ROS appearance rate $V_{2\,Ros}$ of ROS in said sample

$$V_{2\,Ros} = V2^+ * f_{QH_{2o}} * f_{cytC_{ox}} * \left(1 + \frac{[Q]}{K_M^{Q_o}}\right) * f_{O_2}$$

wherein

$$f_{QH_{2o}}([QH_2],[Q]) = \frac{\dfrac{[QH_2]}{K_M^{QH_{2o}}}}{1 + \dfrac{[QH_2]}{K_M^{QH_{2o}}} + \dfrac{[Q]}{K_M^{Q_o}}} \text{ in which } K_M^{QH_{2o}} = \frac{K_{M,0}^{QH_{2o}}}{1 + e^{-\frac{f_{cytC_{ox}} - k_1}{k_2}}}$$

$$f_{cytC_{ox}}([cytC_{ox}],[cytC_{red}]) = (100 - \% saturation\ Antimycine) \frac{\dfrac{[cytC_{ox}]}{K_M^{cytC_{ox}}}}{1 + \dfrac{[cytC_{ox}]}{K_M^{cytC_{ox}}} + \dfrac{[cytC_{red}]}{K_M^{cytC_{red}}}}$$

and

$$f_{Q_i}([Q],[QH_2]) = (100 - \% saturation\ Antimycine) \frac{\dfrac{[Q]}{K_M^{Q_i}}}{1 + \dfrac{[Q]}{K_M^{Q_i}} + \dfrac{[QH_2]}{K_M^{QH_{2i}}}},$$

and

$$f_{O_2}([O_2]) = (\% saturation\ Antimycine) \frac{\dfrac{[O_2]}{K'_{O_2}}}{1 + \alpha' f_{Q_i} + \beta f_{cytC_{ox}}}$$

wherein

$V2^+$ = 3909.06 mmol/min/mg ;
$K_1$ = 19.96, $K_2$= 341.15,
$K_{M,0}^{QH_{2o}}$ = 157.61 μM, $K_M^{QH2i}$ = 478.63,
$K_M^{Qo}$= 50.49 μM, $K_M^{Qi}$ = 280.26μM,
$K_M^{cytCox}$ = 10.98 μM, $K_M^{cytCred}$= 248.18μM,
$K'_{O2}$ = 760.78,
$\alpha'$= 38.66 and $\beta$ = 212.03

d) obtaining the ROS appearance rate such that

$$VRos = V_{1Ros} + V_{2\ Ros.}$$

**3.** The method according to claim 1 or 2, wherein the activity of CI is determined by the following formula:

$$V_{NAD^+} = V^+ * f_{NADH} * f_Q.$$

**4.** The method according to claim 1 or 2, wherein the activity of CIII is determined by the following formula:

$$V_{cytC_{ox}} = V_{cytC_{ox},1} + V_{cytC_{ox},2} = V^+ * f_{QH_{2o}} * f_{cytC_{ox}} * \left( f_{Q_i}^{1/2} + \frac{f_{cytC_{ox}}}{1+\alpha f_{Q_i}} \right).$$

**5.** A computer program comprising instructions which, when the program is executed by a computer, causes the computer to carry out steps b) to d) of the method as defined in anyone of claims claim 1 to 4.

**6.** A method, for determining *in vitro* Ros detoxifying enzyme activity in a sample of an individual, the method comprising:

- quantifying the Ros appearance rate in the biological sample by carrying out the method as defined in anyone of claims 1 to 4, in order to obtain a calculated rate,
- comparing the calculated rate with a reference Ros appearance rate, said reference Ros appearance rate being obtained from a reference sample, the reference sample being of the same nature as the sample, in order to obtain a ratio R between the Ros appearance rate and the reference Ros appearance rate, and
- concluding that

* if R is lower than 0.85, then the sample contains efficient Ros detoxifying enzyme activity, in order to detoxify the sample during an oxidative stress,
* otherwise the sample does not contain efficient Ros detoxifying enzyme activity, in order to detoxify the sample during an oxidative stress.

**7.** The method according to claim 6, wherein

* if R lower than 0.8, then the sample contains efficient Ros detoxifying enzyme activity, in order to detoxify the sample during an oxidative stress,
* otherwise the sample does not contain efficient Ros detoxifying enzyme activity, in order to detoxify the sample during an oxidative stress.

**8.** A method for determining *in vitro* the response of a patient to a compound liable to reduce Ros amount in a biological sample, the method comprising

- quantifying the Ros appearance rate in the biological sample, before the administration of the compound to the patient, by carrying out the method as defined in anyone of claims 1 to 4, in order to obtain an initial Ros rate,
- quantifying the Ros appearance rate in the biological sample, after the administration of the compound to the patient, by carrying out the method as defined in anyone of claims 1 to 4, in order to obtain a final Ros rate,
- comparing the initial Ros rate and the final Ros rate in order to obtain a ratio R between the final Ros and the initial Ros rate,
- concluding that

* if R lower than 0,85, then the compound is able to reduce Ros amount in the sample,
* otherwise the sample is not able to reduce Ros amount in the sample.

**9.** The method according to claim 8, wherein

* if R is lower than to 0.8, then the compound is able to reduce Ros amount in the sample,
* otherwise the sample is not able to reduce Ros amount in the sample.

**10.** A method for determining *in vitro* in a biological sample if a compound is a pro- or an anti-oxidant compound, the method comprising:

- quantifying the Ros appearance rate in the biological sample by carrying out the method as defined in anyone of claims 1 to 4, in order to obtain a calculated rate,
- comparing the calculated rate with a reference Ros appearance rate, said reference Ros appearance rate being obtained from a reference sample, the reference sample being of the same nature as the sample, in order to obtain a ratio R between the Ros appearance rate and the reference Ros appearance rate, and
- concluding that

    * if R is lower than 0.85, then the compound is an anti-oxidant compound,
    * if R is higher than 1.15, then the compound is a pro-oxidant compound, and
    * if R is comprised from 0.85 to 1.15, then the compound has neither anti- nor pro-oxidant properties.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

**Complex I**

[Fig. 17]

**Complex I**

[Fig. 18]

[Fig. 19]

**Citrate synthase
activity**

[Fig. 20]

[Fig. 21]

[Fig. 22]

[Fig. 23]

[Fig. 24]

[Fig. 25]

[Fig. 26]

[Fig. 26 continued]

[Fig. 26 continued]

[Fig. 26 continued]

[Fig. 27]

[Fig. 28]

[Fig. 28 continued]

[Fig. 29]

| | | Europäisches<br>Patentamt<br>European<br>Patent Office<br>Office européen<br>des brevets | **EUROPEAN SEARCH REPORT** | | **Application Number**<br>**EP 23 30 5426** |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| A | BAZIL JASON N ET AL: "Catalytic Coupling of Oxidative Phosphorylation, ATP Demand, and Reactive Oxygen Species Generation", BIOPHYSICAL JOURNAL, ELSEVIER, AMSTERDAM, NL, vol. 110, no. 4, 23 February 2016 (2016-02-23), pages 962-971, XP029441550, ISSN: 0006-3495, DOI: 10.1016/J.BPJ.2015.09.036 * the whole document * ----- | 1-10 | INV.<br>G01N33/84 |
| A | MARKEVICH NIKOLAI I. ET AL: "Computational modeling analysis of mitochondrial superoxide production under varying substrate conditions and upon inhibition of different segments of the electron transport chain", BIOCHIMICA ET BIOPHYSICA ACTA. BIOENERGETICS., vol. 1847, no. 6-7, 1 June 2015 (2015-06-01), pages 656-679, XP93078465, NL ISSN: 0005-2728, DOI: 10.1016/j.bbabio.2015.04.005 * the whole document * ----- -/-- | 1-10 | TECHNICAL FIELDS<br>SEARCHED (IPC)<br><br>G01N |

The present search report has been drawn up for all claims

| Place of search | Date of completion of the search | Examiner |
|---|---|---|
| Munich | 5 September 2023 | Jacques, Patrice |

EPO FORM 1503 03.82 (P04C01)

**page 1 of 3**

**EP 4 439 067 A1**

Europäisches Patentamt

European Patent Office

Office européen des brevets

# EUROPEAN SEARCH REPORT

Application Number

**EP 23 30 5426**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAUTHIER LAURA D ET AL: "A Computational Model of Reactive Oxygen Species and Redox Balance in Cardiac Mitochondria", BIOPHYSICAL JOURNAL, ELSEVIER, AMSTERDAM, NL, vol. 105, no. 4, 20 August 2013 (2013-08-20), pages 1045-1056, XP028695205, ISSN: 0006-3495, DOI: 10.1016/J.BPJ.2013.07.006 * the whole document * | 1-10 | |
| A | QUINLAN CASEY L. ET AL: "The Mechanism of Superoxide Production by the Antimycin-inhibited Mitochondrial Q-cycle", JOURNAL OF BIOLOGICAL CHEMISTRY, vol. 286, no. 36, 27 June 2011 (2011-06-27), pages 31361-31372, XP93078781, US ISSN: 0021-9258, DOI: 10.1074/jbc.M111.267898 Retrieved from the Internet: URL:https://www.ncbi.nlm.nih.gov/pmc/artic les/PMC3173136/pdf/zbc31361.pdf> * the whole document * | 1-10 | |

-----

-----

-/--

TECHNICAL FIELDS SEARCHED (IPC)

The present search report has been drawn up for all claims

| Place of search | Date of completion of the search | Examiner |
|---|---|---|
| Munich | 5 September 2023 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 23 30 5426

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BEARD DANIEL A: "A Biophysical Model of the Mitochondrial Respiratory System and Oxidative Phosphorylation", PLOS COMPUTATIONAL BIOLOGY, vol. 1, no. 4, 9 September 2005 (2005-09-09), page e36, XP093078326, DOI: 10.1371/journal.pcbi.0010036 Retrieved from the Internet: URL:https://journals.plos.org/ploscompbiol/article/file?id=10.1371/journal.pcbi.0010036&type=printable> * the whole document * | 1-10 | |
| A | BAZIL JASON N. ET AL: "Determining the origins of superoxide and hydrogen peroxide in the mammalian NADH:ubiquinone oxidoreductase", FREE RADICAL BIOLOGY & MEDICINE, vol. 77, 1 December 2014 (2014-12-01), pages 121-129, XP093078333, US ISSN: 0891-5849, DOI: 10.1016/j.freeradbiomed.2014.08.023 * the whole document * | 1-10 | TECHNICAL FIELDS SEARCHED (IPC) |

The present search report has been drawn up for all claims

| Place of search | Date of completion of the search | Examiner |
|---|---|---|
| Munich | 5 September 2023 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ABRAMCZYK, H. ; BROZEK-PLUSKA, B. ; KO-PEC, M. ; SURMACKI, J. ; BLASZCZYK, M. ; RADEK, M.** Redox Imbalance and Biochemical Changes in Cancer by Probing Redox-Sensitive Mitochondrial Cytochromes in Label-Free Visible Resonance Raman Imaging. *Cancers,* 2021, vol. 13, 960
- **ABRAMCZYK, H.** *Scientific reports,* 2022
- **ALAVI, M.V. ; FUHRMANN, N. ; NGUYEN, H.P. ; YU-WAI-MAN, P. ; HEIDUSCHKA, P. ; CHINNERY, P.F. ; WISSINGER, B.** Subtle neurological and metabolic abnormalities in an Opa1 mouse model of autosomal dominant optic atrophy. *Experimental neurology,* 2009, vol. 220, 404-409
- **AMATI-BONNEAU, P. ; MILEA, D. ; BONNEAU, D. ; CHEVROLLIER, A. ; FERRE, M. ; GUILLET, V. ; GUEGUEN, N. ; LOISEAU, D. ; DE CRESCENZO, M.A. ; VERNY, C. et al.** OPA1-associated disorders: phenotypes and pathophysiology. *The international journal of biochemistry & cell biology,* 2009, vol. 41, 1855-1865
- **AMATI-BONNEAU, P. ; VALENTINO, M.L. ; REYNIER, P. ; GALLARDO, M.E. ; BORNSTEIN, B. ; BOISSIERE, A. ; CAMPOS, Y. ; RIVERA, H. ; DE LA ALEJA, J.G. ; CARROCCIA, R. et al.** OPA1 mutations induce mitochondrial DNA instability and optic atrophy 'plus' phenotypes. *Brain : a journal of neurology,* 2008, vol. 131, 338-351
- **BARBONI, P. ; VALENTINO, M.L. ; LA MORGIA, C. ; CARBONELLI, M. ; SAVINI, G. ; DE NEGRI, A. ; SIMONELLI, F. ; SADUN, F. ; CAPORALI, L. ; MARESCA, A. et al.** Idebenone treatment in patients with OPA1-mutant dominant optic atrophy. *Brain : a journal of neurology,* 2013, vol. 136, e231
- **BERTHOLET, A.M. ; DELERUE, T. ; MILLET, A.M. ; MOULIS, M.F. ; DAVID, C. ; DALOYAU, M. ; ARNAUNE-PELLOQUIN, L. ; DAVEZAC, N. ; MILS, V. ; MIQUEL, M.C. et al.** Mitochondrial fusion/fission dynamics in neurodegeneration and neuronal plasticity. *Neurobiology of disease,* 2016, vol. 90, 3-19
- **BERTHOLET, A.M. ; MILLET, A.M. ; GUILLERMIN, O. ; DALOYAU, M. ; DAVEZAC, N. ; MIQUEL, M.C. ; BELENGUER, P.** OPA1 loss of function affects in vitro neuronal maturation. *Brain : a journal of neurology,* 2013, vol. 136, 1518-1533

- **BROOKES, P.S. ; YOON, Y. ; ROBOTHAM, J.L. ; ANDERS, M.W. ; SHEU, S.S.** Calcium, ATP, and ROS: a mitochondrial love-hate triangle. *American journal of physiology. Cell physiology,* 2004, vol. 287, C817-833
- **CHAO DE LA BARCA, J.M. ; PRUNIER-MIREBEAU, D. ; AMATI-BONNEAU, P. ; FERRE, M. ; SARZI, E. ; BRIS, C. ; LERUEZ, S. ; CHEVROLLIER, A. ; DESQUIRET-DUMAS, V. ; GUEGUEN, N. et al.** OPA1-related disorders: Diversity of clinical expression, modes of inheritance and pathophysiology. *Neurobiology of disease,* 2016, vol. 90, 20-26
- **CHEVROLLIER, A. ; GUILLET, V. ; LOISEAU, D. ; GUEGUEN, N. ; DE CRESCENZO, M.A. ; VERNY, C. ; FERRE, M. ; DOLLFUS, H. ; ODENT, S. ; MILEA, D. et al.** Hereditary optic neuropathies share a common mitochondrial coupling defect. *Annals of neurology,* 2008, vol. 63, 794-798
- **COHN, A.C. ; TOOMES, C. ; POTTER, C. ; TOWNS, K.V. ; HEWITT, A.W. ; INGLEHEARN, C.F. ; CRAIG, J.E. ; MACKEY, D.A.** Autosomal dominant optic atrophy: penetrance and expressivity in patients with OPA1 mutations. *American journal of ophthalmology,* 2007, vol. 143, 656-662
- **COLOMBANI, A.L. ; CARNEIRO, L. ; BENANI, A. ; GALINIER, A. ; JAILLARD, T. ; DUPARC, T. ; OFFER, G. ; LORSIGNOL, A. ; MAGNAN, C. ; CASTEILLA, L. et al.** Enhanced hypothalamic glucose sensing in obesity: alteration of redox signaling. *Diabetes,* 2009, vol. 55, 2189-2197
- **COUSTHAM, C. ; MERABET, N. ; BORDE-NEUVE-GUIBE, J. ; DAVEZAC, N.** Modeling of Complex I catalytic activity and ROS production: a multi-objective optimization for personalized medicine. *GFB,* 2021
- **DALOYAU, M. ; MILLET, A. ; MIQUEL, M.C. ; MILS, V. ; WISSINGER, B. ; BELENGUER, P. ; DAVEZAC, N.** Brains from aged opa1+/-(B6;C3-Opa1 329-355del) Mouse Strain Are in a Pro-Oxidative State. *ROS,* 2018, vol. 6, 1-10
- **DELETTRE, C. ; LENAERS, G. ; GRIFFOIN, J.M. ; GIGAREL, N. ; LORENZO, C. ; BELENGUER, P. ; PELLOQUIN, L. ; GROSGEORGE, J. ; TURC-CAREL, C. ; PERRET, E. et al.** Nuclear gene OPA1, encoding a mitochondrial dynamin-related protein, is mutated in dominant optic atrophy [In Process Citation. *Nat Genet,* 2000, vol. 26, 207-210

- **GALINIER, A. ; CARRIERE, A. ; FERNANDEZ, Y. ; CARPENE, C. ; ANDRE, M. ; CASPAR-BAUGUIL, S. ; THOUVENOT, J.P. ; PERIQUET, B. ; PENICAUD, L. ; CASTEILLA, L.** Adipose tissue proadipogenic redox changes in obesity. *The Journal of biological chemistry,* 2006, vol. 281, 12682-12687
- **GARCIA, I. ; INNIS-WHITEHOUSE, W. ; LOPEZ, A. ; KENIRY, M. ; GILKERSON, R.** Oxidative insults disrupt OPA1-mediated mitochondrial dynamics in cultured mammalian cells. *Redox Rep,* 2018, vol. 23, 160-167
- **GARDNER, P.R. ; NGUYEN, D.D. ; WHITE, C.W.** Aconitase is a sensitive and critical target of oxygen poisoning in cultured mammalian cells and in rat lungs. *Proceedings of the National Academy of Sciences of the United States of America,* 1994, vol. 91, 12248-12252
- **GREEN, D.R. ; GALLUZZI, L. ; KROEMER, G.** Mitochondria and the autophagyinflammation-cell death axis in organismal aging. *Science,* 2011, vol. 333, 1109-1112
- **GRIPARIC, L. ; VAN DER WEL, N.N. ; OROZCO, I.J. ; PETERS, P.J. ; VAN DER BLIEK, A.M.** Loss of the intermembrane space protein Mgm1/OPA1 induces swelling and localized constrictions along the lengths of mitochondria. *The Journal of biological chemistry,* 2004, vol. 279, 18792-18798
- **GRIVENNIKOVA, V.G. ; VINOGRADOV, A.D.** Generation of superoxide by the mitochondrial Complex I. *Biochimica et biophysica acta,* 2006, vol. 1757, 553-561
- **HEISKE, M. ; NAZARET, C. ; MAZAT, J.P.** Modeling the respiratory chain complexes with biothermokinetic equations - the case of complex I. *Biochimica et biophysica acta,* 2014, vol. 1837, 1707-1716
- **ISHIHARA, N. ; FUJITA, Y. ; OKA, T. ; MIHARA, K.** Regulation of mitochondrial morphology through proteolytic cleavage of OPA1. *The EMBO journal,* 2006, vol. 25, 2966-2977
- **KANAZAWA, T. ; ZAPPATERRA, M.D. ; HASEGAWA, A. ; WRIGHT, A.P. ; NEWMAN-SMITH, E.D. ; BUTTLE, K.F. ; MCDONALD, K. ; MANNELLA, C.A. ; VAN DER BLIEK, A.M.** The C. elegans Opa1 homologue EAT-3 is essential for resistance to free radicals. *PLoS genetics,* 2008, vol. 4, e1000022
- **KELLY, M. ; TRUDEL, S. ; BROUILLARD, F. ; BOUILLAUD, F. ; COLAS, J. ; NGUYEN-KHOA, T. ; OLLERO, M. ; EDELMAN, A. ; FRITSCH, J.** Cystic fibrosis transmembrane regulator inhibitors CFTR(inh)-172 and GlyH-101 target mitochondrial functions, independently of chloride channel inhibition. *The Journal of pharmacology and experimental therapeutics,* 2010, vol. 333, 60-69
- **KIENHOFER, J. ; HAUSSLER, D.J. ; RUCK-ELSHAUSEN, F. ; MUESSIG, E. ; WEBER, K. ; PIMENTEL, D. ; ULLRICH, V. ; BURKLE, A. ; BACHSCHMID, M.M.** Association of mitochondrial antioxidant enzymes with mitochondrial DNA as integral nucleoid constituents. *FASEB journal : official publication of the Federation of American Societies for Experimental Biology,* 2009, vol. 23, 2034-2044
- **KUSSMAUL, L. ; HIRST, J.** The mechanism of superoxide production by NADH:ubiquinone oxidoreductase (complex I) from bovine heart mitochondria. *Proceedings of the National Academy of Sciences of the United States of America,* 2006, vol. 103, 7607-7612
- **LENAERS, G. ; HAMEL, C. ; DELETTRE, C. ; AMATI-BONNEAU, P. ; PROCACCIO, V. ; BONNEAU, D. ; REYNIER, P. ; MILEA, D.** Dominant optic atrophy. *Orphanet journal of rare diseases,* 2012, vol. 7, 46
- **LOPEZ-ARMADA, M.J. ; RIVEIRO-NAVEIRA, R.R. ; VAAMONDE-GARCIA, C. ; VALCARCEL-ARES, M.N.** Mitochondrial dysfunction and the inflammatory response. *Mitochondrion,* 2013, vol. 13, 106-118
- **MA, Q.** Role of nrf2 in oxidative stress and toxicity. *Annual review of pharmacology and toxicology,* 2013, vol. 53, 401-426
- **MACKEY, D.A. ; TROUNCE, !.** Genetics: Optic nerve genetics--more than meets the eye. *Nature reviews. Neurology,* 2010, vol. 6, 357-358
- **MERABET, N. ; BORDENEUVE-GUIBE, J. ; DAVEZAC, N.** Modelling the redox imbalance in Dominant Optic Atrophy: the case of respiratory Complex I. *IFAC-PapersOnLine,* 2017, 50
- **MILLET A., M.N. ; BERTHOLET A. ; DALOYAU M. ; REYNIER P. ; DEVIN A. ; WISSINGER B. ; BORDENEUVE-GUIBE J. ; DAVEZAC N.** Imbalance of the REDOX state in dominant Optic Atrophy: the way of mathematical modeling. *Archives of the International Society of Antioxidants in Nutrition and Health,* 2017, vol. 5, 21-24
- **MILLET, A.M. ; BERTHOLET, A.M. ; DALOYAU, M. ; REYNIER, P. ; GALINIER, A. ; DEVIN, A. ; WISSINGUER, B. ; BELENGUER, P. ; DAVEZAC, N.** Loss of functional OPA1 unbalances redox state: implications in dominant optic atrophy pathogenesis. *Annals of clinical and translational neurology,* 2016, vol. 3, 408-421
- **OLICHON, A. ; EMORINE, L.J. ; DESCOINS, E. ; PELLOQUIN, L. ; BRICHESE, L. ; GAS, N. ; GUILLOU, E. ; DELETTRE, C. ; VALETTE, A. ; HAMEL, C.P. et al.** The human dynamin-related protein OPA1 is anchored to the mitochondrial inner membrane facing the inter-membrane space. *FEBS letters,* 2002, vol. 525, 171-176

- **OLICHON, A. ; GUILLOU, E. ; DELETTRE, C. ; LANDES, T. ; ARNAUNE-PELLOQUIN, L. ; EMORINE, L.J. ; MILS, V. ; DALOYAU, M. ; HAMEL, C. ; AMATI-BONNEAU, P. et al.** Mitochondrial dynamics and disease, OPA1. *Biochimica et biophysica acta,* 2006, vol. 1763, 500-509
- **SATOH, M. ; HAMAMOTO, T. ; SEO, N. ; KAGAWA, Y. ; ENDO, H.** Differential sublocalization of the dynamin-related protein OPA1 isoforms in mitochondria. *Biochemical and biophysical research communications,* 2003, vol. 300, 482-493
- **SCHRINER, S.E. ; LINFORD, N.J. ; MARTIN, G.M. ; TREUTING, P. ; OGBURN, C.E. ; EMOND, M. ; COSKUN, P.E. ; LADIGES, W. ; WOLF, N. ; VAN REMMEN, H. et al.** Extension of murine life span by overexpression of catalase targeted to mitochondria. *Science,* 2005, vol. 308, 1909-1911
- **SHAHRESTANI, P. ; LEUNG, H.T. ; LE, P.K. ; PAK, W.L. ; TSE, S. ; OCORR, K. ; HUANG, T.** Heterozygous mutation of Drosophila Opa1 causes the development of multiple organ abnormalities in an age-dependent and organ-specific manner. *PloS one,* 2009, vol. 4, e6867
- **SPINAZZI, M. ; CAZZOLA, S. ; BORTOLOZZI, M. ; BARACCA, A. ; LORO, E. ; CASARIN, A. ; SOLAINI, G. ; SGARBI, G. ; CASALENA, G. ; CENACCHI, G. et al.** A novel deletion in the GTPase domain of OPA1 causes defects in mitochondrial morphology and distribution, but not in function. *Human molecular genetics,* 2008, vol. 17, 3291-3302
- **TANG, S. ; LE, P.K. ; TSE, S. ; WALLACE, D.C. ; HUANG, T.** Heterozygous mutation of Opa1 in Drosophila shortens lifespan mediated through increased reactive oxygen species production. *PloS one,* 2009, vol. 4, e4492
- **VINCENT, A.M. ; MCLEAN, L.L. ; BACKUS, C. ; FELDMAN, E.L.** Short-term hyperglycemia produces oxidative damage and apoptosis in neurons. *FASEB journal : official publication of the Federation of American Societies for Experimental Biology,* 2005, vol. 19, 638-640
- **YAROSH, W. ; MONSERRATE, J. ; TONG, J.J. ; TSE, S. ; LE, P.K. ; NGUYEN, K. ; BRACHMANN, C.B. ; WALLACE, D.C. ; HUANG, T.** The molecular mechanisms of OPA1-mediated optic atrophy in Drosophila model and prospects for antioxidant treatment. *PLoS genetics,* 2008, vol. 4, e6
- **YU-WAI-MAN, P. ; GRIFFITHS, P.G. ; GORMAN, G.S. ; LOURENCO, C.M. ; WRIGHT, A.F. ; AUER-GRUMBACH, M. ; TOSCANO, A. ; MUSUMECI, O. ; VALENTINO, M.L. ; CAPORALI, L. et al.** Multisystem neurological disease is common in patients with OPA1 mutations. *Brain : a journal of neurology,* 2010, vol. 133, 771-786
- **YU-WAI-MAN, P. ; TRENELL, M.I. ; HOLLINGSWORTH, K.G. ; GRIFFITHS, P.G. ; CHINNERY, P.F.** OPA1 mutations impair mitochondrial function in both pure and complicated dominant optic atrophy. *Brain : a journal of neurology,* 2011, vol. 134, e164
- **ZANNA, C. ; GHELLI, A. ; PORCELLI, A.M. ; KARBOWSKI, M. ; YOULE, R.J. ; SCHIMPF, S. ; WISSINGER, B. ; PINTI, M. ; COSSARIZZA, A. ; VIDONI, S. et al.** OPA1 mutations associated with dominant optic atrophy impair oxidative phosphorylation and mitochondrial fusion. *Brain : a journal of neurology,* 2008, vol. 131, 352-367
- **ZEVIANI, M.** OPA1 mutations and mitochondrial DNA damage: keeping the magic circle in shape. *Brain : a journal of neurology,* 2008, vol. 131, 314-317
- **BAZIL, J.N. ; BEARD, D.A. ; VINNAKOTA, K.C.** Catalytic coupling of oxidative phosphorylation, atp demand, and reactive oxygen species generation. *Biophysical journal,* 2016, vol. 110 (4), 962-971
- **BAZIL, J.N. ; PANNALA, V.R. ; DASH, R.K. ; BEARD, D.A.** Determining the origins of superoxide and hydrogen peroxide in the mammalian nadh:ubiquinone oxidoreductase. *Free radical biology & medicine,* 2014, vol. 77, 121-129
- **BEARD, D.A.** A biophysical model of the mitochondrial respiratory system and oxidative phosphorylation. *PLoS computational biology,* 2005, vol. 1 (4), e36
- **BERRY, E. ; GUERGOVA-KURAS, M. ; HUANG, L. ; CROFTS, A.** Structure and function of cytochrome bc complexes. *Annual review of biochemistry,* 2000, vol. 69, 1005-1075
- **BLEIER, L. ; DRÖSE, S.** Superoxide generation by complex iii: from mechanistic rationales to functional ★ This work is supported by European Union FEDER and INSPIRE. consequences. *Biochimica et biophysica acta,* 2013, vol. 1827 (1112), 1320-1331
- **BOREK, A. ; SAREWICZ, M. ; OSYCZKA, A.** Movement of the ironsulfur head domain of cytochrome bc1 transiently opens the catalytic qo site for reaction with oxygen. *Biochemistry,* 2008, vol. 47 (47), 12365-12370
- **CHEN, H. ; DETMER, S.A. ; EWALD, A.J. ; GRIFFIN, E.E. ; FRASER, S.E. ; CHAN, D.C.** Mitofusins mfn1 and mfn2 coordinately regulate mitochondrial fusion and are essential for embryonic development. *The Journal of cell biology,* 2003, vol. 160 (2), 189-200
- **CHOUCHANI, E.T. ; PELL, V.R. ; JAMES, A.M. ; WORK, L.M. ; SAEB-PARSY, K. ; FREZZA, C. ; KRIEG, T. ; MURPHY, M.P.** A unifying mechanism for mitochondrial superoxide production during ischemia-reperfusion injury. *Cell Metabolism,* 2016, vol. 23 (2), 254-263

- **CROFTS, A.R. ; LHEE, S. ; CROFTS, S.B. ; CHENG, J. ; ROSE, S.** Proton pumping in the bc1 complex: a new gating mechanism that prevents short circuits. *Biochimica et biophysica acta,* 2006, vol. 1757 (8), 1019-1034
- **DEB, K. ; PRATAP, A. ; AGARWAL, S. ; ME-YARIVAN, T.** A fast and elitist multiobjective genetic algorithm: Nsga-ii. *IEEE Transactions on Evolutionary Computation,* 2002, vol. 6 (2), 182-197
- **DRÖSE, S. ; BRANDT, U.** The mechanism of mitochondrial superoxide production by the cytochrome bc1 complex. *The Journal of biological chemistry,* 2008, vol. 283 (31), 21649-21654
- **ESTERH'AZY, D. ; KING, M.S. ; YAKOVLEV, G. ; HIRST, J.** Production of reactive oxygen species by complex i (nadh:ubiquinone oxidoreductase) from escherichia coli and comparison to the enzyme from mitochondria. *Biochemistry,* 2008, vol. 47 (12), 3964-3971
- **FATO, R. ; BERGAMINI, C. ; BORTOLUS, M. ; MANIERO, A.L. ; LEONI, S. ; OHNISHI, T. ; LENAZ, G.** Differential effects of mitochondrial complex i inhibitors on production of reactive oxygen species. *Biochimica et biophysica acta,* 2009, vol. 1787 (5), 384-392
- **FISHER, N. ; BOWMAN, M.K. ; KRAMER, D.M.** Electron Transfer Reactions at the Qo Site of the Cytochrome bc1 Complex: The Good, the Bad, and the Ugly. Springer, 2016, 419-434
- **GENOVA, M. ; VENTURA, B. ; GIULIANO, G. ; BOVINA, C. ; FORMIGGINI, G. ; PARENTI CAS-TELLI, G. ; LENAZ, G.** The site of production of superoxide radical in mitochondrial complex i is not a bound ubisemiquinone but presumably iron-sulfur cluster n2. *FEBS letters,* 2001, vol. 505 (3), 364-368
- **GRIVENNIKOVA, V.G. ; VINOGRADOV, A.D.** Generation of superoxide by the mitochondrial complex i. *Biochimica et Biophysica Acta (BBA) - Bioenergetics,* 2006, vol. 1757 (5), 553-561
- **GRIVENNIKOVA, V.G. ; VINOGRADOV, A.D.** Partitioning of superoxide and hydrogen peroxide production by mitochondrial respiratory complex i. *Biochimica et Biophysica Acta (BBA) - Bioenergetics,* 2013, vol. 1827 (3), 446-454
- **GRODZEVICH, O. ; ROMANKO, O.** Normalization and other topics in multi-objective optimization. *Fields-MITACS Industrial Problems Workshop,* 2006
- **HEISKE, M. ; LETELLIER, T. ; KLIPP, E.** Comprehensive mathematical model of oxidative phosphorylation valid for physiological and pathological conditions. *The FEBS journal,* 2017, vol. 284 (17), 2802-2828
- **HEISKE, M. ; NAZARET, C. ; MAZAT, J.P.** Modeling the respiratory chain complexes with biothermokinetic equations - the case of complex i. *Biochimica et Biophysica Acta (BBA) - Bioenergetics,* 2014, vol. 1837 (10), 1707-1716
- **HINKLE, P. ; BUTOW, R. ; RACKER, E. ; CHANCE, B.** Partial resolution of the enzymes catalyzing oxidative phosphorylation. xv. reverse electron transfer in the flavin-cytochrome beta region of the respiratory chain of beef heart submitochondrial particles. *The Journal of biological chemistry,* 1967, vol. 242 (22), 5169-5173
- **HUNTE, C. ; PALSDOTTIR, H. ; TRUMPOWER, B.L.** Protonmotive pathways and mechanisms in the cytochrome bc1 complex. *FEBS letters,* 2003, vol. 545 (1), 39-46
- **IWATA, S. ; LEE, J. ; OKADA, K. ; LEE, J. ; IWATA, M. ; RASMUSSEN, B. ; LINK, T. ; RAMASWAMY, S. ; JAP, B.** Complete structure of the 11-subunit bovine mitochondrial cytochrome bc1 complex. *Science (New York, N.Y.),* 1998, vol. 281 (5373), 64-71
- **KIM, M. ; STEPANOVA, A. ; NIATSETSKAYA, Z. ; SOSUNOV, S. ; ARNDT, S. ; MURPHY, M.P. ; GALKIN, A. ; TEN, V.S.** Attenuation of oxidative damage by targeting mitochondrial complex i in neonatal hypoxic-ischemic brain injury. *Free Radical Biology and Medicine,* 2018, vol. 124, 517-524
- **KSENZENKO, M. ; KONSTANTINOV, A. ; KHOMU-TOV, G. ; TIKHONOV, A. ; RUUGE, E.** Effect of electron transfer inhibitors on superoxide generation in the cytochrome bc1 site of the mitochondrial respiratory chain. *FEBS letters,* 1983, vol. 155 (1), 19-24
- **KUSSMAUL, L. ; HIRST, J.** The mechanism of superoxide production by nadh:ubiquinone oxidoreductase (complex i) from bovine heart mitochondria. *Proceedings of the National Academy of Sciences of the United States of America,* 2006, vol. 103 (20), 7607-7612
- **LAMBERT, A.J. ; BRAND, M.D.** Superoxide production by nadh:ubiquinone oxidoreductase (complex i) depends on the ph gradient across the mitochondrial inner membrane. *The Biochemical journal,* 2004, vol. 382, 511-517
- **MARANZANA, E. ; BARBERO, G. ; FALASCA, A.I. ; LENAZ, G. ; GENOVA, M.L.** Mitochondrial respiratory supercomplex association limits production of reactive oxygen species from complex i. *Antioxidants & redox signaling,* 2013, vol. 19 (13), 1469-1480
- **MERABET, N. ; BORDENEUVE, J. ; DAVEZAC, N.** Modelling the redox imbalance in dominant optic atrophy: the case of respiratory complex i. *IFAC,* 2017, vol. 50, 862-867
- **MITCHELL, P.** Coupling of phosphorylation to electron and hydrogen transfer by a chemi-osmotic type of mechanism. *Nature,* 1961, vol. 191, 144-148

- **MITCHELL, P.** Chemiosmotic coupling in oxidative and photosynthetic phosphorylation. *Biological Reviews,* 1966, vol. 41 (3), 445-501
- **MOSER, C. ; M. KESKE, J. ; WARNCKE, K. ; FARID, R. ; LESLIE DUTTON, P.** Nature of biological electron transfer. *Nature,* 1992, vol. 355, 796-802
- **MULLER, F. ; CROFTS, A.R. ; KRAMER, D.M.** Multiple q-cycle bypass reactions at the qo site of the cytochrome bc1 complex. *Biochemistry,* 2002, vol. 41 (25), 7866-7874
- **MULLER, F.L. ; ROBERTS, A.G. ; BOWMAN, M.K. ; KRAMER, D.M.** Architecture of the qo site of the cytochrome bc1 complex probed by superoxide production. *Biochemistry,* 2003, vol. 42 (21), 6493-6499
- **MURPHY, M.P.** How mitochondria produce reactive oxygen species. *The Biochemical journal,* 2009, vol. 417 (1), 1-13
- **PELL, V.R. ; SPIROSKI, A.M. ; MULVEY, J. ; BURGER, N. ; COSTA, A.S. ; LOGAN, A. ; GRUSZCZYK, A.V. ; ROSA, T. ; JAMES, A.M. ; FREZZA, C.** Ischemic preconditioning protects against cardiac ischemia reperfusion injury without affecting succinate accumulation or oxidation. *Journal of Molecular and Cellular Cardiology,* 2018, vol. 123, 88-91
- **PRYDE, K.R. ; HIRST, J.** Superoxide is produced by the reduced flavin in mitochondrial complex i: A single, unified mechanism that applies during both forward and reverse electron transfer. *Journal of Biological Chemistry,* 2011, vol. 286 (20), 18056-18065
- **QUINLAN, C.L. ; GERENCSER, A.A. ; TREBERG, J.R. ; BRAND, M.D.** The mechanism of superoxide production by the antimycin-inhibited mitochondrial q-cycle. *The Journal of biological chemistry,* 2011, vol. 286 (36), 31361-31372
- **ROBB, E.L. ; HALL, A.R. ; PRIME, T.A. ; EATON, S. ; SZIBOR, M. ; VISCOMI, C. ; JAMES, A.M. ; MURPHY, M.P.** Control of mitochondrial superoxide production by reverse electron transport at complex i. *The Journal of biological chemistry,* 2018, vol. 293 (25), 9869-9879
- **ROTTENBERG, H. ; COVIAN, R. ; TRUMPOWER, B.L.** Membrane potential greatly enhances superoxide generation by the cytochrome bc1 complex reconstituted into phospholipid vesicles. *The Journal of biological chemistry,* 2009, vol. 284 (29), 19203-19210
- **ROUDENKO, O. ; SCHOENAUER, M.** A steady performance stopping criterion for pareto-based evolutionary algorithms. *Proceedings of the 6th international multi-objective programming and goal programming conference,* 2004
- **TURRENS, J. ; ALEXANDRE, A. ; LEHNINGER, A.** Ubisemiquinone is the electron donor for superoxide formation by complex iii of heart mitochondria. *Archives of biochemistry and biophysics,* 1985, vol. 237 (2), 408-414
- **VINOGRADOV, A. ; GRIVENNIKOVA, V.** Generation of superoxide-radical by the nadh:ubiquinone oxidoreductase of heart mitochondria. *Biochemistry. Biokhimiia,* 2005, vol. 70 (2), 120-127
- **WONG, H.S. ; DIGHE, P.A. ; MEZERA, V. ; MONTERNIER, P.A. ; BRAND, M.D.** Production of superoxide and hydrogen peroxide from specific mitochondrial sites under different bioenergetic conditions. *Journal of Biological Chemistry,* 2017, vol. 292 (41), 16804-16809